# EUROPEAN PATENT APPLICATION

(11) **EP 4 173 674 A1**
(43) Date of publication of application: **03.05.2023**
(21) Application number: 21306491.8
(22) Date of filing: 26.10.2021
(51) Int. Cl.: A61P 3/00, A61P 3/10, A61P 19/02, A61P 25/00, A61P 25/16, A61P 25/28, A61P 31/00, A61P 31/12, A61P 31/16, A61P 31/18, A61P 31/22, A61P 33/00, A61P 33/06, A61P 35/00, C07D 401/06, C07D 401/14, C07D 403/06, C07D 403/14, C07D 405/14, C07D 417/14, C07D 471/04

(54) **IMIDAZOLONE DERIVATIVES AS INHIBITORS OF PROTEIN KINASES IN PARTICULAR DYRK1A, CLK1 AND/OR CLK4**

(71) Applicant: Perha Pharmaceuticals, 29680 Roscoff (FR)
(72) Inventor: DEAU, Emmanuel, 29680 Roscoff (FR); GEORGE, M. Pascal, 78730 Longvilliers (FR); MEIJER, Laurent, 29680 Roscoff (FR); MIEGE, Frédéric, 69008 Lyon (FR); ARCHAMBAUD, Sylvie, 44800 Saint Herblain (FR)
(74) Representative: Cabinet Nony

(57) **Abstract**

The present invention relates to a compound of formula (I) 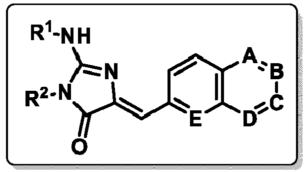 wherein A, B, C, D and E are selected from the group consisting of =CH- and -N=, R² is selected from a hydrogen atom, a (C₁-C₄)alkyl group and a (C₃-C₆)cycloalkyl group, R¹ represents a (C₄-C₆)alkyl group, a (C₃-C₈)cycloalkyl group, a bridged (C₆-C₁₀)cycloalkyl group, a fused phenyl group, a substituted phenyl group, a R'-L- group, wherein L is either a single bond or a (C₁-C₃)alkanediyl group, and R' represents , a (C₃-C₈)heterocycloalkyl group, or a (C₃-C₈)heteroaryl group, or a R'-L- group wherein L is a (C₁-C₃)alkanediyl group, and R' is a an optionally substituted phenyl group or any of its pharmaceutically acceptable salt.

The present invention further relates to a composition comprising a compound of formula (I) and a process for manufacturing said compound as well as its synthesis intermediates.

It also relates to said compound for use as a medicament, in particular in the treatment and/or prevention of cognitive deficits and neuroinflammation associated with Down syndrome, Alzheimer's disease, dementia and/or tauopathies; Parkinson's disease; CDKL5 Deficiency Disorder; Phelan-McDermid syndrome; autism; type 1 and type 2 diabetes; abnormal folate and methionine metabolism; tendinopathy and osteoarthritis; Duchenne muscular dystrophy; several cancers; neuroinflammation, anemia, infections and for regulating body temperature.

## Description

### FIELD OF THE INVENTION

The present invention relates to Leucettinibs, a class of new compounds useful as a medicament. Said new compounds are in particular useful as kinase inhibitors, and even more particularly as inhibitors of DYRK1A and/or CLK1 and/or CLK4. They are efficient for treating and/or preventing cognitive deficits associated with Down syndrome; Alzheimer's disease and related diseases; dementia; tauopathies; Parkinson's disease; other neurodegenerative diseases; CDKL5 Deficiency Disorder; type 1 and type 2 diabetes; abnormal folate and methionine metabolism; osteoarthritis and tendinopathy; Duchenne muscular dystrophy; several cancers and leukemias, viral infections and for regulating body temperature.

Some of said compounds are further inhibitors of other kinases and namely other DYRKs (DYRK1B, 2, 3, 4) and the closely related cdc2-like kinases (CLKs) (CLK 2, 3, 4). Said compounds may then further be efficient for treating and/or preventing Phelan-McDermid syndrome; autism; viral infections, cancers, neuroinflammation, anemia and infections caused by unicellular parasites.

It further relates to the pharmaceutical compositions containing said new compounds and to the chemical synthesis processes for obtaining them.

### BACKGROUND

The **DYRK** and **CLK** kinase families belong to the CMGC group of kinases which also includes the mitogen-activated protein kinases (MAPK), cyclin-dependent kinases (CDKs) and glycogen synthase kinase-3 (GSK-3). They phosphorylate many substrates involved in signaling pathways. **DYRKs** and **CLKs** play key roles in mRNA splicing, chromatin transcription, DNA damage repair, cell survival, cell cycle, differentiation, homocysteine/methionine/folate regulation, endocytosis, neuronal development and functions, synaptic plasticity (review in Lindberg, M. and Meijer, L., 2021. Dual-specificity, tyrosine phosphorylation-regulated kinases (DYRKs) and cdc2-like kinases (CLKs) in human disease, an overview. Internat. J. Mol. Sci. 22, 6047).

### DYRK1A and Down syndrome (DS)

The gene encoding **DYRK1A** is located on chromosome 21, in particular in the **"Down syndrome critical region"** (DSCR), the triploidy of which is responsible for most DS-associated deficiencies. There is considerable genetic and pharmacological evidence showing that the mere 1.5-fold overexpression of **DYRK1A** is responsible for most cognitive deficits, especially memory and learning deficits, observed in DS patients (Feki, A., Hibaoui, Y., 2018. DYRK1A protein, a promising therapeutic target to improve cognitive deficits in Down syndrome. Brain Sci. 8, 187; Rueda N et al., 2020. Translational validity and implications of pharmacotherapies in preclinical models of Down syndrome. Prog Brain Res 251, 245). Pharmacological or genetical normalization of DYRK1A levels restores cognitive functions (Nguyen TL et al., 2017. Dual-specificity tyrosine phosphorylation-regulated kinase 1A (DYRK1A) inhibitors: a survey of recent patent literature. Expert Opin. Ther. Pat. 27, 1183-1199; Nguyen TL et al., 2018. Correction of cognitive deficits in mouse models of Down syndrome by pharmacological inhibitor of DYRK1A. Dis. Model Mech. 11, dmm035634).

### DYRK1A and Alzheimer's disease (AD), Tauopathies

There is mounting evidence for a role of **DYRK1A** in the onset of AD. **DYRK1A** phosphorylates key substrates involved in AD and dementia: Tau, septin 4, amyloid precursor protein (APP), presenilin 1, neprilysin, Munc18-1, α-synuclein, RCAN1, β-Tubulin. There is evidence for abnormal expression and post-translational modifications of DYRK1A in AD. By modulating alternative splicing of exon 10, **DYRK1A** favors the production of the 3R-Tau splice isoform (characteristic for DS/AD/tauopathy) over the normal 4R-Tau isoform. **DYRK1A** inhibition promotes autophagy which could counterbalance the autophagy deficit seen in AD. There is a clear association of AD with DS (Fortea J. et al., 2021. Alzheimer's disease associated with Down syndrome: a genetic form of dementia. The Lancet 20, 930-942): most DS patients show AD neuropathology at an early age (40's) and high prevalence of dementia in later age (>60).

### DYRK1A and Parkinson's disease (PD) and Pick disease

GWAS studies have revealed that **DYRK1A** is a risk factor for PD (Nalls MA et al., 2019. Identification of novel risk loci, causal insights, and heritable risk for Parkinson's disease: a meta-analysis of genome-wide association studies. Lancet Neurol 18, 1091). **DYRK1A** phosphorylates key factors for PD such as Parkin, septin 4, α-synuclein. Upregulation of micro-RNA specific for PD target DYRK1A expression (Chiu CC et al., 2019. Upregulated expression of microRNA-204-5p leads to the death of dopaminergic cells by targeting DYRK1A-mediated apoptotic signaling cascade. Front Cell Neurosci 13, 399). There is further evidence that **DYRK1A** expression is increased in PD. **DYRK1A** is overexpressed in Pick disease.

### DYRK1A and viral infections

**DYRK1A** and **DYRK1B** are utilized during HCMV placental replication. Inhibition of **DYRKs** prevent replication of various viruses including Herpes virus, cytomegalovirus and HIV-1.

### DYRK1A and type 1 and type 2 diabetes

**DYRK1A** inhibitors stimulate the proliferation of pancreatic, insulin-producing β-cells. This constitutes a promising approach to diabetes, both type 1 (which displays low number of β-cells) and type 2 (where the β-cell mass is reduced by half) (Ackeifi C et al., 2020. Pharmacologic and genetic approaches define human pancreatic β cell mitogenic targets of DYRK1A inhibitors. JCI Insight 5, e132594; Kumar K et al., 2021. DYRK1A inhibitors as potential therapeutics for β-cell regeneration for diabetes. J Med Chem 64, 2901-2922. Barzowska A, et al., 2021. DYRK1A kinase inhibitors promote β-cell survival and insulin homeostasis. Cells. 10, 2263; Wang P et al., 2021. Human beta cell regenerative drug therapy for diabetes: past achievements and future challenges. Front Endocrinol 12, 671946). Besides direct application to diabetic patients, **DYRK1A** inhibitors could be applied to stimulate β-cell proliferation in vitro or ex vivo to increase β-cell mass prior to grafting.

### DYRK1A, cancers and leukemias

There is abundant literature linking **DYRK1A** with cancer. The most prominent examples are megakaryoblastic leukemia (Malinge S et al., 2012. Increased dosage of the chromosome 21 ortholog Dyrkla promotes megakaryoblastic leukemia in a murine model of Down syndrome. J Clin Invest 122, 948-962), acute lymphoblastic leukemia (Bhansali RS et al., 2021. DYRK1A regulates B cell Acute Lymphoblastic Leukemia through phosphorylation of FOXO1 and STAT3. J Clin Investig, 131, e135937), pancreatic cancer and brain tumor (glioblastoma) (see review in Lindberg and Meijer, 2021. cited above).

Accordingly, abnormalities in **DYRK1A** dosage are associated with cognitive disorders observed in Down syndrome, and Alzheimer's disease. **DYRK1A** is a risk factor for Parkinson's disease. Inhibition of **DYRK1A** additionally triggers the proliferation of pancreatic, insulin-producing β-cells. **DYRK1A** inhibitors may thus find applications in preventing and/or treating DS, AD, and other Tauopathies (particularly cognitive deficits associated with these pathologies), dementia, PD, Niemann-Pick Type C Disease, CDKL5 deficiency disorder, type 1 and type 2 diabetes, viral infections, several cancers (leukemia, pancreatic cancer, glioblastoma), osteoarthritis, infections caused by unicellular parasites and for regulating body temperature.

### Other DYRKs and human disease

**DYRK1B** is involved in the replication of various viruses including hepatitis C virus, Chikungunya virus, Dengue virus and SARS coronavirus, cytomegalovirus and human papillomavirus. Like **DYRK1A, DYRK1B** inhibition leads to the proliferation of pancreatic, insulin-producing β-cells. **DYRK1B** is involved in neuroinflammation. Targeting **DYRK1B** provides a new rationale for treatment of various cancers such as liposarcoma or breast cancers.

**DYRK2,** in association with GSK-3β, regulates neuronal morphogenesis. **DYRK2** is involved in various ways in cancer development.

**DYRK3** promotes hepatocellular carcinoma. **DYRK3** couples stress granule condensation/dissolution to mTORC1 signaling. **DYRK3** regulates phase transition of membraneless organelles in mitosis. **DYRK3** and **DYRK4** are involved in the regulation of cytoskeletal organization and process outgrowth in neurons.

**DYRK1A** decreases axon growth, **DYRK3** and **DYRK4** increase dendritic branching and **DYRK2** decreases both axon and dendrite growth and branching.

### CLKs and human disease

Note that **CLK** is a confusing abbreviation as it has the following meanings: (a) monooxygenase CLK-1 (human homologue COQ7); (b) Collectin-K1 (CL-K1, or CL-11), a multifunctional Ca(2+)-dependent lectin; (c) MAPK gene of the maize pathogen *Curvularia lunata,* Clk1; **(d)** mitochondrial membrane-bound enzyme Clock-1 (CLK-1); **(e)** *Colletotrichum lindemuthianum* kinase 1 (clk1).

**CLKs** play essential functions in alternative splicing. **CLKs** act as a body-temperature sensor which globally controls alternative splicing and gene expression. The activity of CLKs is indeed highly responsive to physiological temperature changes, which is conferred by structural rearrangements within the kinase activation segment (Haltenhof T et al., 2020. A conserved kinase-based body-temperature sensor globally controls alternative splicing and gene expression. Mol Cell 78, 57).

### CLK1 and human disease

**CLK1** triggers periodic alternative splicing during the cell division cycle. **CLK1** regulates influenza A virus mRNA splicing and its inhibition prevents viral replication. **CLK1** and **CLK2** also regulates HIV-1 gene expression. **CLK1** is an autophagy inducer. **CLK1** inhibition may prevent chemoresistance in glioma and **CLK1** inhibition by TG693 allows the skipping of mutated exon 31 of the dystrophin gene in Duchenne Muscular Dystrophy.

### Other CLKs and human disease

Inhibition of **CLK2** has been proposed as a way to improve neuronal functions and combat intellectual disability and autism in Phelan-McDermid syndrome (PMDS). Dual inhibition of **CLK2** and DYRK1A by Lorecivivint is a potential disease-modifying approach for knee osteoarthritis. **CLK2** inhibition compromises MYC-driven breast tumors, triple negative breast cancer and glioblastoma. Inhibition of **CLK2** improves autistic features in Phelan-McDermid syndrome (PMDS). Alternative splicing of Tau exon 10 is regulated by **CLK2** and other **CLKs,** leading to changes in the 3R/4R isoforms ratio and neurodegeneration in sporadic AD. Inhibition of **CLK2, CLK3, CLK4** blocks HIV-1 production. By regulating alternative splicing **CLKs** modulate the balance between proapoptotic and anti-apoptotic regulators, and inhibition of **CLKs** may thus find applications in the treatment of numerous cancers, in particular prostate cancer and hepatocellular carcinoma.

The following Table 1 summarizes the implication of the DYRKs and CLKs kinases in various diseases.

**Table 1**

| **Kinase target** | **Disease** |
|---|---|
| DYRK1A | Down syndrome (DS) |
| DYRK1A | Alzheimer's disease (AD) and other Tauopathies |
| DYRK1A | Parkinson's disease |
| DYRK1A | Pick disease |
| DYRK1A | CDKL5 Deficiency Disorder |
| DYRK1A | Type 1 and Type 2 diabetes |
| DYRK1A | Abnormalities in folate and methionine metabolism |
| DYRK1A | Glioblastoma |
| DYRK1A | Head and neck squamous cell carcinoma |
| DYRK1A | Pancreatic ductal adenocarcinoma |
| DYRK1A | Megakaryoblastic leukemia |
| DYRK1A | Acute Lymphoblastic Leukemia (ALL) |
| DYRK1A | Knee osteoarthritis, tendinopathy |
| DYRK1A | Human immunodeficiency virus type 1 (HIV-1) |
| **DYRK1A, DYRK1B** | Human cytomegalovirus (HCMV) |
| **DYRK1B** | Hepatitis C virus, Chikungunya virus, Dengue virus and Severe acute respiratory syndrome coronavirus, Cytomegalovirus, Human papillomavirus |
| **DYRK1B** | Type 1 and Type 2 diabetes |
| **DYRK1B** | Neuroinflammation |
| **DYRK1B** | Liposarcoma, Breast cancer, Hedgehog/GLI-dependent cancer |
| DYRK2 | Triple-negative breast cancer (TNBC) and multiple myeloma (MM) |
| DYRK2 | Glioblastoma |
| DYRK3 | Hepatocellular carcinoma |
| DYRK3 | Influenza virus replication |
| DYRK3 | Anemia |
| DYRKs | Glioblastoma |
| DYRKs | Herpes simplex virus, cytomegalovirus, varicella-zoster virus |
| LmDYRK1 | Leishmaniasis |
| TbDYRK | *Trypanosoma brucei* |
| | |
| CLK1 | Glioblastoma |
| CLK1 | Duchenne muscular dystrophy |
| CLK1 | Influenza A |
| CLK2 | HIV-1 |
| CLK1/CLK2 | Triple-negative breast cancer |
| CLK2 | Autism, Phelan-McDermid syndrome (PMDS) |
| CLK2 | Knee osteoarthritis, tendinopathy |
| CLK2 | Breast cancer, Triple negative breast cancer, Glioblastoma |
| CLK2 | Alzheimer's disease (alternative splicing of Tau exon 10) |
| CLK3 | Hepatocellular carcinoma, Prostate cancer |
| CLKs | Body temperature |
| CLKs | Prostate cancer, Gastrointestinal cancer |
| PfCLKs | Malaria |
| | |
| DYRKs/CLKs | Glioblastoma and numerous other cancers |

### DYRKs and CLK inhibitors

Several DYRK1A inhibitors have been reported in recent years. Most DYRK1A inhibitors also inhibit DYRK1B, 2, 3, 4, as well as the closely related CLK1, 2, 3, 4, with several possible inhibition profiles.

Some imidazolone derivatives, named **Leucettines** in the text below, were disclosed in WO 2009/050352 as kinase inhibitors and more particularly as inhibitors of the DYRK1A kinase.

WO 2021/114314 and WO 2021/115489 disclose compounds useful in cardiomyocyte proliferation activity for the treatment of heart diseases. However, these documents comprise a very broad range of compounds illustrated in a very partial way and focused on benzothiazoles derivatives. In other words, none of the compounds disclosed are comprised in the formula (I) of the present invention. Furthermore, the compounds disclosed in these applications do not have an optimized activity with regards to DYRK1A and CLK1 and are not directed to the PATHOLOGIES as defined herein after.

WO 2006/040052 discloses the compound below 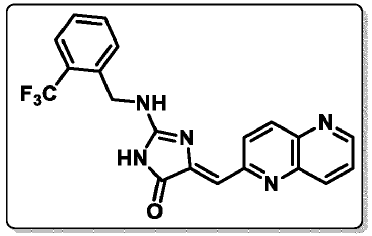 which is useful as inhibitory agent for CDK1 protein kinase but does not concern the inhibition of either DYRK1A or CLK1.

There is still a need to identify new compounds for treating and/or preventing the diseases as recited above, and particularly through the inhibition, and in particular selective inhibition of **DYRK1A,** other DYRKs and the related CLKs kinases.

### SUMMARY OF THE INVENTION

It has now been found that the compounds as defined in formula (I) herein after are useful in the treatment and/or prevention of a disease selected from cognitive deficits and neuroinflammation associated with Down syndrome, Alzheimer's disease and related diseases, dementia and tauopathies Parkinson's disease and other neurodegenerative diseases; CDKL5 Deficiency Disorder; Phelan-McDermid syndrome; autism; type 1 and type 2 diabetes; abnormal folate and methionine metabolism; osteoarthritis and tendinopathy; several cancers and leukemias, neuroinflammation, anemia, infections caused by unicellular parasites, viral infections and for regulating body temperature.

The present invention therefore relates to a compound of formula (I), as defined below.

The present invention further relates to a compound of formula (I) as defined below for use as a medicament.

The present invention further relates to a compound of formula (I) as defined below for use in the treatment and/or prevention of a disease selected from cognitive deficits and neuroinflammation associated with Down syndrome, Alzheimer's disease and related diseases, dementia or tauopathies (in particular cognitive deficits and neuroinflammation associated with these indications); Parkinson's disease; other neurodegenerative diseases; CDKL5 Deficiency Disorder; Phelan-McDermid syndrome; autism; type 1 and type 2 diabetes; abnormal folate and methionine metabolism; osteoarthritis and tendinopathy; several cancers and leukemias, neuroinflammation, anemia, infections caused by unicellular parasites, viral infections and for regulating body temperature.

The present invention further relates to a pharmaceutical composition comprising it and to a process for manufacturing it.

The present invention at last relates to synthetic intermediates of formula (III) and (VI) as defined below.

### DEFINITIONS

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means preventing, reversing, alleviating, inhibiting the progress of, or preventing the disease and its cognitive, motor or metabolic changes resulting from high DYRK1A kinase and/or CLK1 expression and activity, and optionally associated with the abnormalities in other DYRKs (DYRK1B, 2, 3, 4) and the closely related further cdc2-like kinases (CLKs) (CLK 2, 3, 4) and more particularly in connection to the diseases as described herein after in the paragraph "PATHOLOGIES".

Therefore, the term "treating" or "treatment" encompasses within the framework of the present invention the improvement of medical conditions of patients suffering from the diseases as described herein after in the paragraph "PATHOLOGIES", related to high expression and activity of any of the DYRK1A and CLK1 kinases, and optionally associated with the abnormalities in other DYRKs (DYRK1B, 2, 3, 4) and the closely related further cdc2-like kinases (CLKs) (CLK 2, 3, 4).

As used herein, an "effective amount" refers to an amount of a compound of the present invention which is effective in preventing, reducing, eliminating, treating or controlling the symptoms of the herein-described diseases and conditions.

The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "effective amount" includes "prophylaxis-effective amount" as well as "treatment-effective amount".

The term "preventing", as used herein, means reducing the risk of onset or slowing the occurrence of a given phenomenon, namely in the present invention, a disease resulting from abnormal DYRKs/CLKs kinase activity, in particular DYRK1A kinase activity.

As used herein, *"preventing"* also encompasses *"reducing the likelihood of occurrence*" or *"reducing the likelihood of reoccurrence*".

The term *"prophylaxis-effective amount"* refers to a concentration of compound of this invention that is effective in inhibiting, preventing, decreasing the likelihood of anyone of the hereabove described diseases.

Likewise, the term "treatment-effective amount" refers to a concentration of compound that is effective in treating the hereabove described diseases, e.g. leads to a reduction or normalization DYRK1A and/or CLK1 kinase activity, and optionally additionally of DYRKs/CLKs kinase activity in general, following examination when administered after disease has occurred.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### DETAILED DESCRIPTION OF THE INVENTION

The inventors have surprisingly found that the compounds of formula (I) as disclosed herein after inhibit the **DYRK1A,** other DYRKs (DYRK1B, DYRK2, DYRK3, DYRK4) and CLKs (CLK1, CLK2, CLK3, CLK4). This assertion is based on data as illustrated in the following examples and more detailed herein after.

According to a first aspect, a subject-matter of the present invention relates to a compound of formula (I) 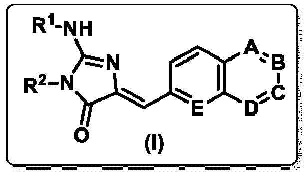 wherein:
A, B, C, D and E are selected from the group consisting of =CH- and -N=,
at least one and not more than two of A, B, C, D and E is -N=,
at least one of A and B is -N=,
R² is selected from a hydrogen atom, a (C₁-C₄)alkyl group and a (C3-C6)cycloalkyl group, and
wherein R¹ represents:
   (i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
   (ii). a bridged (C₆-C₁₀)cycloalkyl group, optionally substituted by a group selected from a (C₁-C₄)alkyl group, a (C₁-C₄)alkoxy group, a halogen atom and a hydroxy group,
   (iii). a fused phenyl group, selected from phenyl groups fused with a (C₅-C₆)cycloalkyl, which (C₅-C₆)cycloalkyl is optionally substituted by a hydroxy group,
   (iv). a phenyl group, substituted by a (C₄-C₇)heterocycloalkyl group, said (C₄-C₇)heterocycloalkyl group being itself optionally substituted by a (C₁-C₄) alkyl group, or
   (v). a R'-L- group, wherein L is either a single bond or a (C₁-C₃)alkanediyl group, optionnally susbstituted by a group selected from a hydroxy group and a (C₁-C₃)alkoxy group, and
      R' represents:
         (v.1). a (C₃-C₈)cycloalkyl group, optionally substituted by a group selected from a hydroxy group and a (C₁-C₃) alkoxy group,
         (v.2). a (C₃-C₉)heterocycloalkyl group, optionally substituted by one or two groups selected from a hydroxy group and (C₁-C₄)alkyl groups, or
         (v.3). a (C₃-C₈)heteroaryl group, optionally substituted by a (C₁-C₄)alkyl group, or
   (vi). a R"-L- group wherein L is a (C₁-C₃)alkanediyl group, optionally substituted by a group selected from a -NR^{b}R^{c} group, a (C₁-C₃)alkoxy group and a hydroxy group, and
      R" is a phenyl group, optionally substituted by a fluoro(C₁-C₄)alkyl group,
R^{b} and R^{c} independently represent a (C₁-C₆)alkyl group or a hydrogen atom, with the provision that the compound of the formula below is excluded 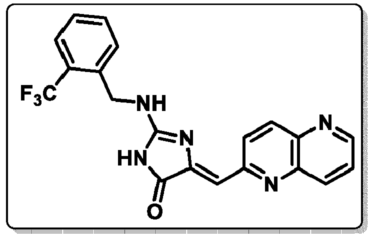 or any of its pharmaceutically acceptable salt.

The inventors have surprisingly discovered that compounds having the following scaffolds (A) to (D) displayed much reduced kinase inhibitory activities on DYRK1A and other related kinases compared to their homologs (compounds according to the invention): IC₅₀ values were reduced by 10 to 1000 -fold factors, and some compounds were completely inactive at the highest dose tested (10 µM).

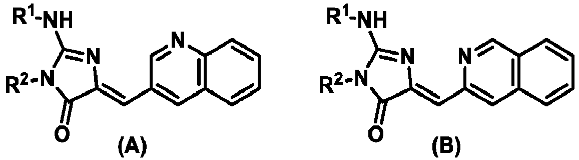

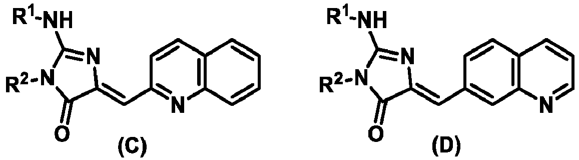

These much-reduced kinase inhibitory activities have been verified, for example, by individual comparison of a compound of formula (I) and of a compound having a scaffold of formula (A) to (D), wherein in both, R¹ is selected from the group consisting of 1-adamantyl, 2-methoxy-1-phenyl-ethyl, and R² is a hydrogen atom.

According to a particular embodiment, the present invention relates to a compound of formula (I) as defined herein above, wherein R¹ represents
(i). a (C₅-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₉-C₁₀)cycloalkyl group, optionally substituted by a group selected from a (C₁-C₄)alkoxy group, a halogen atom and a hydroxy group,
(iii). a phenyl group fused with a cyclopentyl, which cyclopentyl is substituted by a hydroxy group,
(iv). a phenyl group, substituted by a (C₄-C₇)heterocycloalkyl group, said (C₄-C₇)heterocycloalkyl group being itself optionally substituted by a (C₁-C₄) alkyl group, or
(v). a R'-L- group wherein
   - L is either a single bond or a (C₁-C₃)alkanediyl group, optionnally susbstituted by a group selected from a hydroxy group and a (C₁-C₃)alkoxy group, and
   - R' represents:
      (v.1). a (C₅-C₈)cycloalkyl group, optionally substituted by a group selected from a hydroxy group and a (C₁-C₃) alkoxy group,
      (v.2). a (C₅-C₇)heterocycloalkyl group, optionally substituted by one or two groups selected from a hydroxy group and (C₁-C₄)alkyl groups, or
      (v.3). a heteroaryl group, optionally substituted by a (C₁-C₄)alkyl group,
(vi). a R"-L- group wherein
   - L is a (C₁-C₃)alkanediyl group, optionally substituted by a group selected from a -NR^{b}R^{c} group, a (C₁-C₃)alkoxy group and a hydroxy group, L being in particular substituted, and
   - R" is a phenyl group, optionally substituted by a fluoro(C₁-C4)alkyl group, and
R^{b} and R^{c} independently represent a (C₁-C₆)alkyl group or a hydrogen atom, or any of its pharmaceutically acceptable salt.

According to another particular embodiment, the present invention relates to a compound of formula (I) as defined herein above, wherein R¹ represents:
(i). a (C₅-C₆)alkyl group substituted by a group selected from a hydroxy group, a fluorine atom and a methoxy group, said methoxy group being optionally substituted by a phenyl group, said phenyl group being optionally substituted by a fluorine atom,
(ii). a bridged (C₉-C₁₀)cycloalkyl group in particular an adamantyl or a noradamantyl, optionally substituted by a group selected from a methoxy group, a fluorine atom and a hydroxy group,
(iii). a phenyl group fused with a cyclopentyl, which cyclopentyl is substituted by a hydroxy group,
(iv). a phenyl group, substituted by a *N*-methylpiperazinyl group,
(v). a R'-L- group wherein L is either a single bond or a (C₁-C₃)alkanediyl group, optionnally susbstituted by a group chosen from a hydroxy group and a methoxy group,
   and R' is selected from the group consisting of:
   (v.1). a (C₅-C₈)cycloalkyl group in particular a cyclopentyl, a cyclohexyl, a cycloheptyl or a cyclooctyl, optionally substituted by a group selected from a hydroxy group and a methoxy group,
   (v.2). a (C₆-C₇)heterocycloalkyl group, in particular a tetrahydrofuranyl, a tetrahydropyranyl, an oxepanyl or a dioxepanyl, optionally substituted by one or two groups selected from a hydroxy group and a methyl group,
   (v.3). a heteroaryl group, in particular a thiazolyl, a pyrazinyl, a pyrazolyl or a pyridinyl, optionally substituted by a methyl group, or
(vi). a R"-L- group wherein L is a (C₁-C₃)alkanediyl group, optionally substituted by a group selected from the group consisting of a -NH2 group, a methoxy group and a hydroxy group, L being in particular substituted, and
   R" is a phenyl group, optionally substituted by a trifluoromethyl group,
or any of its pharmaceutically acceptable salts.

According to another particular embodiment, the present invention relates to a compound of formula (I) as defined herein above wherein L is selected from a group consisting of a -CH2- group, a -CH(CH₂OH)- group, a -CH(CH₂OCH₃)- group, a -CH(OH)-CH2- group and a -CH(CH2NH2)- group, or any of its pharmaceutically acceptable salts.

According to another particular embodiment, the present invention relates to a compound of formula (I) as defined herein above wherein
(v.1). when R' is a (C₃-C₈)cycloalkyl group, L is a -CH₂- group,
(v.2). when R' is a (C₃-C₉)heterocycloalkyl group, L is a -CH₂-group,
(v.3). when R" is a phenyl, L is selected from the group consisting of a -CH2- group, a -CH(CH₂OH)- group, a -CH(CH₂OCH₃)- group, a -CH(OH)-CH2- group and a -CH(CH2NH2)- group,
(v.4). when R' is a heteroaryl group, L is a -CH₂- group,
or any of its pharmaceutically acceptable salts.

According to another particular embodiment, the present invention relates to a compound of formula (I) as defined herein above wherein A and E are different from -N=.

According to another particular embodiment, the present invention relates to a compound of formula (I') 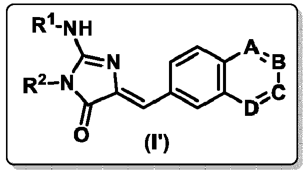 wherein A, B, C, D, R¹ and R² are as defined above.

According to another particular embodiment, the present invention relates to a compound of formula (I) as defined herein above wherein R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkyl group, a (C₁-C₄)alkoxy group, a halogen atom and a hydroxy group,
(iii). a fused phenyl group, selected from phenyl groups fused with a (C₅-C₆)cycloalkyl, which (C₅-C₆)cycloalkyl is optionally substituted by a hydroxy group,
(iv). a phenyl group, substituted by a (C₄-C₇)heterocycloalkyl group, said (C₄-C₇)heterocycloalkyl group being itself optionally substituted by a (C₁-C₄) alkyl group, or
(v). a R'-L- group, wherein L is either a single bond or a (C₁-C₃)alkanediyl group, optionnally susbstituted by a group selected from a hydroxy group and a (C₁-C₃)alkoxy group, and
   R' represents:
   (v.1). a (C₃-C₈)cycloalkyl group, optionally substituted by a group selected from a hydroxy group and a (C₁-C₃) alkoxy group,
   (v.2). a (C₃-C₉)heterocycloalkyl group, optionally substituted by one or two groups selected from a hydroxy group and (C₁-C₄)alkyl groups, or
   (v.3). a (C₃-C₈)heteroaryl group, optionally substituted by a (C₁-C₄)alkyl group,
or any of its pharmaceutically acceptable salt.

According to another particular embodiment, the present invention relates to a compound selected from the following compounds 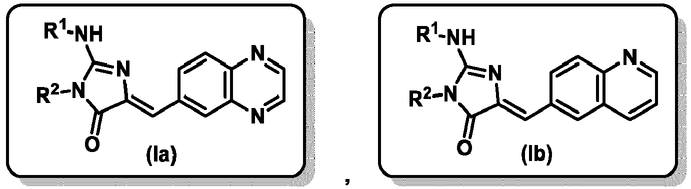 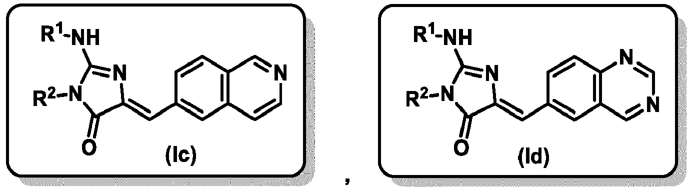 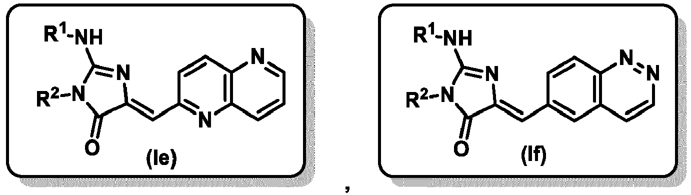 and 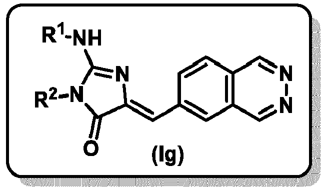
wherein R¹ and R² are as defined above,
or any of its pharmaceutically acceptable salts.

According to another particular embodiment, the present invention relates to a compound of formula (I) as defined herein above wherein R² is a hydrogen atom or a (C₁-C₄)alkyl group, in particular R² is a hydrogen atom.

According to another particular embodiment, the present invention relates to a compound of formula (I) as defined herein above wherein when the compound of formula (I) is chosen from the subformulae (Ia) and (Ib), R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkoxy group, a halogen atom and a hydroxy group, or
(iii). a R'-L- group, wherein L is either a single bond or a (C₁-C₃)alkanediyl group, optionnally susbstituted by a group selected from a hydroxy group and a (C₁-C₃)alkoxy group, and
   R' represents:
   (iii.1). a (C₃-C₈)cycloalkyl group, optionally substituted by a group selected from a hydroxy group and a (C₁-C₃) alkoxy group,
   (iii.2). a (C₃-C₉)heterocycloalkyl group, optionally substituted by one or two groups selected from a hydroxy group and (C₁-C₄)alkyl groups, or
   (iii.3). a (C₃-C₈)heteroaryl group, optionally substituted by a (C₁-C₄)alkyl group, or
(iv). a R"-L- group wherein L is a (C₁-C₃)alkanediyl group, optionally substituted by a group selected from a -NR^{b}R^{c} group, a (C₁-C₃)alkoxy group and a hydroxy group, L being in particular substituted, and
   R" is a phenyl group, optionally substituted by a fluoro(C₁-C₄)alkyl group,

R^{b} and R^{c} independently represent a (C₁-C₆)alkyl group or a hydrogen atom, or any of its pharmaceutically acceptable salt.

According to another particular embodiment, the present invention relates to a compound of formula (I) as defined herein above wherein R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkoxy group, a halogen atom and a hydroxy group, or
(iii). a R'-L- group, wherein L is either a single bond or a (C₁-C₃)alkanediyl group, optionnally susbstituted by a group selected from a hydroxy group and a (C₁-C₃)alkoxy group, and
   R' represents:
   (iii.1). a (C₃-C₈)cycloalkyl group, optionally substituted by a group selected from a hydroxy group and a (C₁-C₃) alkoxy group,
   (iii.2). a (C₃-C₉)heterocycloalkyl group, optionally substituted by one or two groups selected from a hydroxy group and (C₁-C₄)alkyl groups, or
   (iii.3). a (C₃-C₈)heteroaryl group, optionally substituted by a (C₁-C₄)alkyl group, or
(iv). a R"-L- group wherein L is a (C₁-C₃)alkanediyl group, optionally substituted by a group selected from a -NR^{b}R^{c} group, a (C₁-C₃)alkoxy group and a hydroxy group, L being in particular substituted, and
   R" is a phenyl group, optionally substituted by a fluoro(C₁-C₄)alkyl group,

R^{b} and R^{c} independently represent a (C₁-C₆)alkyl group or a hydrogen atom, or any of its pharmaceutically acceptable salt.

According to another particular embodiment, the present invention relates to a compound of formula (Ia) 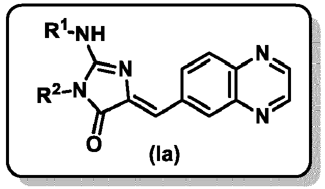 wherein R¹ represents:
- a (C₅-C₆)alkyl group substituted by a group selected from a hydroxy group, a halogen atom, in particular a fluorine atom, and a (C₁-C₃)alkoxy group, in particular a methoxy group or an ethoxy group, said (C₁-C₃)alkoxy group being optionally substituted by a phenyl group, said phenyl group being optionally substituted by a halogen atom, in particular a fluorine atom,
- a bridged (C₉-C₁₀)cycloalkyl group, in particular an adamantyl group or a noradamantyl group, optionally substituted by a group selected from a methoxy group, a fluorine atom and a hydroxy group
- a phenyl group fused with a cyclopentyl, which cyclopentyl is substituted by a hydroxy group,
- a phenyl group, substituted by a *N*-methylpiperazinyl group,
- a R'-L- group, L is either a single bond or a methylene group, and
   R' is selected from the group consisting of:
   ○ a (C₅-C₈)cycloalkyl group in particular a cyclopentyl, a cyclohexyl, a cycloheptyl or a cyclooctyl, optionally substituted by a group selected from a hydroxy group and a methoxy group,
   ○ a (C₃-C₇)heterocycloalkyl group, in particular a tetrahydropyranyl, a tetrahydrofuranyl, an oxepanyl or a dioxepanyl, optionally substituted by one or two groups selected from a hydroxy group and a methyl group, and
   ○ a heteroaryl group, in particular a pyridyl, a pyrazinyl, a pyrazolyl or a thiazolyl, optionally substituted by a methyl group, or
- a R"-L- group wherein
   ○ L is a (C₁-C₂)alkanediyl group, optionnally susbstituted by a group chosen from a hydroxy group, a -NH2 group and a methoxy group, and
   ○ R" is a phenyl group, optionally substituted by a trifluoromethyl, and R² is as defined above, or
   any of its pharmaceutically acceptable salts.

According to another particular embodiment, the present invention relates to a compound of formula (Ib) 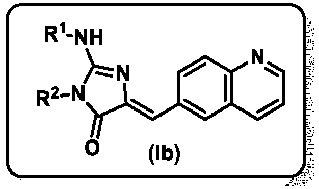 wherein R¹ represents:
- a (C₅-C₆)alkyl group substituted by a group selected from a hydroxy group and a methoxy group,
- a bridged (C₉-C₁₀)cycloalkyl group, in particular an adamantyl group or a noradamantyl gorup, optionally substituted by a hydroxy group,
- a R'-L- group, L is either a single bond or a methylene group, and
   R' is selected from the group consisting of:
   ○ a (C₆-C₇)cycloalkyl group in particular a cyclohexyl or a cycloheptyl,
   ○ a (C₅-C₆)heterocycloalkyl group, in particular a tetrahydropyranyl or a tetrahydrofuranyl, and
   ○ a heteroaryl group, in particular a pyrazinyl or a pyrazolyl, optionally substituted by a methyl group, or
- a R"-L- group wherein
   ○ L is a (C₁-C₂)alkanediyl group, optionnally susbstituted by a methoxy group, and
   ○ R" is a phenyl group, and
   R² is as defined above, or
any of its pharmaceutically acceptable salts.

According to another particular embodiment, the present invention relates to a compound of formula (Ic) 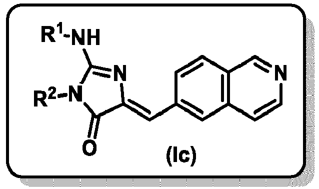 wherein R¹ represents:
- a (C₅-C₆)alkyl group substituted by a group selected from a hydroxy group and a methoxy group,
- an adamantyl group,
- a R'-L- group, L is either a single bond or a methylene group, and
   R' is selected from the group consisting of:
   ○ a (C₆-C₇)cycloalkyl group in particular a cyclohexyl or a cycloheptyl,
   ○ a (C₅-C₆)heterocycloalkyl group, in particular a tetrahydropyranyl or a tetrahydrofuranyl, and
   ○ a heteroaryl group, in particular a pyrazinyl or a pyrazolyl, optionally substituted by a methyl group, or
- a R"-L- group wherein
   ○ L is a (C₁-C₂)alkanediyl group, optionnally susbstituted by a methoxy group, and
   ○ R" is a phenyl group, and
   R² is as defined above, or
any of its pharmaceutically acceptable salts.

According to another particular embodiment, the present invention relates to a compound of formula (Id) 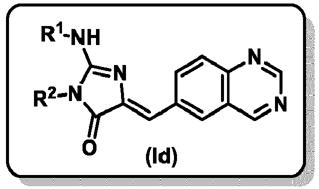 wherein R¹ represents:
- an adamantyl group, or
- a R"-L- group wherein
   ○ L is a (C₁-C₂)alkanediyl group, susbstituted by a methoxy group, and
   ○ R" is a phenyl group, and
   R² is as defined above, or
any of its pharmaceutically acceptable salts.

According to another particular embodiment, the present invention relates to a compound of formula (Ie) 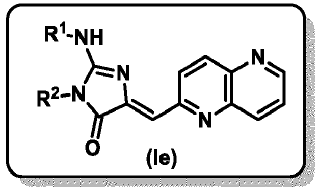 wherein R¹ represents:
- an adamantyl group, or
- a R"-L- group wherein
   ○ L is a (C₁-C₂)alkanediyl group, susbstituted by a methoxy group, and
   ○ R" is a phenyl group, and
   R² is as defined above, or
any of its pharmaceutically acceptable salts.

According to another particular embodiment, the present invention relates to a compound of formula (If) 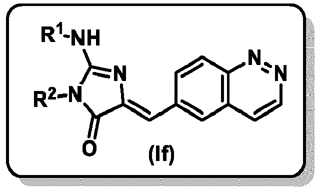 wherein R¹ represents:
- a R"-L- group wherein
   ○ L is a (C₁-C₂)alkanediyl group, susbstituted by a methoxy group, and
   ○ R" is a phenyl group, and
   R² is as defined above, or
any of its pharmaceutically acceptable salts.

According to another particular embodiment, the present invention relates to a compound of formula (Ig) 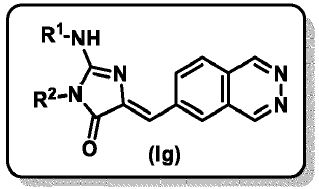 wherein R¹ represents:
- a R"-L- group wherein
   ○ L is a (C₁-C₂)alkanediyl group, susbstituted by a methoxy group, and
   ○ R" is a phenyl group, and
   R² is as defined above, or
   any of its pharmaceutically acceptable salts.

According to another particular embodiment, the present invention relates to a compound of formula (I) as defined herein above, wherein R¹ represents:
- a (C₄-C₆)alkyl group substituted by a group selected from a hydroxy group, a halogen and a (C₁-C₃)alkoxy, said (C₁-C₃)alkoxy group being optionally substituted by a phenyl group, said phenyl group being optionally substituted by a halogen atom,
- a bridged (C₆-C₁₀)cycloalkyl group, optionally substituted by a group selected from a (C₁-C₄)alkoxy group, a halogen atom and a hydroxy group,or any of its pharmaceutically acceptable salt, or
- a R'-L group, wherein
   ○ L is either a single bond or a (C₁-C₃)alkanediyl group and
   R' represents a (C₃-C₈)cycloalkyl group, optionally substituted by a group selected from a hydroxy group and a (C₁-C₃) alkoxy group,
or any pharmaceutically acceptable salt thereof.

Said sub-group of compounds are gathered under the "A1" type of compounds within the herein after table 1.

Still according to said embodiment, R¹ may more particularly represent a cyclohexyl, a cycloheptyl, a cyclooctyl, a 1-adamantyl, a 3-hydroxy-1-adamantyl, a 3-methoxy-1-adamantyl, a 1-(hydroxymethyl)-3-methyl-butyl, a 1-(methoxymethyl)-3-methyl-butyl, a 1-(ethoxymethyl)-3-methyl-butyl, a 1-(benzyloxymethyl)-3-methyl-butyl, a 1-(4-fluorobenzyloxymethyl)-3-methyl-butyl, a 2-methoxycyclopentyl, a 4-hydroxycycloheptyl, a 3-methoxycycloheptyl, a 4-methoxycycloheptyl, a 3-noradamantyl, 3-fluoro-1-adamantyl.

According to another particular embodiment, the present invention relates to a compound of formula (I) as defined herein above wherein R¹ represents:
- a fused phenyl group, selected from phenyl groups fused with a (C₅-C₆)cycloalkyl, which (C₅-C₆)cycloalkyl is substituted by a hydroxy group,
- a phenyl group, substituted by a (C₄-C₇)heterocycloalkyl group, said (C₄-C₇)heterocycloalkyl group being itself optionally substituted by a (C₁-C₄) alkyl group, or
- a R"-L- group, wherein
   ○ L is a (C₁-C₃)alkanediyl group, optionnally susbstituted by a group chosen from a hydroxy group, a (C₁-C₃)alkoxy group and a -NR^{b}R^{c} group, and
   ○ R" is a phenyl group, optionally substituted by a fluoro(C₁-C₄)alkyl group, wherein R^{b} and R^{c} are independently chosen from (C₁-C₆)alkyl and a hydrogen atom
or any pharmaceutically acceptable salt thereof.

Said sub-group of compounds are gathered under the "A2" and "A5" type of compounds within the herein after table 1.

Still according to said embodiment, R¹ may more particularly represent a benzyl, a [2-(trifluoromethyl)phenyl]methyl, a 2-hydroxyindan-1-yl, a 2-amino-1-phenyl-ethyl, a 2-hydroxy-1-phenyl-ethyl, a 2-methoxy-1-phenyl-ethyl, a 2-hydroxy-2-phenyl-ethyl, a 4-(4-methylpiperazin-1-yl)phenyl.

According to another particular embodiment, the present invention relates to a compound of formula (I) as defined herein above wherein R¹ represents a R'-L- group wherein:
- R' is a (C₃-C₈)heteroaryl group, optionally substituted by a (C₁-C₄)alkyl group and
- L is a (C₁-C₃)alkanediyl or a single bond,
or any pharmaceutically acceptable salt thereof.

Said sub-group of compounds are gathered under the "A3" and "A6" type of compounds within the herein after table 1.

Still according to said embodiment, R¹ may more particularly represent a (4-methylthiazol-2-yl)methyl, a (5-methylpyrazin-2-yl)methyl, 1-methylpyrazol-3-yl or a 2-pyridyl.

According to another particular embodiment, the present invention relates to a compound of formula (I) as defined herein above wherein R¹ represents a R'-L- group wherein:
- R' is a (C₃-C₉)heterocycloalkyl group, optionally substituted by one to two groups selected from a hydroxy group and a (C₁-C₄)alkyl, and
- L is a (C₁-C₃)alkanediyl or a single bond,
or any pharmaceutically acceptable salt thereof.

Said sub-group of compounds are gathered under the "A4" and "A7" type of compounds within the herein after table 1.

Still according to said embodiment, R¹ may more particularly a tetrahydropyran-4-yl-methyl, a tetrahydropyran-2-yl, a 6,6-dimethyltetrahydropyran-3-yl, a tetrahydrofuran-3-yl, a 4-hydroxytetrahydropyran-3-yl, an oxepan-3-yl or a dioxepan-6-yl.

In the context of the present invention, the term:
- "halogen" is understood to mean chlorine, fluorine, bromine, or iodine, and in particular denotes chlorine, fluorine or bromine,
- "(Cₓ-C_{y})alkyl", as used herein, respectively refers to a Cₓ-C_{y} normal, secondary or tertiary monovalent saturated hydrocarbon radical, for example (C₁-C₆)alkyl. Examples are, but are not limited to, methyl, ethyl, propyl, n-propyl, isopropyl, butyl, isobutyl, *sec*butyl, *tert-butyl,* pentyl, isopentyl, hexyl and isohexyl groups, and the like.
- "(C₁-C₃)alkanediyl", as used herein, refers to a divalent saturated hydrocarbon radical which is branched or linear, comprises from 1 to 3 carbon atoms, and more particularly a methylene, ethylene or propylene, such as linear propylene or isopropylene, said alkanediyl may be substituted as it is apparent from the following description.
- "(C₃-C₈)cycloalkyl", as used herein, refers to a cyclic saturated hydrocarbon, from 3 to 8 carbon atoms, saturated or partially unsaturated and unsubstituted or substituted. Examples are, but are not limited to, cyclopropyl, cyclobutyl, cyclopentyl, cyclohexyl, cycloheptyl and cyclooctyl.
- "(C₃-C₈)heterocycloalkyl group", as used herein, refers to a (C₃-C₈)cycloalkyl group wherein one or two of the carbon atoms are replaced with a heteroatom such as oxygen, nitrogen or sulphur, and more particularly such as an oxygen or a nitrogen atom. Such heterocycloalkyl group may be saturated or partially saturated and unsubstituted or substituted. Examples are, but are not limited to, morpholinyl, piperazinyl, piperidinyl, pyrrolidinyl, aziridinyl, oxanyl, oxetanyl, tetrahydropyranyl, morpholinyl, tetrahydrofuranyl, oxepanyl, diazepanyl, dioxanyl, dioxepanyl and tetrahydrothiopyranyl, and more particularly tetrahydropyranyl, tetrahydrofuranyl, oxepanyl and dioxepanyl .
- "(C₁-Cₓ)alkoxy", as used herein, refers to a -O-(C₁-Cₓ)alkyl or -O-(C₃-Cₓ)cycloalkyl moiety, wherein alkyl and cycloalkyl are as defined above, for example (C₁-C₆)alkoxy. Examples are, but are not limited to, methoxy, ethoxy, 1-propoxy, 2-propoxy, cyclopropoxy, butoxy, tert-butoxy and pentoxy.
- a "bridged (C₆-C₁₀)cycloalkyl" group, as used herein, refers to a bi- or tricyclic compound where the cycles are cycloalkyls, the rings share three or more atoms and the bridge contains at least one atom, for example 1, 2 or 3 atoms. Such bridged cycloalkyl groups may be substituted by one or more C₁-C₃ alkyl. Examples are, but not limited to adamantyl and noradamantyl.
- a "fused phenyl group" refers to a bicyclic radical that contains a phenyl moiety and may be substituted. Said fused phenyl group may be fused to a cycloalkyl or to a heterocycloalkyl and bound to the rest of the molecule either by its phenyl moeity or by said cycloalkyl or heterocycloalkyl. Examples are, but are not limited to indanyl, acetylindolinyl, methylindazolyl, hydroxyindanyl, benzothiazolyl, indolyl, indazolyl, methoxyindanyl and the like.
- a (C₅-C₁₁)heteroaryl group, as used herein, refers to a monocyclic aromatic group or to a bicyclic aromatic group where at least one of the ring is aromatic and wherein one to three ring carbon atom is replaced by a heteroatom, such as nitrogen, oxygen or sulphur. By way of examples of heteroaryl groups, mention may be made of, but not limited to: oxazole, isoxazole, pyridine, pyrimidine, pyridazine, triazine, pyrazine, oxadiazole, furane, pyrazole, thiazole, isothiazole, thiadiazole, imidazole, triazole and the like. In the framework of the present invention, the heteroaryl is advantageously pyridine, imidazole, pyrazine, furane, thiazole, pyrazole, thiadiazole, pyridazine and pyrimidine.
- an aromatic ring means, according to Hückel's rule, that a molecule has 4*n* + 2 π-electrons.
- a (C₁-Cₓ)fluoroalkyl group, as used herein, refers to a (C₁-Cₓ)alkyl as defined herein above in which one or more hydrogen atom(s) have been substituted by fluorines. In one embodiment all the hydrogen atoms are replaced by fluorine atoms, forming perfluoroalkyl groups, such as trifluoromethyl.

In the context of the present invention, the terms "aromatic ring", and "heteroaryl" include all the positional isomers.

The nomenclature of the following compounds (1) to (73) was generated according to the principles of the International Union of Pure and Applied Chemistry, using Accelrys Draw 4.1 SP1. To avoid any confusion, the "(±)" symbol added to designate a racemic mixture; *"cis"* and *"trans"* prefixes were also used to assign the relative stereochemistry of two adjacent chiral centers.

According to a preferred embodiment of the present invention, the compound of formula (I) is chosen from:
(1). (4Z)-2-(Cycloheptylamino)-4-(quinoxalin-6-ylmethylene)-1*H*-imidazol-5-one,
(2). (4Z)-2-(Cyclooctylamino)-4-(quinoxalin-6-ylmethylene)-1*H*-imidazol-5-one
(3). (4Z)-2-(Cyclohexylmethylamino)-4-(quinoxalin-6-ylmethylene)-1*H-*imidazol-5-one,
(4). (4Z)-2-[[(1*R*,2*R*)-2-Methoxycyclopentyl]amino]-4-(quinoxalin-6-ylmethylene)-1*H*-imidazol-5-one,
(5). (4Z)-2-[[(1*S*,2*S*)-2-Methoxycyclopentyl]amino]-4-(quinoxalin-6-ylmethylene)-1H-imidazol-5-one,
(6). (±)-(4Z)-2- [[*trans*-4-Hydroxycycloheptyl]amino] -4-(quinoxalin-6-ylmethylene)-1H-imidazol-5-one,
(7). (±)-(4Z)-2-[[*trans*-4-Methoxycycloheptyl]amino]-4-(quinoxalin-6-ylmethylene)-1H-imidazol-5-one,
(8). (±)-(4Z)-2-[[*cis*-3-Methoxycycloheptyl]amino]-4-(quinoxalin-6-ylmethylene)-1H-imidazol-5-one,
(9). (4*Z*)-2-[[(1*R*)-1-(Hydroxymethyl)-3-methyl-butyl]amino]-4-(quinoxalin-6-ylmethylene)-1*H*-imidazol-5-one,
(10). (4Z)-2-[[(1*R*)-1-(Methoxymethyl)-3-methyl-butyl]amino]-4-(quinoxalin-6-ylmethylene)-1*H*-imidazol-5-one,
(11). (4*Z*)-2-[[(1R)-1-(Ethoxymethyl)-3-methyl-butyl]amino]-4-(quinoxalin-6-ylmethylene)-1*H*-imidazol-5-one,
(12). (4*Z*)-2-[[(1*R)*-1-(Benzyloxymethyl)-3-methyl-butyl]amino]-4-(quinoxalin-6-ylmethylene)-1*H*-imidazol-5-one,
(13). (4Z)-2- [[(1*R*)-1-[(4-Fluorophenyl)methoxymethyl] -3-methyl-butyl]amino]-4-(quinoxalin-6-ylmethylene)-1*H*-imidazol-5-one,
(14). (4Z)-2-[[(1*R*)-1-(Fluoromethyl)-3-methyl-butyl]amino]-4-(quinoxalin-6-ylmethylene)-1*H*-imidazol-5-one,
(15). (4*Z*)-2-[[(1*S*)-1-(Fluoromethyl)-3-methyl-butyl]amino]-4-(quinoxalin-6-ylmethylene)-1*H*-imidazol-5-one,
(16). (4*Z*)-2-(3-Noradamantylamino)-4-(quinoxalin-6-ylmethylene)-1*H-*imidazol-5-one
(17). (4*Z*)-2-(1-Adamantylamino)-4-(quinoxalin-6-ylmethylene)-1*H-*imidazol-5-one,
(18). (4*Z*)-2-[(3-Hydroxy-1-adamantyl)amino]-4-(quinoxalin-6-ylmethylene)-1H-imidazol-5-one,
(19). (4Z)-2-[(3-Methoxy-1-adamantyl)amino4-(quinoxalin-6-ylmethylene)-1H-imidazol-5-one,
(20). (4*Z*)-2-[(3-Fluoro-1-adamantyl)amino4-(quinoxalin-6-ylmethylene)-1*H*-imidazol-5-one,
(21). (4*Z*)-2-(Benzylamino)-4-(quinoxalin-6-ylmethylene)-1*H*-imidazol-5-one,
(22). (4Z)-4-(Quinoxalin-6-ylmethylene)-2-[[2-(trifluoromethyl)phenyl]methylamino]-1*H*-imidazol-5-one,
(23). (4Z)-2-[[(1*S*,2*S*)-2-Hydroxyindan-1-yl]amino]-4-(quinoxalin-6-ylmethylene)-1H-imidazol-5-one,
(24). (4Z)-2-[[(1*R*)-2-Hydroxy-1-phenyl-ethyl]amino4-(quinoxalin-6-ylmethylene)-1H-imidazol-5-one,
(25). (4*Z*)-2-[[(1*S*)-2-Hydroxy-1-phenyl-ethyl]amino]-4-(quinoxalin-6-ylmethylene)- 1*H*-imidazol-5-one,
(26). (4*Z*)-2-[[(1*R*)-2-Methoxy-1-phenyl-ethyl]amino]-4-(quinoxalin-6-ylmethylene)-1H-imidazol-5-one,
(27). (4Z)-2-[[(2*R*)-2-Hydroxy-2-phenyl-ethyl]amino]-4-(quinoxalin-6-ylmethylene)-1H-imidazol-5-one,
(28). (4*Z*)-2-[[(1*R*)-2-Amino-1-phenyl-ethyl]amino]-4-(quinoxalin-6-ylmethylene)- 1*H*-imidazol-5-one dihydrochloride,
(29). (4Z)-2-[[(1*S*)-2-Amino-1-phenyl-ethyl]amino]-4-(quinoxalin-6-ylmethylene)- 1*H*-imidazol-5-one dihydrochloride,
(30). (4Z)-2-[(5-Methylpyrazin-2-yl)methylamino]-4-(quinoxalin-6-ylmethylene)-1H-imidazol-5-one,
(31). (4Z)-2-[(4-Methylthiazol-2-yl)methylamino]-4-(quinoxalin-6-ylmethylene)-1H-imidazol-5-one,
(32). (4Z)-4-(Quinoxalin-6-ylmethylene)-2-(tetrahydropyran-4-ylmethylamino) -1*H*-imidazol-5-one,
(33). (4Z)-2-[4-(4-Methylpiperazin-1yl)anilino]-4-(quinoxalin-6-ylmethylene)-1H-imidazol-5-one,
(34). (4*Z*)-2-[(1-Methylpyrazol-3-yl)amino]-4-(quinoxalin-6-ylmethylene)-1*H*-imidazol-5-one,
(35). (4*Z*)-2-(2-Pyridylamino)-4-(quinoxalin-6-ylmethylene)-1*H*-imidazol-5-one,
(36). (±)-(4*Z*)-2-[(6,6-Dimethyltetrahydropyran-3-yl)amino]-4-(quinoxalin-6-ylmethylene)-1H-imidazol-5-one,
(37). (4*Z*)-4-(Quinoxalin-6-ylmethylene)-2-[[(3*R*)-tetrahydrofuran-3-yl] amino] -1*H*-imidazol-5-one,
(38). (4*Z*)-4-(Quinoxalin-6-ylmethylene)-2-[[(3*S*)-tetrahydropyran-3-yl] amino] -1*H*-imidazol-5-one,
(39). (4*Z*)-4-(Quinoxalin-6-ylmethylene)-2-[[(3*R*)-tetrahydropyran-3-yl] amino] -1*H*-imidazol-5-one,
(40). (4Z)-2-[[(3*R*,4*R*)-4-Hydroxytetrahydropyran-3-yl]amino]-4-(quinoxalin-6-ylmethylene)-1*H*-imidazol-5-one,
(41). (4*Z*)-2-(Oxepan-3-ylamino)-4-(quinoxalin-6-ylmethylene)-1*H-*imidazol-5-one,
(42). (4Z)-2-(1,4-Dioxepan-6-ylamino)-4-(quinoxalin-6-ylmethylene)-1*H-*imidazol-5-one,
(43). (4Z)-2-(Cyclohexylamino)-4-(6-quinolylmethylene)-1*H*-imidazol-5-one,
(44). (4*Z*)-2-(Cycloheptylamino)-4-(6-quinolylmethylene)-1*H*-imidazol-5-one,
(45). (4*Z*)-2-(Cyclohexylmethylamino)-4-(6-quinolylmethylene)-1*H-*imidazol-5-one,
(46). (4Z)-2-[[(1*R*)-1-(Hydroxymethyl)-3-methyl-butyl]amino]-4-(6-quinolylmethylene)-1*H*-imidazol-5-one,
(47). (4*Z*)-2-[[(1*R*)-1-(Methoxymethyl)-3-methyl-butyl]amino]-4-(6-quinolylmethylene)-1*H*-imidazol-5-one,
(48). (4*Z*)-2-(3-Noradamantylamino)-4-(6-quinolylmethylene)-1*H*-imidazol-5-one,
(49). (4*Z*)-2-(1-Adamantylamino)-4-(6-quinolylmethylene)-1*H*-imidazol-5-one,
(50). (4Z)-2-[(3-Hydroxy-1-adamantyl)amino]-4-(6-quinolylmethylene)-1*H-*imidazol-5-one,
(51). (4*Z*)-2-(Benzylamino)-4-(6-quinolylmethylene)-1*H*-imidazol-5-one,
(52). (4*Z*)-2-[[(1*R*)-2-Methoxy-1-phenyl-ethyl]amino]-4-(6-quinolylmethylene)-1*H*-imidazol-5-one,
(53). (4*Z*)-2-[(5-Methylpyrazin-2-yl)methylamino]-4-(6-quinolylmethylene)-1*H*-imidazol-5-one,
(54). (4*Z*)-2-[(1-Methylpyrazol-3-yl)amino]-4-(6-quinolylmethylene)-1*H-*imidazol-5-one,
(55). (4Z)-4-(6-Quinolylmethylene)-2-[[(3*R)*-tetrahydrofuran-3-yl]amino]-1*H*-imidazol-5-one,
(56). (4Z)-4-(6-Quinolylmethylene)-2-[[(3*R*)-tetrahydropyran-3-yl]amino]-1*H*-imidazol-5-one,
(57). (4*Z*)-2-(Cycloheptylamino)-4-(6-isoquinolylmethylene)-1*H*-imidazol-5-one,
(58). (4*Z*)-2-(Cyclohexylmethylamino)-4-(6-isoquinolylmethylene)-1*H-*imidazol-5-one,
(59). (4*Z*)-2-[[(1*R*)-1-(Hydroxymethyl)-3-methyl-butyl]amino]-4-(6-isoquinolylmethylene)-1*H*-imidazol-5-one,
(60). (4*Z*)-4-(6-Isoquinolylmethylene)-2-[[(1*R*)-1-(methoxymethyl)-3-methyl-butyl] amino]-1*H*-imidazol-5-one,
(61). (4Z)-2-(1-Adamantylamino)-4-(6-isoquinolylmethylene)-1*H*-imidazol-5-one,
(62). (4Z)-2-(Benzylamino)-4-(6-isoquinolylmethylene)-1*H*-imidazol-5-one,
(63). (4*Z*)-4-(6-Isoquinolylmethylene)-2-[[(1*R*)-2-methoxy-1-phenylethyl]amino]-1*H*-imidazol-5-one,
(64). (4Z)-4-(6-Isoquinolylmethylene)-2-[(5-methylpyrazin-2-yl)methylamino]-1*H*-imidazol-5-one,
(65). (4*Z*)-4-(6-Isoquinolylmethylene)-2-[(1-methylpyrazol-3-yl)amino]-1*H-*imidazol-5-one,
(66). (4Z)-4-(6-Isoquinolylmethylene)-2-[[(3*R*)-tetrahydrofuran-3-yl]amino]-1*H*-imidazol-5-one,
(67). (4*Z)*-4-(6-Isoquinolylmethylene)-2-[[(3*R*)-tetrahydropyran-3-yl]amino]-1*H*-imidazol-5-one,
(68). (4*Z*)-2-(1-Adamantylamino)-4-(quinazolin-6-ylmethylene)-1*H-*imidazol-5-one,
(69). (4Z)-2-[[(1*R*)-2-Methoxy-1-phenyl-ethyl]amino]-4-(quinazolin-6-ylmethylene)-1H-imidazol-5-one,
(70). (4*Z*)-2-(1-Adamantylamino)-4-(1,5-naphthyridin-2-ylmethylene)-1*H-*imidazol-5-one,
(71). (4Z)-2-[[(1*R*)-2-Methoxy-1-phenyl-ethyl]amino4-(1,5-naphthyridin-2-ylmethylene)-1H-imidazol-5-one,
(72). (4Z)-4-(Cinnolin-6-ylmethylene)-2-[[(1*R*)-2-methoxy-1-phenylethyl]amino]-1*H*-imidazol-5-one,
(73). (4Z)-2-[[(1*R*)-2-Methoxy-1-phenyl-ethyl]amino4-(phthalazin-6-ylmethylene)-1H-imidazol-5-one,
and their pharmaceutically acceptable salts.

According to an even more preferred embodiment of the present invention, the compound of formula (I) is chosen from the group consisting of compounds (1), (2), (4), (5), (6), (8), (10), (11), (13), (22), (24), (28), (29), (33), (35), (38), (39), (41), (47), (52), (60), (61) and their pharmaceutically acceptable salts.

According to an even more preferred embodiment of the present invention, the compound of formula (I) is chosen from the group consisting of compounds (1), (9), (10), (11), (14), (16), (17), (18), (19), (20), (23), (24), (28), (29), (33), (39), (46), (47), (48), (49), (50), (61), (69), (70) and their pharmaceutically acceptable salts.

According to an even more preferred embodiment of the present invention, the compound of formula (I) is chosen from the group consisting of compounds (9), (16), (18), (19), (20), (48), (49), (50), (69), (70) and their pharmaceutically acceptable salts.

According to an alternative embodiment of the present invention, the compound of formula (I) is chosen from the group consisting of (2), (5), (9), (16), (17), (18), (19), (20), (21), (22), (23), (24), (28), (29), (33), (37), (38), (39), (41), (44), (48), (49), (50), (52), (61), (68), (69), (70), (71) and their pharmaceutically acceptable salts.

According to a preferred embodiment of the present invention, the compound of formula (I) is chosen from the group consisting of compounds (9), (17), (18), (19), (20), (24), (28), (48), (49), (50), (61), (69), (70), (71) and their pharmaceutically acceptable salts.

According to an even more preferred embodiment of the present invention, the compound of formula (I) is chosen from the group consisting of compound (70) and its pharmaceutically acceptable salts.

According to a further embodiment of the present invention, the compound of formula (I) is chosen from the group consisting of compounds (16), (18), (19), (20), (48), (50), (69), (70), (71) and their pharmaceutically acceptable salts.

The groups of compounds as defined by the list of compounds as identified in Example 4 below through tables 4A to table 4F and specifically identified as most potent kinase inhibitors, as well as multi-target kinase inhibitors, also form part of the present invention.

According to another aspect, a subject-matter of the present invention relates to a compound of formula (I) as defined above or any of its pharmaceutically acceptable salts, or at least any of compounds (1) to (73) or any of its pharmaceutically acceptable salts, for use as a medicament.

"Pharmaceutically acceptable salt thereof" refers to salts which are formed from acid addition salts formed with inorganic acids (e.g. hydrochloric acid, hydrobromic acid), as well as salts formed with organic acids such as acetic acid, tartaric acid, succinic acid.

Suitable physiologically acceptable acid addition salts of compounds of formula (I) include hydrobromide, tartrate, hydrochloride, succinate and acetate.

The compounds of formula (I), and any of compounds (1) to (73) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, *i.e*. combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention. In particular, stereoisomers (Z) and (E) form part of the invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

**List of abbreviations:**

| **Abbreviation/acronym** | **name** |
|---|---|
| **Ac** | Acetyl |
| **ACN** | Acetonitrile |
| **Alk** | Alkyl |
| **app t** | Apparent triplet |
| **ATP** | Adenosine triphosphate |
| **br** s | Broad singlet |
| **c** | Molar concentration |
| **Cpd N°** | Compound number |
| **d** | Doublet |
| **DCM** | Methylene chloride |
| **dd** | Doublet of doublets |
| **DIPEA** | *N,N-*Diisopropylethylamine |
| **DMF** | Dimethylformamide |
| **DMSO** | Dimethylsulfoxide |
| **DTT** | Dithiothreitol |
| **Eq** | Equivalent |
| **ESI** | Electrospray ionization |
| **Et** | Ethyl |
| **FC** | Flash chromatography |
| **GP** | General protocol |
| **GST** | Glutathione S-transferase |
| **Hal** | Halogen |
| **HEPES** | 4-(2-Hydroxyethyl)-1-piperazineethanesulfonic acid |
| **His-tagged** | Polyhistidine-tagged |
| **HPLC** | High pressure liquid chromatography |
| **ICso** | Half maximal inhibitory concentration |
| **m** | Multiplet |
| **M** | Molarity |
| **Me** | Methyl |
| **MS** | Mass spectroscopy |
| **MW** | Molecular weight |
| **NMR** | Nuclear magnetic resonance |
| **N/A** | Not applicable |
| **n.t.** | Not tested |
| **PTLC** | Preparative thin layer chromatography |
| **Rac** | Racemic |
| **r.t.** | Room temperature |
| **s** | Singlet |
| **SDS-PAGE** | Sodium dodecyl sulfate-polyacrylamide gel electrophoresis |
| **t** | Triplet |
| **THF** | Tetrahydrofuran |
| **TLC** | Thin layer chromatography |
| **T** °C | Temperature in degrees Celsius |
| **UV** | Ultraviolet |
| **v/v** | Volume per volume |
| **w/v** | Weight per volume |
| **δ_{H}** | Hydrogen chemical shift |
| **µw** | Microwave irradiation |

The compounds of general formula (I) can be prepared according to schemes 1 and 2 below.

### Late-stage Knoevenagel between N2-functionalized 2-amino-1,4-dihydroimidazol-5-ones and heteroarylcarboxaldehydes (ROUTE 1)

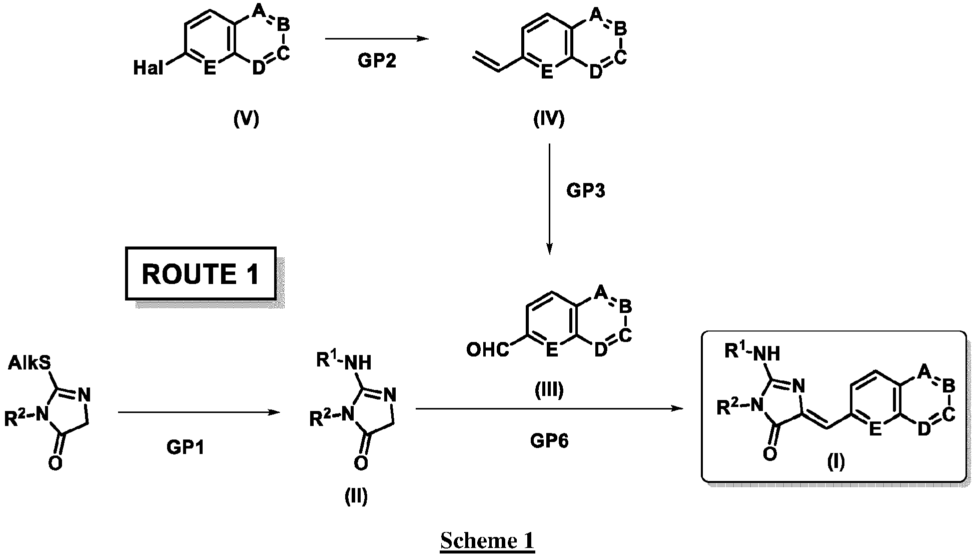

### Late-stage SNAr on (4Z)-4-heteroaryl-2-alkylsulfanyl-1H-imidazol-5-ones with aliphatic and aromatic amines (ROUTE 2)

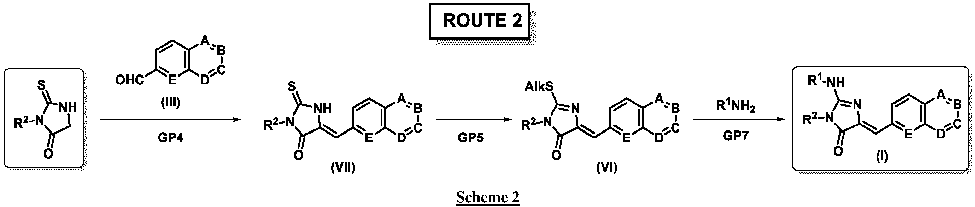

### ROUTE 1

The synthesis is based on late-stage Knoevenagel reaction between a *N2-*substituted-1,4-dihydroimidazol-5-one of formula (II) and a heteroarylcarboxaldehyde of formula (III) wherein R¹, R², A, B, C, D, and E are as defined above, following the general protocol 6 (GP6), described herein after.

According to GP6, the compounds (II) and (III) are placed in a protic solvent such as ethanol. Compounds (II) and (III) may be introduced for example in a molar ratio of compound (III) on compound (II) ranging from 1 to 5, for example of 1.2, in presence of an ammonium carboxylate such as ammonium formate, for example in a molar ratio with respect to compound (II) ranging from 1 to 5, for example of 1.2. The mixture may be irradiated, for example by microwaves, for example for a duration ranging from 1 to 5 hours, in particular 3 hours. The mixture may also be heated for example at a temperature ranging from 100 to 140°C, for example from 110 to 130°C and brought back to room temperature upon completion of the reaction.

The compound of formula (II) as defined above comprises an aliphatic or aromatic amine, it may be obtained by the addition of an amine of formula R¹NH₂ on a 2-alkylsulfanyl-1,4-dihydroimidazol-5-one, wherein R¹ and R² are as defined above and Alk is a (C₁-C₅)alkyl, following to the general protocol 1 (GP1) described herein after.

According to GP1, the 2-alkylsulfanyl-1,4-dihydroimidazol-5-one may be placed in an aprotic solvent such as tetrahydrofuran (THF). Said amine of formula R¹NH₂ may then be added, for example in a molar ratio ranging from 1.2 to 6, with respect to the 2-alkylsulfanyl-1,4-dihydroimidazol-5-one. Acetic acid may be added to the reaction mixture, in a molar ratio ranging from 2 to 5. The reaction mixture may be placed in a sealed tube, stirred and heated to a temperature ranging from 90°C to 130°C, for example of 110°C. Upon completion of the reaction, the mixture may be brought back to room temperature and a solid may crystallize. The mixture may then be stirred at a temperature ranging from -10°C to 10°C, for example 0°C, for a duration ranging from 30 to 90 minutes, for example of 60 minutes.

### ROUTE 2

The synthesis is based on a late-stage functionalization of a compound of formula (VI) by an amine of formula R¹NH₂ wherein Alk, R¹, R², A, B, C, D and E are as defined above, following the general protocol 7 (GP7), described herein after.

According to GP7, the compound of formula (VI) may be placed in an aprotic solvent such as THF or dioxane, or a mixture of both. The amine of formula R¹NH₂ may be added, for example in a molar ratio ranging from 2 to 6, in particular of 4, with respect to the compound of formula (VI). Acetic acid may be added to the reaction mixture, in a molar ratio with respect to the compound of formula (VI) ranging for example from 4 to 8. The reaction mixture may be placed in a sealed tube and may receive energy, for example from a heating block or from microwaves. Upon completion of the reaction, the mixture may be brought back to room temperature.

In an embodiment named GP7-A, the reaction mixture may be stirred at a temperature ranging from -10°C to 10°C, for example at 0°C, for a duration ranging from 30 minutes to 2 hours, for example for 1 hour.

Depending on the state of the product obtained (solid, precipitate), purification methods well-known by the person skilled in the art may be performed, for example filtering, washing, triturating, drying *in vacuo,* flash chromatography, precipitating and refluxing.

A compound of formula (VI) as defined above may be obtained by the S-alkylation of a compound of formula (VII) wherein R², A, B, C, D and E are as defined above, following the general protocol 5 (GP5).

According to GP5, a compound of formula (VII) may be placed in a polar aprotic solvent such as dimethylformamide (DMF). An alkylhalide of formula Alk-Hal wherein Hal is an halide such as iodide or bromide and Alk is a (C₁-C₅)alkyl such as methyl or ethyl may then be added dropwise, for example in a molar ratio ranging from 0.75 to 1.50, in particular of 1.05, with respect to the compound of formula (VII), in presence of an inorganic base such as K2CO3, for example in a molar ratio ranging from 0.7 to 1.5, in particular of 1, still with respect to the compound of formula (VII). The reaction mixture may be stirred during the addition of the alkylhalide. The resulting mixture may then be stirred, for example from 8 to 16 hours, in particular for 12 hours at a temperature ranging from -10°C to 30°C, in particular at 0°C when Alk is a methyl or at room temperature when Alk is an ethyl.

A compound of formula (VII) as defined above may be obtained from a compound of formula (III) wherein R², A, B, C, D and E are as defined above, following the general protocol 4 (GP4).

According to GP4, the compound of formula (III) may be placed in a protic solvent such as ethanol in presence of 2-thiohydantoin, for example in a molar ratio ranging from 0.85 to 1.15, in particular of 1, with respect to the compound of formula (III), in presence of an organic base such as piperidine or ethanolamine for example in a molar ratio ranging from 0.85 to 1.15, in particular of 1, still with respect to the compound of formula (III), in presence of an organic acid such as acetic acid for example in a molar ratio ranging from 0.85 to 1.15, in particular of 1, still with respect to the compound of formula (III). The reaction mixture may be placed in a sealed tube, stirred and heated at a temperature ranging for example from 60°C to 130°C, in particular from 80°C to a duration ranging from 10 to 100 minutes, in particular from 15 to 90 minutes. The reaction mixture may be irradiated, for example by microwaves.

In both routes of synthesis described herein above, a compound of formula (III), or heteroarylcarboxaldehyde, may be implemented.

A compound of formula (III) as defined above may be obtained by a vinylation of a heteroarylbromide of formula (V) wherein A, B, C, D and E are as defined above and Hal is a halogen atom, following the general protocol 2 (GP2) followed by at least a step of a Lemieux-Johnson oxidation, according to the general protocol 3 (GP3).

According to one embodiment, GP2 implements a palladium-catalyzed vinylation of a heteroarylbromide. The compound of formula (V) may be placed in a mixture together with potassium vinyltrifluoroborate, in presence of an inorganic base such as Cs₂CO₃ and of a source of Pd[0], in a solvent mixture containing at least a solvent able to stabilize cations, such as dioxane.

According to GP2, the palladium source is a tetrakis(triphenylphosphine)palladium(0) (Pd(Ph3)4). In this embodiment, the compound of formula (V) may be placed in a mixture together with potassium vinyltrifluoroborate in a molar ratio with respect to the compound of formula (V) ranging for example from 1 to 1.4, in particular of 1.2, in presence of an inorganic base such as Cs₂CO₃, for example in a molar ratio ranging from 1 to 5, in particular 2, still with respect to the compound of formula (V), in the presence of Pd(PPh3)4 for example in an amount ranging from 2 to 10 mol%, in particular of 5 mol% relative to the total amount of compound of formula (V) and in a solvent mixture containing at least a solvent able to stabilize cations, such as dioxane. The mixture may be brought to reflux and heated for example for 6 to 18 hours, in particular 12 hours.

According to the general protocol GP3, the vinylheteroaryl (IV) obtained from protocol GP2 and wherein A, B, C, D and E are as defined above, may be placed in a solvent mixture containing at least a solvent able to stabilize cations, such as dioxane. An oxidising agent such as periodate may then be added, for example in a molar ratio ranging from 2 to 6, in particular 4, with respect to the vinylheteroaryl (IV) obtained from protocol GP2 in presence of an osmium compound such as osmium tetroxide (OsO₄), for example in an amount ranging from 1 to 10 mol%, in particular 5 mol%, relative to the total amount of vinylheteroaryl (IV) obtained from protocol GP2, in the presence of a non-nucleophilic base such as 2,6-lutidine, for example in a molar ratio ranging from 2 to 6, in particular 4, still with respect to the vinylheteroaryl (IV) obtained from protocol GP2. The reaction mixture may be stirred and maintained at a temperature for example ranging from -5°C to 5°C, in particular 0°C, for example for a duration ranging from 30 minutes to 2 hours.

Accordingly, the present invention further relates to the synthesis process for manufacturing new compounds of formula (I) as defined above, comprising at least a step of coupling a compound of formula (III) with a compound of formula (II).

The present invention relates to a synthesis process for manufacturing a compound of formula (I) as defined above or any of its pharmaceutically acceptable salt or any of the compounds (1) to (73) as defined above or any of their pharmaceutically acceptable salts, comprising at least a step of coupling a compound of formula (II) below 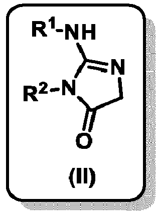
wherein R¹ and R² is as defined above,
with a compound of formula (III) below 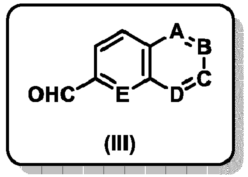
wherein A, B, C, D and E are as defined above.

The present invention further relates to a synthetic intermediate of formula (II) below 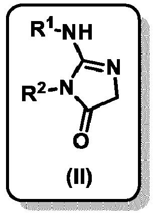 wherein R¹ and R² is as defined above.

The present invention further relates to a synthetic intermediate of formula (III) below 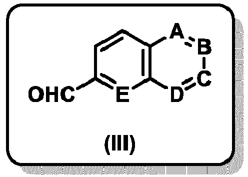 wherein A, B, C, D and E are as defined above.

Accordingly, the present invention further relates to the synthesis process for manufacturing new compounds of formula (I) as defined above, comprising at least a step of substituting a compound of formula (VI) with a primary amine.

The present invention relates to a synthesis process for manufacturing a compound of formula (I) as defined above or any of its pharmaceutically acceptable salt or any of the compounds (1) to (73) as defined above or any of their pharmaceutically acceptable salts, comprising at least a step of coupling a compound of formula (VI) below 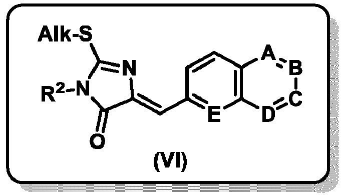 wherein R², A, B, C, D and E are as defined above and Alk is a (C₁-C₅)alkyl, with an amine of formula R¹NH₂ wherein R¹ is as defined above.

The present invention further relates to synthetic intermediates of formula (VI) below 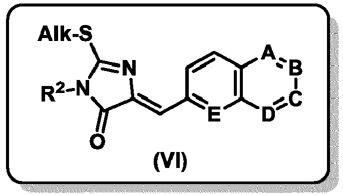 wherein R², A, B, C, D and E are as defined above and Alk is a (C₁-C₅)alkyl, in particular Alk is selected from the group consisting of an ethyl and a methyl.

The chemical structures, the analytical and spectroscopic data of some compounds of formula (I) of the invention are illustrated respectively in the following Table 2 and Table 3.

Reactions were performed using oven-dried glassware under inert atmosphere of argon. Unless otherwise noted, all reagent-grade chemicals and solvents were obtained from commercial suppliers and were used as received. Reactions were monitored by thinlayer chromatography with silica gel 60 F254 pre-coated aluminium plates (0.25 mm). Visualization was performed under UV light and 254 or 312 nm, or with appropriate TLC stains including, but not limited to: phosphomolybdic acid, KMnO₄, ninhydrin, CAM, vanillin, *p*-anisaldehyde.

Microwave experiments were conducted in an Anton Paar Monowave 400^{®} microwave reactor. The experiments were conducted in a monomode cavity with a power delivery ranging from 0 to 850 W, allowing pressurized reactions (0 to 30 bars) to be performed in sealed glass vials (4 to 30 mL) equipped with snap caps and silicon septa. The temperature (0 to 300 °C) was monitored by a contactless infrared sensor and calibrated with a ruby thermometer. Temperature, pressure, and power profiles were edited and monitored through a touch-screen control panel. Times indicated in the various protocols are the times measured when the mixtures reached the programmed temperature after a ramp period of 3 min.

Chromatographic purifications of compounds were achieved on an automated Interchim Puriflash XS420 equipped with 30 µm spherical silica-filled prepacked columns as stationary phase.

Some compounds of the invention are described with their structure in the below Table 2, which is merely illustrative and does not limit the scope of the present invention.

**Table 2: Structure of compounds (1) to (73). Formulas and molecular weights were generated using Perkin Elmer's Chemdraw^{®} version 17.1.0.105.**

| 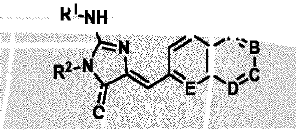 | | | | | | | |
|---|---|---|---|---|---|---|---|
| **Cpd** | **R¹-NH-** | **R²** | 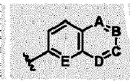 | **Class** | **Formula** | **MW** | **chemisty** |
| **1** | 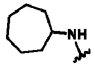 | H | 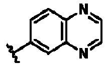 | A1 | C₁₉H₂₁N₅O | 335.41 | N/A |
| **2** | 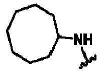 | H | 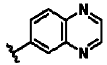 | A1 | C₂₀H₂₃N₅O | 349.44 | N/A |
| **3** | 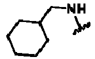 | H | 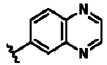 | A1 | C₁₉H₂₁N₅O | 335.41 | N/A |
| **4** | 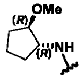 | H | 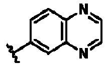 | A1 | C₁₈H₁₉N₅O₂ | 337.38 | (1*R,*2*R*) |
| **5** | 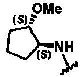 | H | 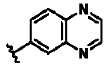 | A1 | C₁₈H₁₉N₅O₂ | 337.38 | (1*S*,2*S*) |
| **6** | 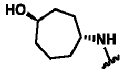 | H | 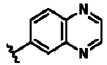 | A1 | C₁₉H₂₁N₅O₂ | 351.41 | *Trans-rac* |
| **7** | 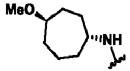 | H | 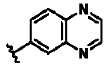 | A1 | C₂₀H₂₃N₅O₂ | 365.44 | *Trans-*rac |
| **8** | 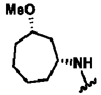 | H | 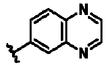 | A1 | C₂₀H₂₃N₅O₂ | 365.44 | *Cis-*rac |
| **9** | 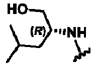 | H | 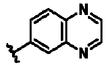 | A1 | C₁₈H₂₁N₅O₂ | 339.40 | (1*R*) |
| **10** | 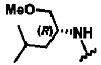 | H | 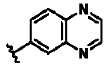 | A1 | C₁₉H₂₃N₅O₂ | 353.43 | (1*R*) |
| **11** | 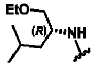 | H | 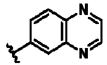 | A1 | C₂₀H₂₅N₅O₂ | 367.45 | (1*R*) |
| **12** | 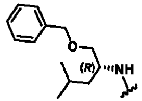 | H | 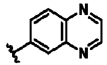 | A1 | C₂₅H₂₇N₅O₂ | 429.52 | *(1R)* |
| **13** | 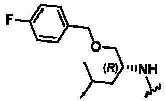 | H | 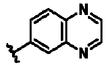 | A1 | C₂₅H₂₆FN₅O₂ | 447.51 | *(1R)* |
| **14** | 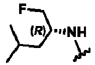 | H | 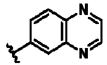 | A1 | C₁₈H₂₀FN₅O | 341.39 | *(1R)* |
| **15** | 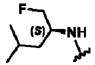 | H | 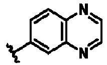 | A1 | C₁₈H₂₀FN₅O | 341.39 | (1*S*) |
| **16** | 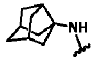 | H | 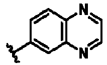 | A1 | C₂₁H₂₁N₅O | 359.43 | N/A |
| **17** | 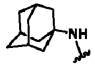 | H | 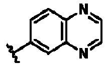 | A1 | C₂₂H₂₃N₅O | 373.46 | N/A |
| **18** | 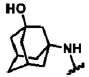 | H | 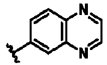 | A1 | C₂₂H₂₃N₅O₂ | 389.46 | N/A |
| **19** | 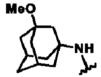 | H | 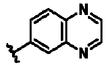 | A1 | C₂₃H₂₅N₅O2 | 403.49 | N/A |
| **20** | 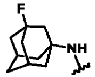 | H | 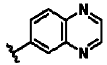 | A1 | C₂₂H₂₂FN₅O | 391.45 | N/A |
| **21** | 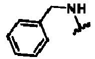 | H | 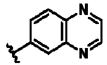 | A2 | C₁₉H₁₅N₅O | 329.36 | N/A |
| **22** | 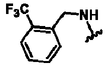 | H | 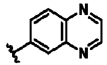 | A2 | C₂₀H₁₄F₃N₅O | 397.36 | N/A |
| **23** | 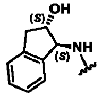 | H | 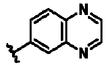 | A2 | C₂₁H₁₇N₅O₂ | 371.40 | (1*S*,2*S*) |
| **24** | 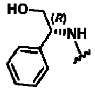 | H | 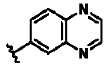 | A2 | C₂₀H₁₇N₅O₂ | 359.39 | *(1R)* |
| **25** | 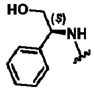 | H | 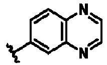 | A2 | C₂₀H₁₇N₅O₂ | 359.39 | *(1R)* |
| **26** | 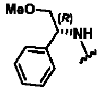 | H | 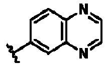 | A2 | C₂₁H₁₉N₅O₂ | 373.42 | (1*R*) |
| **27** | 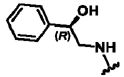 | H | 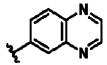 | A2 | C₂₀H₁₇N₅O₂ | 359.39 | *(2R)* |
| **28** | 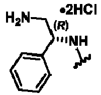 | H | 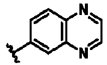 | A2 | C₂₀H₂₀Cl₂N₆O | 431.32 | (1*R*) |
| **29** | 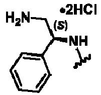 | H | 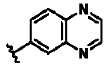 | A2 | C₂₀H₂₀Cl₂N₆O | 431.32 | (1*R*) |
| **30** | 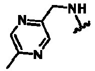 | H | 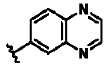 | A3 | C₁₈H₁₅N₇O | 345.37 | N/A |
| **31** | 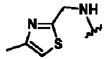 | H | 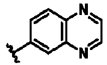 | A3 | C₁₇H₁₄N₆OS | 350.40 | N/A |
| **32** | 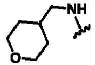 | H | 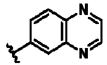 | A4 | C₁₈H₁₉N₅O₂ | 337.38 | N/A |
| **33** | 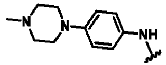 | H | 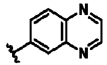 | A5 | C₂₃H₂₃N₇O | 413.49 | N/A |
| **34** | 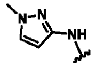 | H | 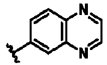 | A6 | C₁₆H₁₃N₇O | 319.33 | N/A |
| **35** | 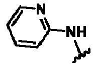 | H | 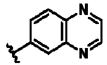 | A6 | C₁₇H₁₂N₆O | 316.32 | N/A |
| **36** | 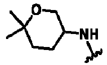 | H | 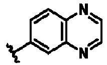 | A7 | C₁₉H₂₁N₅O₂ | 351.41 | rac |
| **37** | 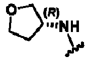 | H | 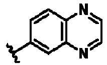 | A7 | C₁₆H₁₅N₅O₂ | 309.33 | (3*R*) |
| **38** | 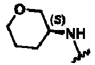 | H | 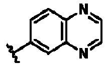 | A7 | C₁₇H₁₇N₅O₂ | 323.36 | (3*S*) |
| **39** | 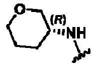 | H | 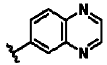 | A7 | C₁₇H₁₇N₅O₂ | 323.36 | (3*R*) |
| **40** | 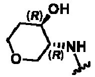 | H | 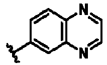 | A7 | C₁₇H₁₇N₅O₃ | 339.36 | (3*R*,4*R*) |
| **41** | 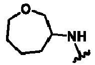 | H | 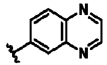 | A7 | C₁₈H₁₉N₅O₂ | 337.38 | rac |
| **42** | 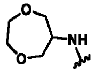 | H | 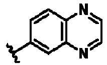 | A7 | C₁₇H₁₇N₅O₃ | 339.36 | N/A |
| **43** | 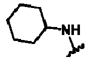 | H | 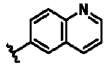 | A1 | C₁₉H₂₀N₄O | 320.40 | N/A |
| **44** | 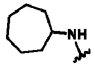 | H | 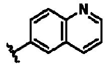 | A1 | C₂₀H₂₂N₄O | 334.42 | N/A |
| **45** | 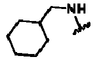 | H | 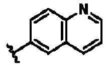 | A1 | C₂₀H₂₂N₄O | 334.42 | N/A |
| **46** | 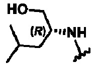 | H | 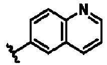 | A1 | C₉H₂₂N₄O₂ | 338.41 | *(1R)* |
| **47** | 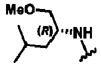 | H | 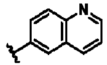 | A1 | C₂₀H₂₄N₄O₂ | 352.44 | *(1R)* |
| **48** | 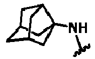 | H | 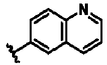 | A1 | C₂₂H₂₂N₄O | 358.45 | N/A |
| **49** | 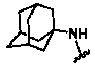 | H | 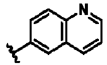 | A1 | C₂₃H₂₄N₄O | 372.47 | N/A |
| **50** | 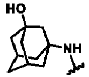 | H | 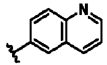 | A1 | C₂₃H₂₄N₄O₂ | 388.47 | N/A |
| **51** | 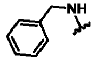 | H | 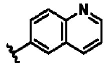 | A2 | C₂₀H₁₆N₄O | 328.38 | N/A |
| **52** | 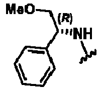 | H | 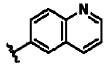 | A2 | C₂₂H₂₀N₄O₂ | 372.43 | *(1R)* |
| **53** | 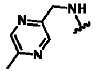 | H | 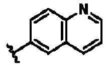 | A3 | C₁₉H₁₆N₆O | 344.38 | N/A |
| **54** | 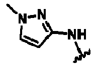 | H | 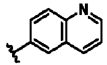 | A6 | C₁₇H₁₄N₆O | 318.34 | N/A |
| **55** | 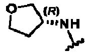 | H | 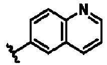 | A7 | C₁₇H₁₆N₄O₂ | 308.34 | (3*R*) |
| **56** | 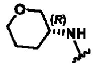 | H | 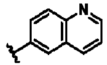 | A7 | C₁₈H₁₈N₄O₂ | 322.37 | (3*R*) |
| **57** | 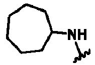 | H | 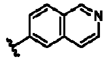 | A1 | C₂₀H₂₂N₄O | 334.42 | N/A |
| **58** | 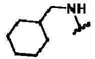 | H | 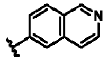 | A1 | C₂₀H₂₂N₄O | 334.42 | N/A |
| **59** | 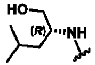 | H | 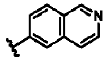 | A1 | C₁₉H₂₂N₄O₂ | 338.41 | *(1R)* |
| **60** | 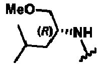 | H | 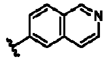 | A1 | C₂₀H₂₄N₄O₂ | 352.44 | *(1R)* |
| **61** | 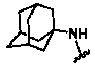 | H | 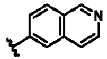 | A1 | C₂₃H₂₄N₄O | 372.47 | N/A |
| **62** | 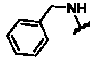 | H | 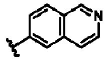 | A2 | C₂₀H₁₆N₄O | 328.38 | N/A |
| **63** | 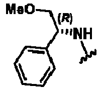 | H | 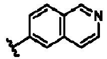 | A2 | C₂₂H₂₀N₄O₂ | 372.43 | *(1R)* |
| **64** | 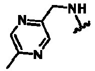 | H | 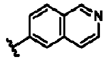 | A3 | C₁₉H₁₆N₆O | 344.38 | N/A |
| **65** | 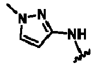 | H | 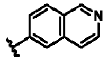 | A6 | C₁₇H₁₄N₆O | 318.34 | N/A |
| **66** | 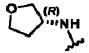 | H | 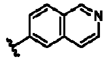 | A7 | C₁₇H₁₆N₄O₂ | 308.34 | *(3R)* |
| **67** | 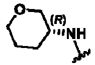 | H | 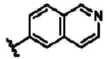 | A7 | C₁₈H₁₈N₄O₂ | 322.37 | (3*R*) |
| **68** | 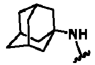 | H | 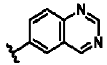 | A1 | C₂₂H₂₃N₅O | 373.46 | N/A |
| **69** | 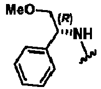 | H | 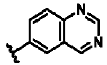 | A2 | C₂₁H₁₉N₅O₂ | 373.42 | (1*R*) |
| **70** | 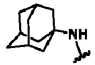 | H | 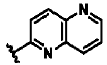 | A1 | C₂₂H₂₃N₅O | 373.46 | N/A |
| **71** | 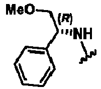 | H | 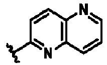 | A2 | C₂₁H₁₉N₅O₂ | 373.42 | *(1R)* |
| **72** | 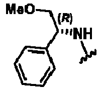 | H | 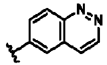 | A2 | C₂₁H₁₉N₅O₂ | 373.42 | *(1R)* |
| **73** | 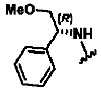 | H | 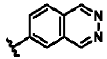 | A2 | C₂₁H₁₉N₅O₂ | 373.42 | (1*R*) |

The below Table 3 describes the analytical and spectroscopic data of the compounds introduced in Table 2.

¹H NMR analyses (400 or 500 MHz) and ¹³C NMR spectra (101 MHz) were recorded with a Bruker ULTRASHIELD 500 or 400 spectrometer. Processing and analyses of the spectra were performed with MestReNova. Data appear in the following order: chemical shifts in ppm which were referenced to the internal solvent signal, multiplicity, number of protons, and coupling constant J in Hertz.

Reversed-phase HPLC/MS analyses were carried out with a Waters Alliance 2795 HPLC equipped with an autosampler, an inline membrane degasser, a column oven (T° = 45 °C), a UV detector, and a ZQ quadrupole mass detector working in ionization electrospray mode. Compounds (0.1 to 0.3 mg) were solubilized in a minimum amount of DMSO completed with acetonitrile (Vₜₒₜₐₗ: 1 mL). Standard analytical parameters: flow rate: 1mL/min, V_{inj.}: 5µL .
- Acidic conditions: Waters XSelect CSH C18 column (3.5µm, 2.1x 50 mm). Gradient: (H₂O + 0.04% v/v HCOOH (10mM))/ACN from 95/5 to 0/100 in 18.5 min.
- Alkaline conditions: Waters Xbridge C18 column (3.5µm, 2.1× 50 mm). Gradient: (H₂O + 0.06% v/v NH_{3(aq)} (10mM))/ACN from 95/5 to 0/100 in 18.5 min.

**Table 3. Spectroscopic and analytical characterization of compounds (1) to (73)**

| **Cpd N°** | **Structure** | **Formula** | **MW** | **HPLC** | **Description** |
|---|---|---|---|---|---|
| **1** | 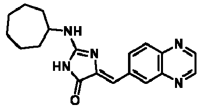 | C₁₉H₂₁N₅O | 335.41 | 97% | Beige solid. ¹H NMR (400 MHz, DMSO-*d*6, 323K) of major tautomer δ_{H} 10.51 (br s, 1H, NH, D₂O exchanged), 8.89 (s, 1H), 8.83 (s, 1H), 8.76 (br s, 1H), 8.51 (br s, 1H), 8.00 (d, *J* = 8.8 Hz, 1H), 7.66 (br s, 1H, NH, D₂O exchanged), 6.47 (s, 1H), 3.99 (br s, 1H), 2.05 - 1.90 (m, 2H), 1.75 - 1.40 (m, 10H). MS (ESI⁺) : [M+H]⁺ 336.3. |
| **2** | 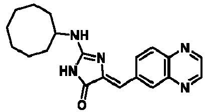 | C₂₀H₂₃N₅O | 349.44 | 96% | Beige solid. ¹H NMR (400 MHz, DMSO-*d*₆, 323K) of major tautomer δ_{H} 10.48 (br s, 1H, NH, D₂O exchanged), 8.89 (s, 1H), 8.83 (s, 1H), 8.76 (br s, 1H), 8.53 (br s, 1H), 7.99 (d, *J* = 8.9 Hz, 1H), 7.63 (br s, 1H, NH, D₂O exchanged), 6.47 (s, 1H), 4.02 (br s, 1H), 1.95 - 1.83 (m, 2H), 1.81 - 1.40 (m, 12H). MS (ESI⁺) : [M+H]⁺ 350.3. |
| **3** | 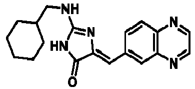 | C₁₉H₂₁N₅O | 335.41 | >98% | Pale yellow solid. ¹H NMR (400 MHz, DMSO-*d*₆, 373K) of major tautomer δ_{H} 10.59 (br s, 1H, NH, D₂O exchanged), 8.88 (s, 1H), 8.82 (s, 1H), 8.75 (s, 1H), 8.52 (d, *J* = 8.9 Hz, 1H), 7.99 (d, *J* = 8.8 Hz, 1H), 7.60 (br s, 1H, NH, D₂O exchanged), 6.46 (s, 1H), 3.28 (s, 2H), 1.90 - 1.50 (m, 6H), 1.36 - 1.11 (m, 3H), 1.11 - 0.91 (m, 2H). MS (ESI⁺) : [M+H]⁺ 336.3. |
| **4** | 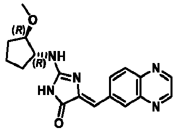 | C₁₈H₁₉N₅O₂ | 337.38 | >98% | Yellow solid. ¹H NMR (400 MHz, DMSO-*d*₆, 323K) of major tautomer δ_{H} 10.64 (br s, 1H, NH, D₂O exchanged), 8.89 (s, 1H), 8.84 (s, 1H), 8.78 (br s, 1H), 8.53 (br s, 1H), 8.00 (d, *J* = 8.8 Hz, 1H), 7.81 (br s, 1H, NH, D₂O exchanged), 6.51 (s, 1H), 4.20 (br s, 1H), 3.80 (br s, 1H), 3.37 (s, 3H), 2.20 - 2.04 (m, 1H), 2.00 - 1.85 (m, 1H), 1.80 - 1.50 (m, 4H). MS (ESI⁺) : [M+H]⁺ 338.3. |
| **5** | 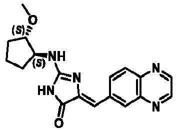 | C₁₈H₁₉N₅O₂ | 337.38 | 98% | Yellow solid. ¹H NMR (400 MHz, DMSO-*d*₆, 323K) of major tautomer δ_{H} 10.64 (br s, 1H, NH, D₂O exchanged), 8.89 (s, 1H), 8.84 (s, 1H), 8.78 (br s, 1H), 8.53 (br s, 1H), 8.00 (d, *J* = 8.8 Hz, 1H), 7.81 (br s, 1H, NH, D₂O exchanged), 6.51 (s, 1H), 4.20 (br s, 1H), 3.80 (br s, 1H), 3.37 (s, 3H), 2.20 - 2.04 (m, 1H), 2.00 - 1.85 (m, 1H), 1.80 - 1.50 (m, 4H). MS (ESI⁺) : [M+H]⁺ 338.3. |
| **6** | 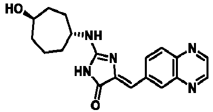 | C₁₉H₂₁N₅O₂ | 351.41 | 98% | Yellow solid. ¹H NMR (400 MHz, DMSO-*d*₆, 323K) of major tautomer δ_{H} 10.52 (br s, 1H, NH, D₂O exchanged), 8.89 (s, 1H), 8.84 (s, 1H), 8.74 (br s, 1H), 8.51 (br s, 1H), 8.01 (d, *J* = 8.7 Hz, 1H), 7.67 (br s, 1H, NH, D₂O exchanged), 6.47 (s, 1H), 4.34 (d, *J* = 4.1 Hz, 1H, OH, D₂O exchanged), 3.98 (br s, 1H), 3.71 (br s, 1H), 2.15 - 1.70 (m, 4H), 1.69 - 1.40 (m, 6H). MS (ESI⁺) : [M+H]⁺ 352.3. |
| **7** | 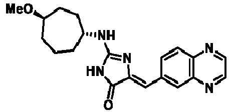 | C₂₀H₂₃N₅O₂ | 365.44 | 98% | Yellow solid. ¹H NMR (400 MHz, DMSO-*d*₆, 323K) of major tautomer δ_{H} 10.56 (br s, 1H, NH, D₂O exchanged), 8.89 (s, 1H), 8.84 (s, 1H), 8.75 (br s, 1H), 8.51 (br s, 1H), 8.00 (d, *J* = 8.9 Hz, 1H), 7.70 (br s, 1H, NH, D₂O exchanged), 6.47 (s, 1H), 3.98 (br s, 1H), 3.38 (br s, 1H), 3.24 (s, 3H), 2.15 - 1.90 (m, 3H), 1.88 - 1.73 (m, 1H), 1.72 - 1.45 (m, 6H). MS (ESI⁺) : [M+H]⁺ 366.3. |
| **8** | 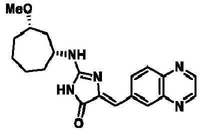 | C₂₀H₂₃N₅O₂ | 365.44 | 94% | Yellow solid. ¹H NMR (400 MHz, DMSO-*d*₆, 323K) of major tautomer δ_{H} 10.57 (br s, 1H, NH, D₂O exchanged), 8.89 (s, 1H), 8.84 (s, 1H), 8.77 (br s, 1H), 8.52 (br s, 1H), 8.00 (d, *J* = 8.7 Hz, 1H), 7.62 (br s, 1H, NH, D₂O exchanged), 6.49 (s, 1H), 3.98 (br s, 1H), 3.48 (br s, 1H), 3.26 (s, 3H), 2.40 - 2.20 (m, 1H), 2.03 - 1.80 (m, 2H), 1.80 - 1.40 (m, 7H). MS (ESI⁺) : [M+H]⁺ 366.2. |
| **9** | 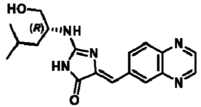 | C₁₈H₂₁N₅O₂ | 339.40 | 98% | Yellow solid. ¹H NMR (400 MHz, DMSO-*d*₆, 323K) of major tautomer δ_{H} 10.42 (br s, 1H, NH, D₂O exchanged), 8.89 (s, 1H), 8.84 (s, 1H), 8.73 (br s, 1H), 8.52 (br s, 1H), 7.99 (d, *J* = 8.7 Hz, 1H), 7.35 (br s, 1H, NH, D₂O exchanged), 6.47 (s, 1H), 4.80 (br s, 1H, OH, D₂O exchanged), 4.12 (br s, 1H), 3.52 (br s, 2H), 1.80 - 1.60 (m, 1H), 1.58 - 1.35 (m, 2H), 1.05 - 0.85 (m, 6H). MS (ESI⁺) : [M+H]+ 340.3. |
| **10** | 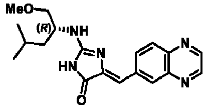 | C₁₉H₂₃N₅O₂ | 353.43 | 97% | Yellow solid. ¹H NMR (400 MHz, DMSO-*d*₆, 300K) of major tautomer δ_{H} 10.58 (br s, 1H, NH, D₂O exchanged), 8.89 (s, 1H), 8.84 (s, 1H), 8.79 (br s, 1H), 8.52 (d, *J* = 9.0 Hz, 1H), 7.99 (d, *J* = 8.8 Hz, 1H), 7.57 (br s, 1H, NH, D₂O exchanged), 6.48 (s, 1H), 4.37 (br s, 1H), 3.55 - 3.32 (m, 2H), 3.30 (s, 3H), 1.77 - 1.62 (m, 1H), 1.61 - 1.30 (m, 2H), 1.05 - 0.85 (m, 6H). MS (ESI⁺) : [M+H]+ 354.3. |
| **11** | 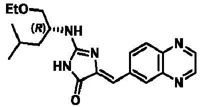 | C₂₀H₂₅N₅O₂ | 367.45 | 95% | Yellow solid. ¹H NMR (400 MHz, DMSO-*d*₆, 343K) of major tautomer δ_{H} 10.48 (br s, 1H, NH, D₂O exchanged), 8.96 - 8.70 (m, 3H), 8.52 (d, *J* = 8.8 Hz, 1H), 7.97 (d, *J* = 8.8 Hz, 1H), 7.40 (br s, 1H, NH, D₂O exchanged), 6.48 (s, 1H), 4.35 - 4.04 (m, 1H), 3.63 - 3.35 (m, 4H), 1.80 - 1.62 (m, 1H), 1.62 - 1.36 (m, 2H), 1.13 (t, *J* = 7.0 Hz, 3H), 1.02 - 0.86 (m, 6H). MS (ESI⁺) : [M+H]⁺ 368.3. |
| **12** | 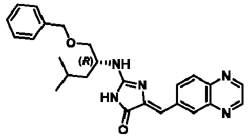 | C₂₅H₂₇N₅O₂ | 429.52 | 95% | Yellow solid. ¹H NMR (400 MHz, DMSO-*d*₆, 343K) of major tautomer δ_{H} 10.50 (br s, 1H, NH, D₂O exchanged), 8.98 - 8.75 (m, 3H), 8.52 (d, *J* = 8.8 Hz, 1H), 7.97 (d, *J* = 8.8 Hz, 1H), 7.45 (br s, 1H, NH, D₂O exchanged), 7.38 - 7.19 (m, 5H), 6.48 (s, 1H), 4.65 - 4.50 (m, 2H), 4.35 - 4.25 (m, 1H), 3.81 - 3.44 (m, 2H), 1.79 - 1.64 (m, 1H), 1.64 - 1.41 (m, 2H), 1.05 - 0.83 (m, 6H). MS (ESI⁺) : [M+H]+ 430.3. |
| **13** | 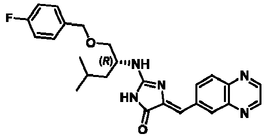 | C₂₅H₂₆FN₅O₂ | 447.51 | 95% | Yellow solid. ¹H NMR (400 MHz, DMSO-*d*₆, 343K) of major tautomer δ_{H} 10.50 (br s, 1H, NH, D₂O exchanged), 8.96 - 8.72 (m, 3H), 8.51 (d, *J* = 8.8 Hz, 1H), 7.97 (d, *J* = 8.8 Hz, 1H), 7.45 (br s, 1H, NH, D₂O exchanged), 7.41 - 7.31 (m, 2H), 7.19 - 7.04 (m, 2H), 6.48 (s, 1H), 4.61 - 4.48 (m, 2H), 4.27 (br s, 1H), 3.66 - 3.49 (m, 2H), 1.79 - 1.64 (m, 1H), 1.62 - 1.39 (m, 2H), 1.02 - 0.86 (m, 6H). MS (ESI⁺) : [M+H]⁺ 448.3. |
| **14** | 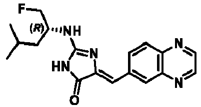 | C₁₈H₂₀FN₅O | 341.39 | 95% | Yellow solid. ¹H NMR (400 MHz, DMSO-*d*₆, 373K) of major tautomer δ_{H} 10.00 (br s, 1H, NH, D₂O exchanged), 9.00 - 8.63 (m, 3H), 8.51 (s, 1H), 8.06 - 7.88 (m, 1H), 7.43 (br s, 1H, NH, D₂O exchanged), 6.52 (s, 1H), 4.64 - 4.53 (m, 1H), 4.52 - 4.41 (m, 1H), 4.32 (s, 1H), 1.82 - 1.69 (m, 1H), 1.67 - 1.54 (m, 1H), 1.54 - 1.40 (m, 1H), 1.05 - 0.80 (m, 6H). MS (ESI⁺) : [M+H]⁺ 342.2. |
| **15** | 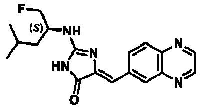 | C₁₈H₂₀FN₅O | 341.39 | >98% | Yellow solid. ¹H NMR (400 MHz, DMSO-*d*₆, 373K) of major tautomer δ_{H} 10.00 (br s, 1H, NH, D₂O exchanged), 9.00 - 8.63 (m, 3H), 8.51 (s, 1H), 8.06 - 7.88 (m, 1H), 7.43 (br s, 1H, NH, D₂O exchanged), 6.52 (s, 1H), 4.64 - 4.53 (m, 1H), 4.52 - 4.41 (m, 1H), 4.32 (s, 1H), 1.82 - 1.69 (m, 1H), 1.67 - 1.54 (m, 1H), 1.54 - 1.40 (m, 1H), 1.05 - 0.80 (m, 6H). MS (ESI⁺) : [M+H]⁺ 342.3. |
| **16** | 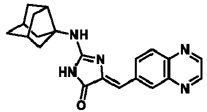 | C₂₁H₂₁N₅O | 359.43 | 95% | Yellow solid. ¹H NMR (400 MHz, DMSO-*d*₆, 343K) of major tautomer δ_{H} 10.04 (br s, 1H, NH, D₂O exchanged), 8.88 (d, *J =* 1.9 Hz, 1H), 8.82 (d, *J* = 1.8 Hz, 1H), 8.77 (d, *J* = 1.8 Hz, 1H), 8.52 (dd, *J* = 8.8, 1.9 Hz, 1H), 7.97 (d, *J* = 8.8 Hz, 1H), 7.47 (br s, 1H, NH, D₂O exchanged), 6.49 (s, 1H), 2.75 - 2.65 (m, 1H), 2.39 - 2.26 (m, 4H), 2.26 - 2.14 (m, 2H), 2.10 - 1.98 (m, 2H), 1.73 - 1.52 (m, 4H). MS (ESI⁺) : [M+H]+ 360.2. |
| **17** | 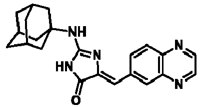 | C₂₂H₂₃N₅O | 373.46 | 98% | Yellow solid. ¹H NMR (400 MHz, DMSO-*d*₆, 323K) of major tautomer δ_{H} 9.82 (br s, 1H, NH, D₂O exchanged), 8.90 (s, 1H), 8.84 (m, 2H), 8.47 (br s, 1H), 8.00 (d, *J* = 8.7 Hz, 1H), 7.08 (br s, 1H, NH, D₂O exchanged), 6.49 (s, 1H), 2.30 - 2.05 (m, 9H), 1.85 - 1.65 (m, 6H). MS (ESI⁺) : [M+H]+ 374.3. |
| **18** | 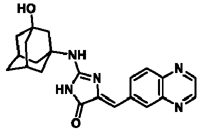 | C₂₂H₂₃N₅O₂ | 389.46 | 94% | Yellow solid. ¹H NMR (400 MHz, DMSO-*d*₆, 323K) of major tautomer δ_{H} 9.95 (br s, 1H, NH, D₂O exchanged), 8.90 (s, 1H), 8.83 (s, 1H), 8.78 (s, 1H), 8.54 (d, *J* = 8.8 Hz, 1H), 8.00 (d, *J* = 8.7 Hz, 1H), 7.15 (br s, 1H, NH, D₂O exchanged), 6.50 (s, 1H), 4.49 (br s, 1H, OH, D₂O exchanged), 2.26 (br s, 2H), 2.20 - 2.10 (m, 2H), 2.10 - 1.90 (m, 4H), 1.75 - 1.45 (m, 6H). MS (ESI⁺) : [M+H]⁺ 390.3. |
| **19** | 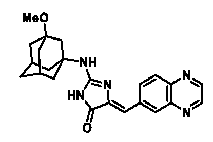 | C₂₃H₂₅N₅O₂ | 403.49 | >98% | Yellow solid. ¹H NMR (400 MHz, DMSO-*d*₆, 373K) of major tautomer δ_{H} 9.76 (br s, 1H, NH, D₂O exchanged), 8.88 (s, 1H), 8.82 (s, 1H), 8.80 (br s, 1H), 8.48 (br s 1H), 7.99 (d, *J* = 8.7 Hz, 1H), 6.90 (br s, 1H, NH, D₂O exchanged), 6.51 (s, 1H), 3.22 (s, 3H), 2.34 (br s, 2H), 2.26 - 2.03 (m, 6H), 1.88 - 1.50 (m, 6H). MS (ESI⁺) : [M+H]⁺ 404.3. |
| **20** | 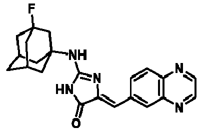 | C₂₂H₂₂FN₅O | 391.45 | >98% | Yellow solid. ¹H NMR (400 MHz, DMSO-*d*₆, 343K) of major tautomer δ_{H} 9.98 (br s, 1H, NH, D₂O exchanged), 8.92 - 8.79 (m, 3H), 8.46 (dd, *J* = 8.8, 1.9 Hz, 1H), 8.00 (d, *J* = 8.8 Hz, 1H), 7.20 (br s, 1H, NH, D₂O exchanged), 6.52 (s, 1H), 2.44 - 2.32 (m, 4H), 2.24 - 2.04 (m, 4H), 2.03 - 1.81 (m, 4H), 1.70 - 1.54 (m, 2H). MS (ESI⁺) : [M+H]+ 392.3. |
| **21** | 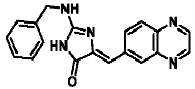 | C₁₉H₁₅N₅O | 329.36 | 97% | Yellow solid. ¹H NMR (400 MHz, DMSO-*d*₆, 373K) of major tautomer δ_{H} 10.70 (br s, 1H, NH, D₂O exchanged), 8.88 (d, *J* = 1.8 Hz, 1H), 8.83 (d, *J* = 1.9 Hz, 1H), 8.72 (br s, 1H), 8.50 (br s, 1H), 8.08 (br s, 1H, NH, D₂O exchanged), 8.01 (d, *J* = 8.8 Hz, 1H), 7.50 - 7.42 (m, 2H), 7.37 (app t, *J* = 7.6 Hz, 2H), 7.33 - 7.23 (m, 1H), 6.52 (s, 1H), 4.64 (s, 2H). MS (ESI⁺) : [M+H]⁺ 330.2. |
| **22** | 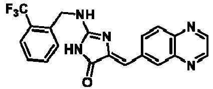 | C₂₀H₁₄F₃N₅O | 397.36 | 98% | Yellow solid. ¹H NMR (400 MHz, DMSO-*d*₆, 323K) of major tautomer δ_{H} 10.83 (br s, 1H, NH, D₂O exchanged), 8.88 (s, 1H), 8.83 (s, 1H), 8.65 (br s, 1H), 8.54 (br s, 1H), 8.18 (br s, 1H, NH, D₂O exchanged), 7.96 (d, *J* = 8.9 Hz, 1H), 7.78 (d, *J* = 7.9 Hz, 1H), 7.74 - 7.64 (m, 2H), 7.55 - 7.46 (m, 1H), 6.55 (s, 1H), 4.85 (s, 2H). MS (ESI⁺) : [M+H]⁺ 398.2. |
| **23** | 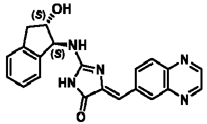 | C₂₁H₁₇N₅O₂ | 371.40 | >98% | Beige solid. ¹H NMR (400 MHz, DMSO-*d*₆, 323K) of major tautomer δ_{H} 10.76 (br s, 1H, NH, D₂O exchanged), 8.86 (s, 1H), 8.83 (s, 1H), 8.73 (br s, 1H), 8.52 (br s, 1H), 8.19 (br s, 1H, NH, D₂O exchanged), 8.00 (d, *J* = 8.6 Hz, 1H), 7.34 - 7.16 (m, 4H), 6.55 (s, 1H), 5.90 - 4.90 (br m, 1H + OH, D₂O exchanged), 4.47 (app q, *J* = 7.1 Hz, 1H), 3.23 (dd, *J* = 15.7, 7.4 Hz, 1H), 2.81 (dd, *J* = 16.1, 7.5 Hz, 1H). MS (ESI⁺) : [M+H]⁺ 372.2. |
| **24** | 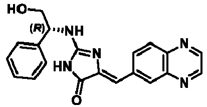 | C₂₀H₁₇N₅O₂ | 359.39 | >98% | Orange solid. ¹H NMR (400 MHz, DMSO-*d*₆, 373K) of major tautomer δ_{H} 10.26 (br s, 1H, NH, D₂O exchanged), 8.88 (s, 1H), 8.82 (s, 1H), 8.64 (br s, 1H), 8.44 (br s, 1H), 7.99 (d, *J* = 8.7 Hz, 1H), 7.77 (br s, 1H, NH, D₂O exchanged), 7.52 - 7.43 (m, 2H), 7.41 - 7.31 (m, 2H), 7.30 - 7.22 (m, 1H), 6.49 (s, 1H), 5.18 - 5.03 (m, 1H), 4.80 (br s, 1H, OH, D₂O exchanged), 3.83 (d, *J =* 5.8 Hz, 2H). MS (ESI⁺) : [M+H]⁺ 360.2. |
| **25** | 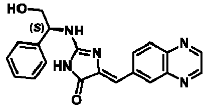 | C₂₀H₁₇N₅O₂ | 359.39 | >98% | Orange solid. ¹H NMR (400 MHz, DMSO-*d*₆, 373K) of major tautomer δ_{H} 10.26 (br s, 1H, NH, D₂O exchanged), 8.88 (s, 1H), 8.82 (s, 1H), 8.64 (br s, 1H), 8.44 (br s, 1H), 7.99 (d, *J* = 8.7 Hz, 1H), 7.77 (br s, 1H, NH, D₂O exchanged), 7.52 - 7.43 (m, 2H), 7.41 - 7.31 (m, 2H), 7.30 - 7.22 (m, 1H), 6.49 (s, 1H), 5.18 - 5.03 (m, 1H), 4.80 (br s, 1H, OH, D₂O exchanged), 3.83 (d, *J* = 5.8 Hz, 2H). MS (ESI⁺) : [M+H]⁺ 360.2. |
| **26** | 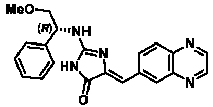 | C₂₁H₁₉N₅O₂ | 373.42 | 98% | Orange solid. ¹H NMR (400 MHz, DMSO-*d*₆, 323K) of major tautomer δ_{H} 10.19 (br s, 1H, NH, D₂O exchanged), 8.95 (s, 1H), 8.88 (s, 1H), 8.78 (s, 1H), 8.56 (d, *J* = 8.8 Hz, 1H), 8.28 (br s, 1H, NH, D₂O exchanged), 8.05 (d, *J* = 8.7 Hz, 1H), 7.60 - 7.47 (m, 2H), 7.46 - 7.36 (m, 2H), 7.32 - 7.23 (m, 1H), 6.54 (s, 1H), 5.30 (br s, 1H), 3.87 - 3.65 (m, 2H), 3.39 (s, 3H). MS (ESI⁺) : [M+H]⁺ 374.2. |
| **27** | 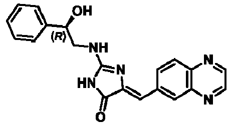 | C₂₀H₁₇N₅O₂ | 359.39 | 98% | Yellow solid. ¹H NMR (400 MHz, DMSO-*d*₆, 343K) of major tautomer δ_{H} 10.43 (br s, 1H, NH, D₂O exchanged), 8.90 (d, *J =* 1.8 Hz, 1H), 8.84 (d, *J* = 1.9 Hz, 1H), 8.77 (br s, 1H), 8.47 (br s, 1H), 8.02 (d, *J* = 8.8 Hz, 1H), 7.62 - 7.43 (m, 2H + NH, D₂O exchanged), 7.39 (t, *J* = 7.5 Hz, 2H), 7.32 - 7.23 (m, 1H), 6.51 (s, 1H), 5.51 (br s, 1H, OH, D₂O exchanged), 4.93 (br s, 1H), 3.79 - 3.67 (m, 1H), 3.50 (dd, *J* = 13.4, 8.0 Hz, 1H). MS (ESI⁺) : [M+H]⁺ 360.2. |
| **28** | 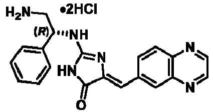 | C₂₀H₂₀Cl₂N₆O | 431.32 | 95% | Beige solid. ¹H NMR (400 MHz, D₂O, 323K) of major tautomer δ_{H} 8.80 (s, 1H), 8.77 (s, 1H), 7.98 (br s, 1H), 7.88 - 7.65 (m, 7H), 6.48 (s, 1H), 5.57 - 5.46 (m, 1H), 3.86 - 3.72 (m, 2H), MS (ESI⁺) : [M+H]⁺ 359.3 (+2HCl). |
| **29** | 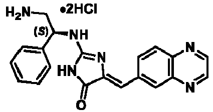 | C₂₀H₂₀Cl₂N₆O | 431.32 | 95% | Beige solid. ¹H NMR (400 MHz, D₂O, 323K) of major tautomer δ_{H} 8.80 (s, 1H), 8.77 (s, 1H), 7.98 (br s, 1H), 7.88 - 7.65 (m, 7H), 6.48 (s, 1H), 5.57 - 5.46 (m, 1H), 3.86 - 3.72 (m, 2H), MS (ESI⁺) : [M+H]⁺ 359.3 (+2HCl). |
| **30** | 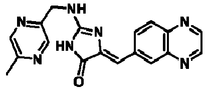 | C₁₈H₁₅N₇O | 345.37 | 96% | Yellow solid. ¹H NMR (400 MHz, DMSO-*d*₆, 343K) of major tautomer δ_{H} 10.71 (br s, 1H, NH, D₂O exchanged), 8.89 (s, 1H), 8.84 (s, 1H), 8.67 (br s, 1H), 8.64 (s, 1H), 8.53 (br s, 2H), 8.09 (br s, 1H, NH, D₂O exchanged), 8.01 (d, *J* = 8.8 Hz, 1H), 6.54 (s, 1H), 4.77 (s, 3H), 2.45 (s, 3H). MS (ESI⁺) : [M+H]⁺ 346.2. |
| **31** | 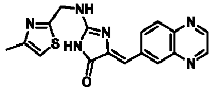 | C₁₇H₁₄N₆OS | 350.40 | >98% | Yellow solid. ¹H NMR (400 MHz, DMSO-*d*₆, 323K) of major tautomer δ_{H} 10.88 (br s, 1H, NH, D₂O exchanged), 8.89 (d, *J =* 1.8 Hz, 1H), 8.85 (d, *J* = 1.9 Hz, 1H), 8.75 - 8.20 (br s, 1H, NH, D₂O exchanged), 8.70 (br s, 1H), 8.54 (br s, 1H), 8.01 (d, *J* = 8.8 Hz, 1H), 7.17 (s, 1H), 6.58 (s, 1H), 4.89 (s, 2H), 2.37 (s, 3H). MS (ESI⁺) : [M+H]⁺ 351.2. |
| **32** | 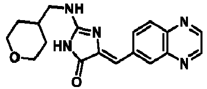 | C₁₈H₁₉N₅O₂ | 337.38 | >98% | Yellow solid. ¹H NMR (400 MHz, DMSO-*d*₆) of major tautomer δ_{H} 10.74 (br s, 1H, NH, D₂O exchanged), 8.90 (s, 1H), 8.85 (s, 1H), 8.77 (br s, 1H), 8.60 - 8.45 (br s, 1H), 8.02 (d, *J* = 8.7 Hz, 1H), 7.71 (br s, 1H, NH, D₂O exchanged), 6.48 (s, 1H), 3.94 - 3.81 (m, 2H), 3.42 - 3.15 (m, 4H), 2.05 - 1.70 (s, 1H), 1.72 - 1.58 (m, 2H), 1.40 - 1.10 (m, 2H). MS (ESI⁺) : [M+H]⁺ 338.2. |
| **33** | 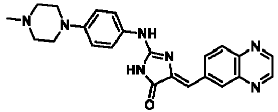 | C₂₃H₂₃N₇O | 413.49 | >98% | Brown solid. ¹H NMR (400 MHz, DMSO-*d*₆, 323K) of major tautomer δ_{H} 10.62 (br s, 1H, NH, D₂O exchanged), 9.74 (br s, 1H, NH, D₂O exchanged), 8.93 (s, 1H), 8.87 (s, 1H), 8.71 (br s, 1H), 8.62 (br s, 1H), 8.07 (d, *J* = 7.8 Hz, 1H), 7.64 (d, *J* = 8.4 Hz, 2H), 7.00 (d, *J* = 8.7 Hz, 2H), 6.65 (s, 1H), 3.18 - 3.11 (m, 4H), 2.49 - 2.46 (m, 4H), 2.25 (s, 3H). MS (ESI⁺) : [M+H]⁺ 414.3. |
| **34** | 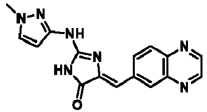 | C₁₆H₁₃N₇O | 319.33 | 97% | Yellow solid. ¹H NMR (400 MHz, CF₃COOD, 300K) of major tautomer δ_{H} 10.14 - 9.89 (m, 2H), 9.36 (s, 1H), 9.15 (d, *J* = 8.9 Hz, 1H), 8.92 (d, *J* = 9.0 Hz, 1H), 8.17 - 7.98 (m, 2H), 6.85 (s, 1H), 4.50 (s, 3H). MS (ESI⁺) : [M+H]+ 320.2. |
| **35** | 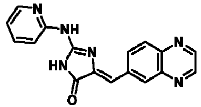 | C₁₇H₁₂N₆O | 316.32 | 95% | Yellow solid. ¹H NMR (400 MHz, DMSO-*d*₆, 323K) of major tautomer δ_{H} 11.09 (br s, 2H, NH, D₂O exchanged), 8.94 (s, 1H), 8.89 (s, 1H), 8.81 (br s, 1H), 8.59 (br s, 1H), 8.36 (d, *J* = 4.9 Hz, 1H), 8.10 (d, *J* = 8.9 Hz, 1H), 7.90 - 7.81 (m, 1H), 7.53 (br s, 1H), 7.16 - 7.06 (m, 1H), 6.82 (s, 1H). MS (ESI⁺) : [M+H]⁺ 317.2. |
| **36** | 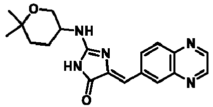 | C₁₉H₂₁N₅O₂ | 351.41 | >98% | Orange solid. ¹H NMR (400 MHz, DMSO-*d*₆, 323K) of major tautomer δ_{H} 10.57 (br s, 1H, NH, D₂O exchanged), 8.90 (d, *J =* 1.9 Hz, 1H), 8.85 (d, *J* = 1.9 Hz, 1H), 8.72 (br s, 1H), 8.50 (br s, 1H), 8.02 (d, *J* = 8.7 Hz, 1H), 7.64 (br s, 1H, NH, D₂O exchanged), 6.51 (s, 1H), 3.89 (br s, 1H), 3.83 - 3.68 (m, 1H), 3.52 (dd, *J* = 11.5, 7.9 Hz, 1H), 2.00 - 1.75 (m, 2H), 1.72 - 1.60 (m, 1H), 1.56 - 1.45 (m, 1H), 1.23 (s, 3H), 1.21 (s, 3H). MS (ESI⁺) : [M+H]⁺ 352.3. |
| **37** | 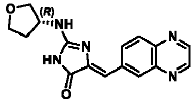 | C₁₆H₁₅N₅O₂ | 309.33 | 97% | Yellow solid. ¹H NMR (400 MHz, DMSO-*d*₆, 373K) of major tautomer δ_{H} 10.37 (br s, 1H, NH, D₂O exchanged), 8.88 (d, *J =* 1.8 Hz, 1H), 8.83 (d, *J* = 1.9 Hz, 1H), 8.72 (br s, 1H), 8.49 (br s, 1H), 8.01 (d, *J* = 8.8 Hz, 1H), 7.89 (br s, 1H, NH, D₂O exchanged), 6.52 (s, 1H), 4.53 (br s, 1H), 4.00 - 3.85 (m, 2H), 3.83 - 3.74 (m, 1H), 3.69 (dd, *J* = 9.1, 4.1 Hz, 1H), 2.35 - 2.22 (m, 1H), 2.06 - 1.91 (m, 1H). MS (ESI⁺) : [M+H]⁺ 310.1. |
| **38** | 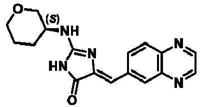 | C₁₇H₁₇N₅O₂ | 323.36 | >98% | Yellow solid. ¹H NMR (400 MHz, DMSO-*d*₆, 343K) of major tautomer δ_{H} 10.42 (br s, 1H, NH, D₂O exchanged), 8.99 - 8.80 (m, 2H), 8.74 (s, 1H), 8.51 (d, *J* = 8.8 Hz, 1H), 8.01 (d, *J* = 8.8 Hz, 1H), 7.59 (br s, 1H, NH, D₂O exchanged), 6.51 (s, 1H), 4.06 - 3.83 (m, 2H), 3.80 - 3.67 (m, 1H), 3.58 - 3.33 (m, 2H), 2.11 - 1.90 (m, 1H), 1.87 - 1.38 (m, 3H). MS (ESI⁺) : [M+H]⁺ 324.2. |
| **39** | 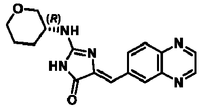 | C₁₇H₁₇N₅O₂ | 323.36 | >98% | Yellow solid. ¹H NMR (400 MHz, DMSO-*d*₆, 343K) of major tautomer δ_{H} 10.42 (br s, 1H, NH, D₂O exchanged), 8.99 - 8.80 (m, 2H), 8.74 (br s, 1H), 8.51 (d, *J* = 8.8 Hz, 1H), 8.01 (d, *J* = 8.8 Hz, 1H), 7.59 (br s, 1H, NH, D₂O exchanged), 6.51 (s, 1H), 4.06 - 3.83 (m, 2H), 3.80 - 3.67 (m, 1H), 3.58 - 3.33 (m, 2H), 2.11 - 1.90 (m, 1H), 1.87 - 1.38 (m, 3H). MS (ESI⁺) : [M+H]⁺ 324.3. |
| **40** | 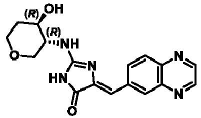 | C₁₇H₁₇N₅O₃ | 339.36 | >98% | Orange solid. ¹H NMR (400 MHz, DMSO-*d*₆, 323K) of major tautomer δ_{H} 10.60 (br s, 1H, NH, D₂O exchanged), 8.90 (s, 1H), 8.85 (s, 1H), 8.75 (br s, 1H), 8.51 (br s, 1H), 8.02 (d, *J* = 8.8 Hz, 1H), 7.64 (br s, 1H, NH, D₂O exchanged), 6.51 (s, 1H), 5.05 (br s, 1H, OH, D₂O exchanged), 4.15 - 3.93 (m, 1H), 3.91 - 3.82 (m, 1H), 3.81 - 3.60 (br s, 2H), 3.48 - 3.33 (m, 1H), 3.30 - 3.15 (m, 1H), 2.00 - 1.89 (m, 1H), 1.67 - 1.43 (m, 1H). MS (ESI⁺) : [M+H]⁺ 340.2. |
| **41** | 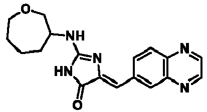 | C₁₈H₁₉N₅O₂ | 337.38 | >98% | Yellow solid. ¹H NMR (400 MHz, DMSO-*d*₆) of major tautomer δ_{H} 10.38 (br s, 1H, NH, D₂O exchanged), 8.89 (s, 1H), 8.84 (s, 1H), 8.74 (br s, 1H), 8.52 (br s, 1H), 8.01 (d, *J* = 8.7 Hz, 1H), 7.60 (br s, 1H, NH, D₂O exchanged), 6.50 (s, 1H), 4.16 (br s, 1H), 3.90 - 3.77 (m, 1H), 3.76 - 3.60 (m, 3H), 2.05 - 1.88 (m, 1H), 1.87 - 1.68 (m, 4H), 1.67 - 1.50 (m, 1H). MS (ESI⁺) : [M+H]⁺ 338.2. |
| **42** | 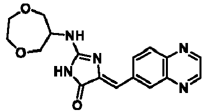 | C₁₇H₁₇N₅O₃ | 339.36 | >98% | Yellow solid. ¹H NMR (400 MHz, DMSO-*d*₆, 343K) of major tautomer δ_{H} 10.54 (br s, 1H, NH, D₂O exchanged), 8.99 - 8.80 (m, 2H), 8.73 (br s, 1H), 8.53 (d, *J* = 8.8 Hz, 1H), 8.01 (d, *J* = 8.8 Hz, 1H), 7.55 (br s, 1H, NH, D₂O exchanged), 6.52 (s, 1H), 3.92 - 3.73 (m, 3H), 3.71 - 3.58 (m, 2H), 3.57 - 3.42 (m, 3H), 3.40 - 3.30 (m, 1H). MS (ESI⁺) : [M+H]⁺ 340.3. |
| **43** | 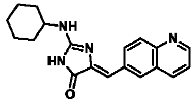 | C₁₉H₂₀N₄O | 320.40 | >98% | Yellow solid. ¹H NMR (400 MHz, DMSO-*d*6, 343K) of major tautomer δ_{H} 10.44 (br s, 1H, NH, D₂O exchanged), 8.83 (s, 1H), 8.62 - 8.37 (m, 2H), 8.23 (d, *J* = 8.3 Hz, 1H), 7.95 (d, *J* = 8.7 Hz, 1H), 7.60 - 7.43 (m, 1H), 7.39 (br s, 1H, NH, D₂O exchanged), 6.44 (s, 1H), 3.94 - 3.65 (m, 1H), 1.96 (br s, 2H), 1.86 - 1.55 (m, 3H), 1.54 - 1.13 (m, 5H). MS (ESI⁺) : [M+H]⁺ 321.2. |
| **44** | 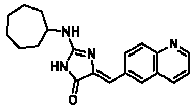 | C₂₀H₂₂N₄O | 334.42 | >98% | Colorless solid. ¹H NMR (400 MHz, DMSO-*d*6, 343K) of major tautomer δ_{H} 10.35 (br s, 1H, NH, D₂O exchanged), 8.83 - 8.69 (m, 1H), 8.60 - 8.44 (m, 2H), 8.22 (d, *J* = 8.3 Hz, 1H), 7.94 (d, *J* = 9.0 Hz, 1H), 7.60 - 7.44 (m, 1H), 7.37 (br s, 1H, NH, D₂O exchanged), 6.43 (s, 1H), 3.99 (br s, 1H), 2.08 - 1.85 (m, 2H), 1.79 - 1.40 (m, 10H). MS (ESI⁺) : [M+H]⁺ 335.3. |
| **45** | 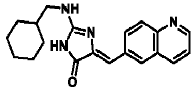 | C₂₀H₂₂N₄O | 334.42 | >98% | Pale Yellow solid. ¹H NMR (400 MHz, DMSO-*d*6, 343K) of major tautomer δ_{H} 10.36 (br s, 1H, NH, D₂O exchanged), 8.83 (dd, *J* = 4.2, 1.7 Hz, 1H), 8.48 (br s, 2H), 8.23 (d, *J* = 8.3 Hz, 1H), 7.95 (d, *J* = 8.8 Hz, 1H), 7.49 (dd, *J* = 8.3, 4.2 Hz, 1H), 7.42 (br s, 1H, NH, D₂O exchanged), 6.43 (s, 1H), 3.27 (d, *J* = 6.6 Hz, 2H), 1.85 - 1.56 (m, 6H), 1.34 - 1.10 (m, 3H), 1.10 - 0.92 (m, 2H). MS (ESI⁺) : [M+H]+ 335.2. |
| **46** | 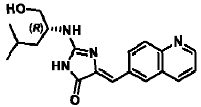 | C₁₉H₂₂N₄O₂ | 338.41 | 97% | Orange solid. ¹H NMR (400 MHz, DMSO-*d*6, 343K) of major tautomer δ_{H} 10.32 (br s, 1H, NH, D₂O exchanged), 8.96 - 8.75 (m, 1H), 8.62 - 8.44 (m, 2H), 8.21 (d, *J* = 8.3 Hz, 1H), 7.93 (d, *J* = 8.7 Hz, 1H), 7.61 - 7.42 (m, 1H), 7.13 (br s, 1H, NH, D₂O exchanged), 6.44 (s, 1H), 4.70 (br s, 1H, OH, D₂O exchanged), 4.06 (br s, 1H), 3.54 (s, 2H), 1.85 - 1.62 (m, 1H), 1.60 - 1.39 (m, 2H), 1.07 - 0.77 (m, 6H). MS (ESI⁺) : [M+H]⁺ 339.3. |
| **47** | 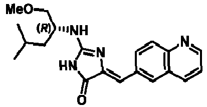 | C₂₀H₂₄N₄O₂ | 352.44 | >98% | Yellow solid. ¹H NMR (400 MHz, DMSO-*d*₆, 343K) of major tautomer δ_{H} 10.34 (br s, 1H, NH, D₂O exchanged), 8.92 - 8.77 (m, 1H), 8.51 (br s, 2H), 8.21 (d, *J* = 8.3 Hz, 1H), 8.04 - 7.84 (m, 1H), 7.58 - 7.43 (m, 1H), 7.26 (br s, 1H, NH, D₂O exchanged), 6.45 (s, 1H), 4.22 (br s, 1H), 3.52 - 3.39 (m, 2H), 3.33 (s, 3H), 1.81 - 1.64 (m, 1H), 1.62 - 1.34 (m, 2H), 0.97 (d, *J* = 7.0 Hz, 6H). MS (ESI⁺) : [M+H]⁺ 353.3. |
| **48** | 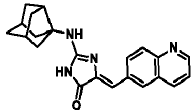 | C₂₂H₂₂N₄O | 358.45 | 95% | Yellow solid. ¹H NMR (400 MHz, DMSO-*d*₆, 343K) of major tautomer δ_{H} 9.98 (br s, 1H, NH, D₂O exchanged), 8.83 (dd, *J =* 4.2, 1.7 Hz, 1H), 8.64 (s, 1H), 8.42 (dd, *J* = 8.8, 1.9 Hz, 1H), 8.16 (d, *J* = 8.3 Hz, 1H), 7.92 (d, *J* = 8.8 Hz, 1H), 7.49 (dd, *J* = 8.3, 4.2 Hz, 1H), 7.33 (br s, 1H, NH, D₂O exchanged), 6.45 (s, 1H), 2.79 - 2.70 (m, 1H), 2.40 - 2.26 (m, 4H), 2.26 - 2.14 (m, 2H), 2.09 - 1.96 (m, 2H), 1.75 - 1.51 (m, 4H). MS (ESI+) : [M+H]⁺ 359.2. |
| **49** | 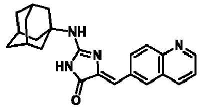 | C₂₃H₂₄N₄O | 372.47 | >98% | Beige solid. ¹H NMR (400 MHz, DMSO-*d*₆, 300K) of major tautomer δ_{H} 10.00 (br s, 1H, NH, D₂O exchanged), 8.86 - 8.80 (m, 1H), 8.64 (d, *J* = 1.9 Hz, 1H), 8.47 (dd, *J* = 8.8, 1.9 Hz, 1H), 8.16 (d, *J* = 8.2 Hz, 1H), 7.94 (d, *J* = 8.8 Hz, 1H), 7.52 (dd, *J* = 8.3, 4.2 Hz, 1H), 7.11 (br s, 1H, NH, D₂O exchanged), 6.43 (s, 1H), 2.27 - 2.03 (m, 9H), 1.84 - 1.62 (m, 6H). MS (ESI⁺) : [M+H]⁺ 373.3. |
| **50** | 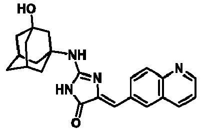 | C₂₃H₂₄N₄O₂ | 388.47 | >98% | Pale yellow solid. ¹H NMR (400 MHz, DMSO-*d*₆, 343K) of major tautomer δ_{H} 9.80 (br s, 1H, NH, D₂O exchanged), 8.89 - 8.79 (m, 1H), 8.66 (s, 1H), 8.42 (d, *J* = 8.8 Hz, 1H), 8.19 (d, *J* = 8.1 Hz, 1H), 7.94 (d, *J* = 8.8 Hz, 1H), 7.50 (dd, *J* = 8.3, 4.2 Hz, 1H), 6.88 (br s, 1H, NH, D₂O exchanged), 6.45 (s, 1H), 4.43 (br s, 1H, OH, D₂O exchanged), 2.35 - 2.19 (m, 2H), 2.17 - 1.92 (m, 6H), 1.76 - 1.46 (m, 6H). MS (ESI⁺) : [M+H]⁺ 389.3. |
| **51** | 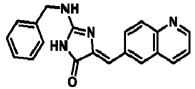 | C₂₀H₁₆N₄O | 328.38 | >98%% | Pale yellow solid. ¹H NMR (400 MHz, DMSO-*d*₆, 343K) of major tautomer δ_{H} 10.66 (br s, 1H, NH, D₂O exchanged), 8.92 - 8.75 (m, 1H), 8.65 - 8.41 (m, 2H), 8.25 (d, *J* = 8.3 Hz, 1H), 8.13 - 7.75 (m, 1H + NH, D₂O exchanged), 7.55 - 7.41 (m, 3H), 7.41 - 7.33 (m, 2H), 7.32 - 7.24 (m, 1H), 6.48 (s, 1H), 4.64 (s, 2H). MS (ESI⁺) : [M+H]⁺ 329.3. |
| **52** | 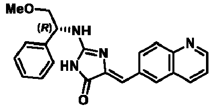 | C₂₂H₂₀N₄O₂ | 372.43 | >98% | Yellow solid. ¹H NMR (400 MHz, DMSO-*d*₆, 373K) of major tautomer δ_{H} 10.42 (br s, 1H, NH, D₂O exchanged), 8.88 - 8.78 (m, 1H), 8.47 (br s, 2H), 8.25 (d, *J* = 8.3 Hz, 1H), 8.10 - 7.80 (m, 1H + NH, D₂O exchanged), 7.58 - 7.44 (m, 3H), 7.42 - 7.34 (m, 2H), 7.31 - 7.24 (m, 1H), 6.45 (s, 1H), 5.33 - 5.18 (m, 1H), 3.78 (dd, *J* = 10.2, 7.4 Hz, 1H), 3.68 (dd, *J* = 10.2, 5.1 Hz, 1H), 3.35 (s, 3H). MS (ESI⁺) : [M+H]⁺ 373.2. |
| **53** | 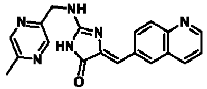 | C₉H₁₆N₆O | 344.38 | >98% | Yellow solid. ¹H NMR (400 MHz, DMSO-*d*₆, 323K) of major tautomer δ_{H} 10.80 (br s, 1H, NH, D₂O exchanged), 8.93 - 8.80 (m, 1H), 8.68 (br s, 1H), 8.62 - 8.38 (m, 3H), 8.29 (d, *J* = 8.3 Hz, 1H), 8.08 (br s, 1H, NH, D₂O exchanged), 7.93 (d, *J* = 8.7 Hz, 1H), 7.50 (dd, *J* = 8.3, 4.2 Hz, 1H), 6.47 (s, 1H), 4.74 (s, 2H), 2.48 (s, 3H). MS (ESI⁺) : [M+H]⁺ 345.2. |
| **54** | 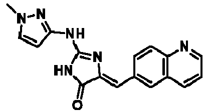 | C₁₇H₁₄N₆O | 318.34 | 97% | Yellow solid. ¹H NMR (400 MHz, CF₃COOD, 300K) of major tautomer δ_{H} 9.78 (d, *J* = 8.5 Hz, 1H), 9.68 (br s, 1H), 9.16 (br s, 1H), 9.04 - 8.80 (m, 2H), 8.77 - 8.62 (m, 1H), 8.05 (br s, 2H), 6.84 (s, 1H), 4.51 (s, 3H). MS (ESI⁺) : [M+H]⁺ 319.2. |
| **55** | 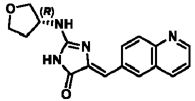 | C₁₇H₁₆N₄O₂ | 308.34 | >98% | Yellow solid. ¹H NMR (400 MHz, DMSO-*d*₆, 343K) of major tautomer δ_{H} 10.49 (br s, 1H, NH, D₂O exchanged), 8.92 - 8.78 (m, 1H), 8.56 (d, *J* = 8.8 Hz, 1H), 8.50 (s, 1H), 8.26 (d, *J* = 8.3 Hz, 1H), 7.96 (d, *J* = 8.8 Hz, 1H), 7.71 (br s, 1H, NH, D₂O exchanged), 7.48 (dd, *J* = 8.2, 4.2 Hz, 1H), 6.49 (s, 1H), 4.53 (s, 1H), 4.02 - 3.85 (m, 2H), 3.82 - 3.74 (m, 1H), 3.69 (dd, *J* = 9.1, 4.1 Hz, 1H), 2.36 - 2.21 (m, 1H), 2.08 - 1.89 (m, 1H). MS (ESI⁺) : [M+H]⁺ 309.1. |
| **56** | 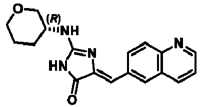 | C₁₈H₁₈N₄O₂ | 322.37 | >98% | Yellow solid. ¹H NMR (400 MHz, DMSO-*d*₆, 343K) of major tautomer δ_{H} 10.34 (br s, 1H, NH, D₂O exchanged), 8.93 - 8.77 (m, 1H), 8.70 - 8.51 (m, 1H), 8.45 (s, 1H), 8.25 (d, *J* = 8.2 Hz, 1H), 7.96 (d, *J* = 8.8 Hz, 1H), 7.65 - 7.45 (m, 1H), 7.42 (br s, 1H, NH, D₂O exchanged), 6.48 (s, 1H), 4.06 - 3.80 (m, 2H), 3.78 - 3.65 (m, 1H), 3.59 - 3.32 (m, 2H), 2.08 - 1.92 (m, 1H), 1.82 - 1.53 (m, 3H). MS (ESI⁺) : [M+H]⁺ 323.2. |
| **57** | 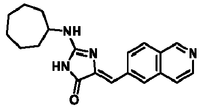 | C₂₀H₂₂N₄O | 334.42 | 97% | Yellow solid. ¹H NMR (400 MHz, DMSO-*d*₆, 343K) of major tautomer δ_{H} 10.41 (br s, 1H, NH, D₂O exchanged), 9.21 (s, 1H), 8.64 - 8.42 (m, 2H), 8.38 (br s, 1H), 8.03 (d, *J* = 8.5 Hz, 1H), 7.67 (d, *J* = 5.8 Hz, 1H), 7.50 (br s, 1H, NH, D₂O exchanged), 6.41 (s, 1H), 3.99 (s, 1H), 2.06 - 1.91 (m, 2H), 1.82 - 1.42 (m, 10H). MS (ESI⁺) : [M+H]⁺ 335.2. |
| **58** | 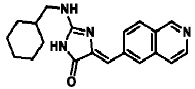 | C₂₀H₂₂N₄O | 334.42 | >98% | Yellow solid. ¹H NMR (400 MHz, DMSO-*d*₆, 343K) of major tautomer δ_{H} 10.48 (br s, 1H, NH, D₂O exchanged), 9.20 (s, 1H), 8.56 - 8.39 (m, 2H), 8.39 - 8.26 (m, 1H), 8.02 (d, *J* = 8.6 Hz, 1H), 7.67 (d, *J* = 5.8 Hz, 1H), 7.59 (br s, 1H, NH, D₂O exchanged), 6.40 (s, 1H), 3.28 (d, *J* = 6.7 Hz, 2H), 1.86 - 1.53 (m, 6H), 1.36 - 1.12 (m, 3H), 1.12 - 0.94 (m, 2H). MS (ESI⁺) : [M+H]⁺ 335.2. |
| **59** | 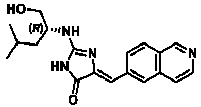 | C₁₉H₂₂N₄O₂ | 338.41 | 95% | Yellow solid. ¹H NMR (400 MHz, DMSO-*d*₆, 343K) of major tautomer δ_{H} 10.36 (br s, 1H, NH, D₂O exchanged), 9.21 (s, 1H), 8.74 - 8.17 (m, 3H), 8.02 (d, *J* = 8.6 Hz, 1H), 7.66 (d, *J* = 5.7 Hz, 1H), 7.26 (br s, 1H, NH, D₂O exchanged), 6.42 (s, 1H), 4.71 (br s, 1H, OH, D₂O exchanged), 4.06 (br s, 1H), 3.63 - 3.45 (m, 2H), 1.79 - 1.64 (m, 1H), 1.59 - 1.40 (m, 2H), 0.98 (d, *J* = 6.6 Hz, 6H). MS (ESI⁺) : [M+H]⁺ 339.2. |
| **60** | 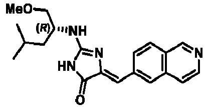 | C₂₀H₂₄N₄O₂ | 352.44 | 97% | Yellow solid. ¹H NMR (400 MHz, DMSO-*d*₆, 343K) of major tautomer δ_{H} 10.47 (br s, 1H, NH, D₂O exchanged), 9.21 (s, 1H), 8.73 - 8.24 (m, 3H), 8.02 (d, *J* = 8.6 Hz, 1H), 7.73 - 7.59 (m, 1H), 7.38 (br s, 1H, NH, D₂O exchanged), 6.43 (s, 1H), 4.23 (br s, 1H), 3.54 - 3.40 (m, 2H), 3.33 (s, 3H), 1.81 - 1.64 (m, 1H), 1.61 - 1.37 (m, 2H), 1.06 - 0.86 (m, 6H). MS (ESI⁺) : [M+H]⁺ 353.4. |
| **61** | 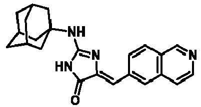 | C₂₃H₂₄N₄O | 372.47 | 97% | Yellow solid. ¹H NMR (400 MHz, DMSO-*d*₆, 343K) of major tautomer δ_{H} 9.84 (br s, 1H, NH, D₂O exchanged), 9.21 (s, 1H), 8.59 (s, 1H), 8.46 (d, *J* = 5.7 Hz, 1H), 8.38 (d, *J* = 8.6 Hz, 1H), 8.03 (d, *J* = 8.6 Hz, 1H), 7.61 (d, *J* = 5.7 Hz, 1H), 6.91 (br s, 1H, NH, D₂O exchanged), 6.43 (s, 1H), 2.28 - 2.04 (m, 9H), 1.82 - 1.65 (m, 6H). MS (ESI⁺) : [M+H]⁺ 373.4. |
| **62** | 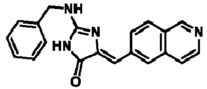 | C₂₀H₁₆N₄O | 328.38 | 96% | Yellow solid. ¹H NMR (400 MHz, DMSO-*d*₆, 323K) of major tautomer δ_{H} 10.05 (br s, 1H, NH, D₂O exchanged), 9.21 (s, 1H), 8.50 - 8.44 (m, 2H), 8.40 (s, 1H), 8.15 (br s, 1H, NH, D₂O exchanged), 8.04 (d, *J* = 8.6 Hz, 1H), 7.71 (d, *J* = 5.5 Hz, 1H), 7.57 - 7.33 (m, 4H), 7.32 - 7.25 (m, 1H), 6.44 (s, 1H), 4.63 (s, 2H). MS (ESI⁺) : [M+H]⁺ 329.2. |
| **63** | 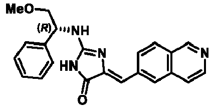 | C₂₂H₂₀N₄O₂ | 372.43 | 98% | Yellow solid. ¹H NMR (400 MHz, DMSO-*d*₆, 343K) of major tautomer δ_{H} 10.52 (br s, 1H, NH, D₂O exchanged), 9.21 (s, 1H), 8.57 - 8.40 (m, 2H), 8.35 (d, *J* = 8.6 Hz, 1H), 8.07 (br s, 1H, NH, D₂O exchanged), 8.01 (d, *J* = 8.6 Hz, 1H), 7.75 - 7.67 (m, 1H), 7.60 - 7.44 (m, 2H), 7.39 (t, *J* = 7.5 Hz, 2H), 7.33 - 7.23 (m, 1H), 6.43 (s, 1H), 5.26 (br s, 1H), 3.83 - 3.73 (m, 1H), 3.68 (dd, *J* = 10.3, 5.1 Hz, 1H), 3.35 (s, 3H). MS (ESI⁺) : [M+H]⁺ 373.3. |
| **64** | 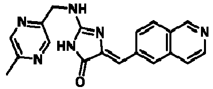 | C₁₉H₁₆N₆O | 344.38 | 98% | Yellow solid. ¹H NMR (400 MHz, DMSO-*d*₆, 323K) of major tautomer δ_{H} 10.84 (br s, 1H, NH, D₂O exchanged), 9.21 (s, 1H), 8.68 (s, 1H), 8.53 (s, 1H), 8.50 - 8.42 (m, 2H), 8.35 (d, *J* = 8.5 Hz, 1H), 8.19 (br s, 1H, NH, D₂O exchanged), 8.01 (d, *J* = 8.6 Hz, 1H), 7.73 (d, *J* = 5.8 Hz, 1H), 6.45 (s, 1H), 4.74 (s, 2H), 2.48 (s, 3H). MS (ESI⁺) : [M+H]⁺ 345.2. |
| **65** | 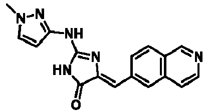 | C₁₇H₁₄N₆O | 318.34 | 95% | Yellow solid. ¹H NMR (400 MHz, CF₃COOD, 300K) of major tautomer δ_{H} 10.28 - 10.00 (m, 1H), 9.24 - 8.90 (m, 4H), 8.79 - 8.58 (m, 1H), 8.11-7.90 (m, 2H), 6.81 (s, 1H), 4.47 (s, 3H). MS (ESI⁺) : [M+H]⁺ 319.2. |
| **66** | 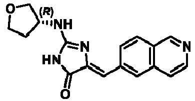 | C₁₇H₁₆N₄O₂ | 308.34 | 95% | Yellow solid. ¹H NMR (400 MHz, DMSO-*d*₆, 343K) of major tautomer δ_{H} 9.79 (br s, 1H, NH, D₂O exchanged), 9.21 (s, 1H), 8.62 - 8.30 (m, 3H), 8.03 (d, *J* = 8.6 Hz, 1H), 7.83 (br s, 1H, NH, D₂O exchanged), 7.71 (d, *J* = 5.8 Hz, 1H), 6.46 (s, 1H), 4.53 (br s, 1H), 4.01 - 3.84 (m, 2H), 3.83 - 3.73 (m, 1H), 3.69 (dd, *J =* 9.1, 4.1 Hz, 1H), 2.35 - 2.22 (m, 1H), 2.04 - 1.92 (m, 1H). MS (ESI⁺) : [M+H]⁺ 309.2. |
| **67** | 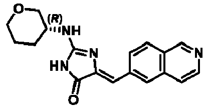 | C₁₈H₁₈N₄O₂ | 322.37 | 93% | Yellow solid. ¹H NMR (400 MHz, DMSO-*d*₆, 343K) of major tautomer δ_{H} 10.43 (br s, 1H, NH, D₂O exchanged), 9.21 (s, 1H), 8.46 (d, *J* = 5.6 Hz, 1H), 8.43 - 8.34 (m, 2H), 8.04 (d, *J* = 8.6 Hz, 1H), 7.70 (d, *J* = 5.8 Hz, 1H), 7.55 (br s, 1H, NH, D₂O exchanged), 6.46 (s, 1H), 4.04 - 3.83 (m, 2H), 3.79 - 3.68 (m, 1H), 3.54 - 3.33 (m, 2H), 2.07 - 1.93 (m, 1H), 1.85 - 1.52 (m, 3H). MS (ESI⁺) : [M+H]⁺ 323.2. |
| **68** | 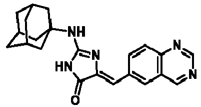 | C₂₂H₂₃N₅O | 373.46 | 97% | Orange solid. ¹H NMR (400 MHz, DMSO-*d*₆, 323K) of major tautomer δ_{H} 9.95 (s, 1H, NH), 9.39 (s, 1H), 9.23 (s, 1H), 8.80 - 8.77 (m, 2H), 8.72 (dd, *J* = 8.8, 1.8 Hz, 1H), 7.06 (s, 1H), 6.46 (s, 1H), 2.25 - 2.00 (m, 9H), 1.85 - 1.60 (m, 6H). MS (ESI⁺) : [M+H]⁺ 374.1. |
| **69** | 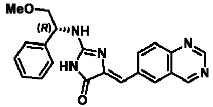 | C₂₁H₁₉N₅O₂ | 373.42 | >98% | Yellow solid. ¹H NMR (400 MHz, DMSO-*d*₆, 323K) of major, tautomer δ_{H} 10.58 (br s, 1H, NH, D₂O exchanged), 9.50 (s, 1H), 9.23 (s, 1H), 8.85 - 8.55 (m, 2H), 8.23 (br s, 1H, NH, D₂O exchanged), 7.95 (d, *J* = 8.8 Hz, 1H), 7.62 - 7.48 (m, 2H), 7.39 (t, *J* = 7.6 Hz, 2H), 7.32 - 7.23 (m, 1H), 6.46 (s, 1H), 5.29 (br s, 1H), 3.85 - 3.73 (m, 1H), 3.66 (dd, *J* = 10.1, 4.9 Hz, 1H), 3.35 (s, 3H). MS (ESI⁺) : [M+H]⁺ 374.1. |
| **70** | 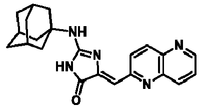 | C₂₂H₂₃N₅O | 373.46 | >98% | Pale yellow solid. ¹H NMR (400 MHz, DMSO-*d*₆, 323K) of major tautomer δ_{H} 10.63 (br s, 1H, NH, D₂O exchanged), 8.96 (d, *J* = 4.1 Hz, 1H), 8.49 (d, *J* = 8.5 Hz, 1H), 8.37 (d, *J* = 8.7 Hz, 1H), 8.13 (br s, 1H, NH, D₂O exchanged), 7.90 (d, *J* = 8.7 Hz, 1H), 7.84 (dd, *J* = 8.5, 4.1 Hz, 1H), 6.50 (s, 1H), 2.27 - 2.01 (m, 9H), 1.87 - 1.59 (m, 6H). MS (ESI⁺) : [M+H]⁺ 374.1. |
| **71** | 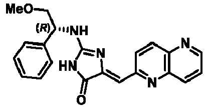 | C₂₁H₁₉N₅O₂ | 373.42 | 97% | Yellow solid. ¹H NMR (400 MHz, DMSO-*d*₆, 323K) of major tautomer δ_{H} 10.86 (br s, 1H, NH, D₂O exchanged), 9.10 - 8.75 (m, 1H + NH, D₂O exchanged), 8.51 (d, *J* = 8.5 Hz, 1H), 8.38 (d, *J* = 8.6 Hz, 1H), 7.92 (d, *J* = 8.7 Hz, 1H), 7.85 (dd, *J* = 8.4, 4.0 Hz, 1H), 7.50 - 7.25 (m, 5H), 6.55 (s, 1H), 5.28 (br s, 1H), 3.90 - 3.60 (m, 2H), 3.32 (s, 3H). MS (ESI⁺) : [M+H]+ 374.1. |
| **72** | 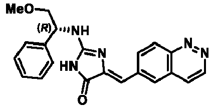 | C₂₁H₁₉N₅O₂ | 373.42 | 98% | Orange solid. ¹H NMR (400 MHz, DMSO-*d*₆, 323K) of major tautomer δ_{H} 10.65 (br s, 1H, NH, D₂O exchanged), 9.30 (d, *J =* 5.8 Hz, 1H), 8.85 - 8.15 (m, 2H + NH, D₂O exchanged), 8.35 (d, *J* = 9.0 Hz, 1H), 8.06 (d, *J* = 5.8 Hz, 1H), 7.56 - 7.48 (m, 2H), 7.39 (app t, *J* = 7.6 Hz, 2H), 7.29 (app t, *J* = 7.4 Hz, 1H), 6.44 (s, 1H), 5.30 (br s, 1H), 3.85 - 3.73 (m, 1H), 3.72 - 3.60 (m, 1H), 3.36 (s, 3H). MS (ESI⁺) : [M+H]⁺ 374.1. |
| **73** | 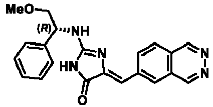 | C₂₁H₁₉N₅O₂ | 373.42 | 94% | Orange solid. ¹H NMR (400 MHz, DMSO-*d*₆, 373K) of major tautomer δ_{H} 10.36 (br s, 1H, NH, D₂O exchanged), 9.52 (d, *J =* 4.2 Hz, 2H), 8.59 (br s, 2H), 8.03 (d, *J* = 8.4 Hz, 1H), 7.95 (br s, 1H, NH, D₂O exchanged), 7.56 - 7.48 (m, 2H), 7.39 (app t, *J =* 7.5 Hz, 2H), 7.29 (app t, *J* = 7.2 Hz, 1H), 6.47 (s, 1H), 5.29 (br s, 1H), 3.81 (dd, *J* = 10.7, 7.7 Hz, 1H), 3.73 (dd, *J* = 10.5, 5.5 Hz, 1H), 3.38 (s, 3H). MS (ESI⁺) : [M+H]⁺ 374.2. |

### PATHOLOGIES

The compounds of formula (I) may be useful in the treatment and/or in the prevention of a disease selected from cognitive deficits and neuroinflammation associated with Down syndrome (Trisomy 21), Alzheimer's disease and related diseases, dementia; tauopathies; and other neurodegenerative diseases (Parkinson's disease; Pick disease, including Niemann-Pick Type C Disease); CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as malaria, Leishmaniasis, Chagas and sleeping sickness *(Trypanosoma* sp.), cattle diseases due to unicellular pathogens and for regulating body temperature.

According to a particular embodiment, the compounds of formula (I) of the present invention may be useful in the treatment and/or in the prevention of a disease selected from cognitive deficits and neuroinflammation associated with Down syndrome (Trisomy 21), Alzheimer's disease and related diseases, dementia or tauopathies; other neurodegenerative diseases (Parkinson's disease; Pick disease, including Niemann-Pick Type C Disease); CDKL5 Deficiency Disorder; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC) and viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature. Said diseases are more particularly associated with the abnormalities in DYRK1A and/or CLK1 dosage.

Still according to this particular embodiment, the compounds of formula (I) of the present invention may be useful in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions, more particularly due to high expression and activity of DYRK1A.

Still according to this particular embodiment, the compounds of formula (I) of the present invention may be useful in the treatment and/or prevention of a disease selected from Down syndrome, Alzheimer's disease and related Tauopathies, Parkinson's disease, the cognitive/motor disorders associated therewith, or one or more symptoms of such diseases. As a typical symptom of such diseases is a decline in learning and memory and social interactions.

Still according to this particular embodiment, the compounds of formula (I) of the present invention may be useful in combatting cognitive decline associated with Down syndrome (Trisomy 21), in learning and memory, in particular associated with the cognitive or neurodegenerative disorders as mentioned above.

Still according to this particular embodiment, the compounds of formula (I) of the present invention may be useful in the treatment and/or prevention of type 1 and type 2 diabetes.

The compounds of formula (I) of the present invention may be useful in the treatment and/or prevention of type 1 and type 2 diabetes either by direct treatment of the diabetic patient or by treating isolated/cultured pancreatic islets or β-cells *in vitro* or *ex vivo* prior to transplantation into diabetic patients.

Herein is further provided a method of treatment of type 1 and type 2 diabetes in a patient in need thereof comprising a step of administering a compound of formula (I) as defined above.

Herein is further provided a method of treatment of type 1 and type 2 diabetes in a patient in need thereof comprising a step of treating isolated or cultured pancreatic islets or β-cells *in vitro* or *ex vivo* prior to transplantation into said patient with a compound of formula (I) as defined above.

Still according to this particular embodiment, the compounds of formula (I) of thre present invention may be useful in the treatment and/or prevention of viral infections, in particular caused by such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV), and in particular by Herpes, Coronaviruses, Cytomegaloviruses and Influenzas. These infections may be associated with high expression and activity of DYRK1A and/or CLK1, and optionally additionally with dual inhibitors of CLKs/DYRKS.

Acute respiratory disease has recently been caused by a new coronavirus (SARS-CoV-2, previously known as 2019-nCoV), also known here as coronavirus 2019 (COVID-19), which belongs to *Coronaviridae.* The compounds of formula (I) according to the present invention can also treat said infection caused by SARS-CoV-2 virus.

Still according to this particular embodiment, the compounds of formula (I) of the present invention may be useful in the treatment and/or prevention of cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC). These cancers may be associated with high expression and activity of DYRK1A and/or CLK1, and optionally additionally with dual inhibitors of CLKs/DYRKS.

Still according to this particular embodiment, the compounds of formula (I) of the present invention may be useful in the treatment and/or prevention of tendinopathy and osteoarthritis. Tendionpathy and osteoarthritis may be associated with high expression and activity of DYRK1A and/or CLK2.

Still according to this particular embodiment, the compounds of formula (I) of the present invention may be useful in the treatment and/or prevention of infections caused by unicellular parasites, such as such as malaria, Leishmaniasis, Chagas and sleeping sickness *(Trypanosoma* sp.), and cattle diseases due to unicellular pathogens Said parasitic infections may be associated with expression and activity of DYRKs/CLKs.

Still according to this particular embodiment, the compounds of formula (I) of the present invention may be useful in the regulation of the body temperature. Said body temperature regulation may be associated with expression and activity of CLKs.

According to another particular embodiment, the compounds of formula (I) of the present invention may be useful in the treatment and/or in the prevention of a disease selected from Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as such as malaria, Leishmaniasis, Chagas and sleeping sickness *(Trypanosoma* sp.), and cattle diseases due to unicellular pathogens. Said diseases are more particularly associated with the abnormalities in other DYRKs (DYR1B, 2, 3, 4) and the closely related further cdc2-like kinases (CLKs) (CLK 2, 3, 4).

The following examples are provided as illustrations and in no way limit the scope of this invention.

The following examples illustrate in detail the preparation of some compounds according to the invention. The structures of the products obtained have been confirmed by NMR analyses and mass spectroscopy.

### Exemple 1: S-alkylation of 2-thiohydantoins

### Example 1.1: Synthesis of 2-methylsulfanyl-1,4-dihydroimidazol-5-one (1.1)

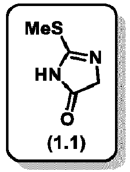

MeI (51.4 mL, 0.827 mol, 4 eq) was slowly added dropwise to a stirred suspension of 2-thiohydantoin (24 g, 206.6 mmol, 1 eq), DIPEA (72 mL, 413.2 mmol, 2 eq) and DMAP (10.096 g, 82.64 mmol, 0.4 eq) in DCM (413 mL) maintained at 0 °C. The resulting mixture was stirred 6h at 0 °C. A precipitate gradually appeared. Upon completion (TLC), the precipitate was filtered on a fritted glass funnel. The resulting solid was adsorbed on silica and purified by FC on silica gel (elution : DCM/MeOH : 99/1 to 9/1). The volume of the collected fractions was reduced to approximately an eighth of its initial volume, until yellow crystals started to appear. The mixture was stirred 30 min at 0 °C and the solid was collected by filtration on a fritted glass funnel to yield 2-methylsulfanyl-1,4-dihydroimidazol-5-one (15.368 g, 118.1 mmol, 57%) in analytically pure form. Pale yellow solid. ¹H NMR (400 MHz, DMSO-*d*₆, 300K) of major tautomer δ_{H} 11.24 (br s, 1H, NH, D₂O exchanged), 4.01 (s, 2H), 2.47 (s, 3H). MS (ESI⁺) : [M+H]+ 131.0.

### Example 1.2: Synthesis of 1-methyl-2-methylsulfanyl-4H-imidazol-5-one (1.2)

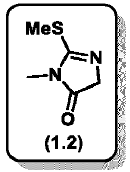

MeO₃BF₄ (6.818 g, 46.1 mmol, 1.5 eq) was added portionwise to a stirred solution of 3-methyl-2-thiohydantoin (4 g, 30.73 mmol, 1 eq) in DCM at stirred at room temperature for 12h. Upon completion (TLC), the resulting mixture was quenched with sat. Na₂CO_{3(aq)}. The aqueous layer was extracted three times with DCM. The combined organic layers were dried over MgSO₄, filtered, concentrated *in vacuo,* adsorbed on silica, and purified by FC on silica gel (elution : Cyclohexane/AcOEt/DCM: 92/5/3 to 50/47/3) to give the desired isothiourea (3.797 g, 26.33 mmol, 85%) in analytically pure form. ¹H NMR (400 MHz, DMSO-*d*₆, 300K) δ_{H} 4.11 (s, 2H), 2.93 (s, 3H), 2.51 (s, 3H). ¹³C NMR (101 MHz, DMSO) δ_{C} 179.6, 162.6, 58.3, 25.9, 11.8. MS (ESI⁺) : [M+H]⁺ 145.0.

### Example 2: General Protocol 1 - Addition of aliphatic and aromatic amines on 2-alkylsulfanyl-1,4-dihydroimidazol-5-ones - ROUTE 1

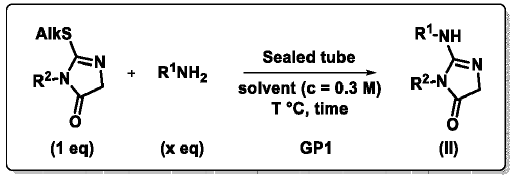

In the above scheme, R¹ is as defined above and R² represents a hydrogen atom, a (C₁-C₄)alkyl or a (C3-C6)cycloalkyl group, in particular R² represents a hydrogen atom or a methyl group.

**GP1**: the appropriate amine (x eq) was added to a stirred solution of the appropriate 2-alkylsulfanyl-1,4-dihydroimidazol-5-one^{(a)} (1 eq) in dry THF (C = 0.3 M/isothiourea) in a sealed tube or sealed round flask (heating block). The mixture was thoroughly purged with vacuum/argon cycles and heated at the appropriate temperature for the indicated time (see details below). Upon completion (followed by consumption of the isothiourea on TLC), the mixture was brought back to room temperature.
- **GP1-A: direct precipitation of the desired product:** The reaction medium was stirred 1h at 0 °C. The precipitated solid was filtered off on a fritted glass funnel. High purity may be achieved after filtration by washing, reprecipitation, trituration, or recrystallization.
- **GP1-B: the product failed to precipitate:** the reaction mixture was poured on Et₂O maintained at 0 °C. The precipitated solid was filtered off on a fritted glass funnel. High purity may be achieved after filtration by washing, reprecipitation, trituration, or recrystallization.
- **GP1-C: the product failed to precipitate:** the reaction mixture was concentrated *in vacuo,* adsorbed on silica, and purified by FC. High purity may be achieved after purification by washing, reprecipitation, trituration, or recrystallization.
- (a) May require activation with AcOH, depending on the amine (see details below).

### Example 2.1: Synthesis of 2-(cyclohexylamino)-1,4-dihydroimidazol-5-one (2.1)

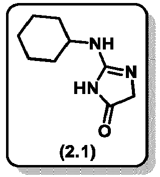

Compound (2.1) was synthesized according to **GP1-A** : reaction carried out in THF, with 2-methylsulfanyl-1,4-dihydroimidazol-5-one (1.1) (8.91 mmol) and 4 eq of cyclohexylamine, at 120 °C (heating block), for 12h. The product directly precipitated in the reaction medium: it was isolated after filtration, washing with cold THF, then pentane. Off-white solid, 35% (571 mg). ¹H NMR (400 MHz, DMSO-*d*₆, 300K) of major tautomer δ_{H} 7.41 (br s, 1H, NH, D₂O exchanged), 6.95 (br s, 1H, NH, D₂O exchanged), 3.61 (s, 2H), 3.39 - 3.23 (m, 1H), 1.88 - 1.75 (m, 2H), 1.74 - 1.62 (m, 2H), 1.61 - 1.51 (m, 1H), 1.33 - 1.03 (m, 5H). MS (ESI⁺) : [M+H]⁺ 182.0.

### Example 2.2: Synthesis of 2-(cycloheptylamino)-1,4-dihydroimidazol-5-one (2.2)

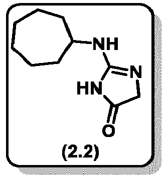

Compound (2.2) was synthesized according to **GP1-A** : reaction carried out in THF, with 2-methylsulfanyl-1,4-dihydroimidazol-5-one (1.1) (11.52 mmol) and 4 eq of cycloheptylamine, at 120 °C (sealed tube, heating bock), for 12h. The product directly precipitated in the reaction medium: it was isolated after filtration, washing with cold THF, then pentane. Off-white solid, 64% (1.429 g). ¹H NMR (400 MHz, DMSO-*d*₆, 300K) of major tautomer δ_{H} 7.42 (br s, 1H, NH, D₂O exchanged), 6.88 (br s, 1H, NH, D₂O exchanged), 3.86 - 3.68 (m, 1H), 3.59 (s, 2H), 1.89 - 1.76 (m, 2H), 1.70 - 1.32 (m, 10H). MS (ESI⁺) : [M+H]⁺ 196.0.

### Example 2.3 : Synthesis of 2-(cyclooctylamino)-1,4-dihydroimidazol-5-one (2.3)

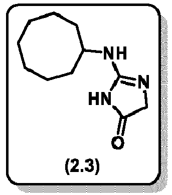

Compound (2.3) was synthesized according to **GP1-A** : reaction carried out in THF, with 2-methylsulfanyl-1,4-dihydroimidazol-5-one (1.1) (11.52 mmol) and 4 eq of cyclooctylamine, at 120 °C (sealed tube, heating bock), for 12h. The product directly precipitated in the reaction medium: it was isolated after filtration, washing with cold THF, then pentane. Off-white solid, 72% (1.739 g). ¹H NMR (400 MHz, DMSO-*d*₆, 300K) of major tautomer δ_{H} 7.43 (br s, 1H, NH, D₂O exchanged), 6.86 (br s, 1H, NH, D₂O exchanged), 3.89 - 3.74 (m, 1H), 3.59 (s, 2H), 1.90 - 1.27 (m, 14H). MS (ESI⁺) : [M+H]⁺ 210.0.

### Example 2.4: Synthesis of 2-[[(1R)-1-(hydroxymethyl)-3-methylbutyl]amino]-1,4-dihydroimidazol-5-one (2.4)

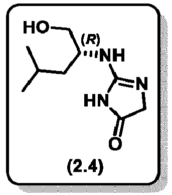

Compound (2.4) was synthesized according to **GP1-A:** reaction carried out in THF with 2-methylsulfanyl-1,4-dihydroimidazol-5-one (1.1) (16.90 mmol), 2.5 eq of (D)-leucinol, at 125 °C, for 4h. The product directly precipitated in the reaction medium: it was isolated after filtration, washing with cold THF, then pentane. Light beige solid, 52% (1.750 g). ¹H NMR (400 MHz, DMSO-*d*₆, 343K) of major tautomer δ_{H} 7.11 (br s, 2H, NH, D₂O exchanged), 4.61 (s, 1H, OH, D₂O exchanged), 3.91 - 3.70 (m, 1H), 3.61 (s, 2H), 3.46 - 3.28 (m, 2H), 1.70 - 1.56 (m, 1H), 1.37 (t, *J* = 7.0 Hz, 2H), 0.89 (t, *J* = 7.1 Hz, 6H). MS (ESI⁺) : [M+H]⁺ 200.1.

### Example 2.5 : Synthesis of 2-[[(1R)-1-(methoxymethyl)-3-methylbutyl]amino]-1,4-dihydroimidazol-5-one (2.5)

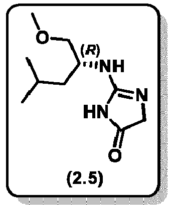

Compound (2.5) was synthesized according to **GP1-B** : reaction carried out in THF, with 2-methylsulfanyl-1,4-dihydroimidazol-5-one (1.1) (11.52 mmol), 2 eq of (2*R*)-1-methoxy-4-methyl-pentan-2-amine and 4 eq of AcOH. Light beige solid, 39% (948 mg). ¹H NMR (400 MHz, DMSO-*d*₆, 343K) of major tautomer δ_{H} 7.20 (br s, 1H, NH, D₂O exchanged), 6.98 (br s, 1H, NH, D₂O exchanged), 4.09 - 3.75 (m, 1H), 3.61 (s, 2H), 3.39 - 3.29 (m, 2H), 3.27 (s, 3H), 1.71 - 1.56 (m, 1H), 1.47 - 1.27 (m, 2H), 0.89 (t, *J* = 6.7 Hz, 6H). MS (ESI⁺) : [M+H]⁺ 214.3.

### Example 2.6: Preparation of 2-[[(1R)-1-(fluoromethyl)-3-methylbutyl]amino]-1,4-dihydroimidazol-5-one (2.6)

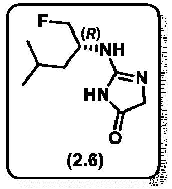

Compound (2.6) was synthesized according to **GP1-B** : reaction carried out in THF, with 2-methylsulfanyl-1,4-dihydroimidazol-5-one (1.1) (4.088 mmol), 1.6 eq of (2R)-1-fluoro-4-methyl-pentan-2-amine and 4 eq of AcOH, at 120 °C (heating block), for 4h. Beige solid, 32% (267 mg). ¹H NMR (400 MHz, DMSO-*d*₆, 343K) of major tautomer δ_{H} 7.39 (br s, 1H, NH, D₂O exchanged), 7.12 (br s, 1H, NH, D₂O exchanged), 4.47 - 4.38 (m, 1H), 4.36 - 4.26 (m, 1H), 4.11 - 3.89 (m, 1H), 3.63 (s, 2H), 1.73 - 1.59 (m, 1H), 1.52 - 1.42 (m, 1H), 1.40 - 1.29 (m, 1H), 0.97 - 0.86 (m, 6H). MS (ESI⁺) : [M+H]⁺ 202.1.

### Example 2.7: Synthesis of 2-[[(1S)-1-(fluoromethyl)-3-methylbutyl]amino]-1,4-dihydroimidazol-5-one (2.7)

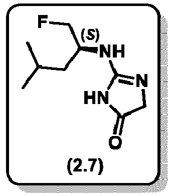

Compound (2.7) was synthesized according to **GP1-B** : reaction carried out in THF, with 2-methylsulfanyl-1,4-dihydroimidazol-5-one (1.1) (4.088 mmol), 1.6 eq of (2*S*)-1-fluoro-4-methyl-pentan-2-amine and 4 eq of AcOH, at 120 °C (heating block), for 4h. Beige solid, 44% (366 mg). ¹H NMR (400 MHz, DMSO-*d*₆, 343K) of major tautomer δ_{H} 7.39 (br s, 1H, NH, D₂O exchanged), 7.12 (br s, 1H, NH, D₂O exchanged), 4.47 - 4.38 (m, 1H), 4.36 - 4.26 (m, 1H), 4.11 - 3.89 (m, 1H), 3.63 (s, 2H), 1.73 - 1.59 (m, 1H), 1.52 - 1.42 (m, 1H), 1.40 - 1.29 (m, 1H), 0.97 - 0.86 (m, 6H). MS (ESI⁺) : [M+H]⁺ 202.1.

### Example 2.8: Synthesis of 2-[[(1R,2R)-2-methoxycyclopentyl]amino]-1,4-dihydroimidazol-5-one (2.8)

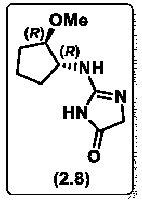

Compound (2.8) was synthesized according to **GP1-A:** reaction carried out in THF with 2-methylsulfanyl-1,4-dihydroimidazol-5-one (1.1) (868 µmol), 3 eq of (1*R,*2*R*)-2-methoxycyclopentanamine, at 115 °C, for 12h. The product directly precipitated in the reaction medium: it was isolated after filtration, washing with cold THF, then pentane. Colorless solid, 74% (127 mg). ¹H NMR (400 MHz, DMSO-*d*₆, 343K) of major tautomer δ_{H} 7.57 (br s, 1H, NH, D₂O exchanged), 7.13 (br s, 1H, NH, D₂O exchanged), 3.99 - 3.79 (m, 1H), 3.69 - 3.63 (m, 1H), 3.61 (s, 2H), 3.25 (s, 3H), 2.05 - 1.83 (m, 2H), 1.73 - 1.40 (m, 4H). MS (ESI⁺) : [M+H]+ 198.1.

### Example 2.9: Synthesis of 2-[[(1S,2S)-2-methoxycyclopentyl]amino]-1,4-dihydroimidazol-5-one (2.9)

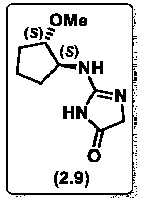

Compound (2.9) was synthesized according to **GP1-A:** reaction carried out in THF with 2-methylsulfanyl-1,4-dihydroimidazol-5-one (1.1) (2.89 mmol), 3 eq of (1S*R,*2*S*)-2-methoxycyclopentanamine, at 115 °C, for 12h. The product directly precipitated in the reaction medium: it was isolated after filtration, washing with cold THF, then pentane. Colorless solid, 71% (405 mg). ¹H NMR (400 MHz, DMSO-*d*₆, 343K) of major tautomer δ_{H} 7.57 (br s, 1H, NH, D₂O exchanged), 7.13 (br s, 1H, NH, D₂O exchanged), 3.99 - 3.79 (m, 1H), 3.69 - 3.63 (m, 1H), 3.61 (s, 2H), 3.25 (s, 3H), 2.05 - 1.83 (m, 2H), 1.73 - 1.40 (m, 4H). MS (ESI⁺) : [M+H]⁺ 198.1.

### Example 2.10: Synthesis of 2-(3-noradamantylamino)-1,4-dihydroimidazol-5-one (2.10)

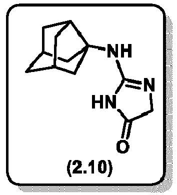

Compound (2.10) was synthesized according to **GP1-C** : reaction carried out in dioxane, with 2-methylsulfanyl-1,4-dihydroimidazol-5-one (1.1) (3.55 mmol), 3 eq of 3-noradamantanamine and 4 eq of AcOH, at 130 °C (heating block), for 12h. Purification by FC (elution: DCM/MeOH (7N NH₃): 99/1 to 9/1). The final product required a trituration in DCM at 0 °C. Light beige solid, 65% (502 mg). ¹H NMR (400 MHz, DMSO-*d*₆, 343K) δ_{H} 7.47 (br s, 1H, NH, D₂O exchanged), 6.64 (br s, 1H, NH, D₂O exchanged), 3.56 (s, 2H), 2.41 (t, *J* = 6.8 Hz, 1H), 2.29 - 2.20 (m, 2H), 2.10 - 1.90 (m, 6H), 1.64 - 1.45 (m, 4H). MS (ESI⁺) : [M+H]⁺ 220.2.

### Example 2.11: Synthesis of 2-(1-adamantylamino)-1,4-dihydroimidazol-5-one (2.11)

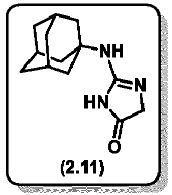

Compound (2.11) was synthesized according to **GP1-C** : reaction carried out in dioxane, with 2-methylsulfanyl-1,4-dihydroimidazol-5-one (1.1) (3.073 mmol), 3 eq of adamantan-1-amine and 4 eq of AcOH, at 150 °C (heating block), for 16h. Purification by FC (elution: DCM/MeOH (7N NH₃): 99/1 to 9/1). The final product required a trituration in DCM at 0 °C. Beige solid, 35% (254 mg). ¹H NMR (400 MHz, DMSO-*d*₆, 300K) δ_{H} 7.18 (br s, 1H, NH, D₂O exchanged), 6.73 (br s, 1H, NH, D₂O exchanged), 3.52 (s, 2H), 2.03 (s, 3H), 1.96 (s, 6H), 1.62 (s, 6H). MS (ESI⁺) : [M+H]⁺ 234.2.

### Example 2.12: Synthesis of 2-[(3-hydroxy-1-adamantyl)amino]-1,4-dihydroimidazol-5-one (2.12)

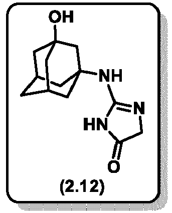

Compound (2.11) was synthesized according to **GP1-C** : reaction carried out in dioxane, with 2-methylsulfanyl-1,4-dihydroimidazol-5-one (1.1) (9.22 mmol), 3 eq of 3-aminoadamantan-1-ol and 4 eq of AcOH, at 145 °C (heating block), for 12h. Purification by FC (elution: DCM/MeOH (7N NH₃): 99/1 to 85/15). The final product required a trituration in refluxing DCM. Beige solid, 58% (1.336 g). ¹H NMR (400 MHz, DMSO-*d*₆, 300K) δ_{H} 7.25 (br s, 1H, NH, D₂O exchanged), 6.75 (br s, 1H, NH, D₂O exchanged), 4.53 (s, 1H, OH, D₂O exchanged), 3.53 (s, 2H), 2.15 (br s, 2H), 1.90 - 1.78 (m, 6H), 1.60 - 1.35 (m, 6H). MS (ESI⁺) : [M+H]⁺ 250.3.

### Example 2.13: Synthesis of 2-[(3-methoxy-1-adamantyl)amino]-1,4-dihydroimidazol-5-one (2.13)

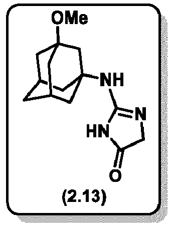

Compound (2.13) was synthesized according to **GP1-C** : reaction carried out in dioxane, with 2-methylsulfanyl-1,4-dihydroimidazol-5-one (1.1) (768 µmol), 3 eq of 3-methoxyadamantan-1-amine and 4 eq of AcOH, at 150 °C (heating block), for 16h. Purification by FC (elution: DCM/MeOH (7N NH₃): 99/1 to 9/1). Brown solid, 50% (102 mg). ¹H NMR (400 MHz, DMSO-*d*₆, 300K) δ_{H} 7.30 (br s, 1H, NH, D₂O exchanged), 6.77 (br s, 1H, NH, D₂O exchanged), 3.53 (s, 2H), 3.12 (s, 3H), 2.21 (br s, 2H), 1.95 - 1.80 (m, 6H), 1.67 - 1.56 (m, 4H), 1.55 - 1.40 (m, 2H). MS (ESI⁺) : [M+H]⁺ 264.3.

### Example 2.14: Synthesis of 2-[(3-fluoro-1-adamantyl)amino]-1,4-dihydroimidazol-5-one (2.14)

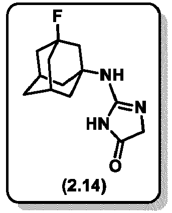

Compound (2.14) was synthesized according to **GP1-C** : reaction carried out in dioxane, with 2-methylsulfanyl-1,4-dihydroimidazol-5-one (1.1) (3.073 mmol), 3 eq of 3-fluoro-adamantan-1-amine and 4 eq of AcOH, at 130 °C (heating block), for 12h. Purification by FC (elution: DCM/MeOH (7N NH₃): 99/1 to 85/15). The final product required a trituration in Et₂O at 0 °C. Beige solid, 44% (343 mg). ¹H NMR (400 MHz, DMSO-*d*₆, 300K) δ_{H} 7.41 (br s, 1H, NH, D₂O exchanged), 6.83 (br s, 1H, NH, D₂O exchanged), 3.54 (s, 2H), 2.35 - 2.24 (m, 2H), 2.14 (d, *J* = 5.9 Hz, 2H), 2.01 - 1.72 (m, 8H), 1.56 - 1.43 (m, 2H). MS (ESI⁺) : [M+H]⁺ 252.3.

### Example 2.15 : Synthesis of 2-[[(1R)-2-methoxy-1-phenyl-ethyl]amino]-1,4-dihydroimidazol-5-one (2.15)

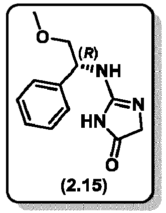

Compound (2.15) was synthesized according to **GP1-A** : reaction carried out in THF, with 2-methylsulfanyl-1,4-dihydroimidazol-5-one (1.1) (3.073 mmol), 3 eq of (1*R*)-2-methoxy-1-phenyl-ethanamine and 4 eq of AcOH, at 115 °C (sealed tube, heating bock), for 12h. The product directly precipitated in the reaction medium: it was isolated after filtration, washing with cold THF, then pentane. Beige solid, 58% (414 mg). ¹H NMR (400 MHz, DMSO-*d*₆, 343K) 7.95 (bs, 1H, NH, D₂O exchanged), 7.60 - 7.22 (m, 5H), 7.17 (bs, 1H, NH, D₂O exchanged), 5.09 - 4.85 (m, 1H), 3.67 - 3.63 (m, 1H), 3.62 (s, 2H), 3.56 (dd, *J* = 10.2, 5.2 Hz, 1H), 3.29 (s, 3H). MS (ESI⁺) : [M+H]⁺ 234.2.

### Example 2.16: Synthesis of tert-butyl N-[(2R)-2-[(5-oxo-1,4-dihydroimidazol-2-yl)amino]-2-phenyl-ethyl]carbamate (2.16)

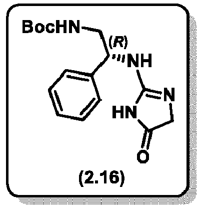

Compound (2.16) was synthesized according to **GP1-A** : reaction carried out in THF, with 2-methylsulfanyl-1,4-dihydroimidazol-5-one (1.1) (10.18 mmol), 1.5 eq of *tert-*butyl *N*-[(2*R*)-2-amino-2-phenyl-ethyl]carbamate and 2.3 eq of AcOH, at 115 °C (sealed tube, heating bock), for 6h. The product directly precipitated in the reaction medium: it was isolated after filtration, washing with cold THF, then pentane. Colorless solid, 40% (1.517 g). ¹H NMR (400 MHz, DMSO-*d*₆, 343K) 7.83 (br s, 1H, NH, D₂O exchanged), 7.56 - 7.05 (m, 5H + NH, D₂O exchanged), 6.63 (br s, 1H, NH, D₂O exchanged), 4.91 (br s, 1H), 3.59 (s, 2H), 3.29 (t, *J* = 6.5 Hz, 2H), 1.36 (s, 9H). MS (ESI⁺) : [M+H]⁺ 319.2.

### Example 2.17: Synthesis of tert-butyl N-[(2S)-2-[(5-oxo-1,4-dihydroimidazol-2-yl)amino]-2-phenyl-ethyl]carbamate (2.17)

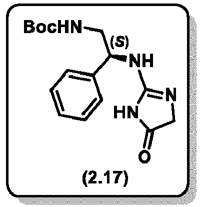

Compound (2.17) was synthesized according to **GP1-A** : reaction carried out in THF, with 2-methylsulfanyl-1,4-dihydroimidazol-5-one (1.1) (10.92 mmol), 1.5 eq of *tert-*butyl *N*-[(2*S*)-2-amino-2-phenyl-ethyl]carbamate and 2.3 eq of AcOH, at 115 °C (heating bock), for 6h. The product directly precipitated in the reaction medium: it was isolated after filtration, washing with cold THF, then pentane. Colorless solid, 38% (1.306 g). ¹H NMR (400 MHz, DMSO-*d*₆, 343K) 7.83 (br s, 1H, NH, D₂O exchanged), 7.56 - 7.05 (m, 5H + NH, D₂O exchanged), 6.63 (br s, 1H, NH, D₂O exchanged), 4.91 (br s, 1H), 3.59 (s, 2H), 3.29 (t, *J* = 6.5 Hz, 2H), 1.36 (s, 9H). MS (ESI⁺) : [M+H]⁺ 319.3.

### Example 2.18: Synthesis of 2-[(4-methylthiazol-2-yl)methylamino]-1,4-dihydroimidazol-5-one (2.18)

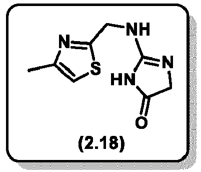

Compound (2.18) was synthesized according to **GP1-A** : reaction carried out in THF, with 2-methylsulfanyl-1,4-dihydroimidazol-5-one (1.1) (3.073 mmol), 2 eq of (4-methylthiazol-2-yl)methanamine and 3 eq of AcOH, at 110 °C (sealed tube, heating block), for 3h. The product directly precipitated in the reaction medium: it was isolated after filtration, washing with cold THF, then pentane. Beige solid, 35% (248 mg). ¹H NMR (400 MHz, DMSO-*d*₆, 300K) δ_{H} 8.21 (br s, 1H, NH, D₂O exchanged), 7.59 (br s, 1H, NH, D₂O exchanged), 7.17 (s, 1H), 4.65 (s, 2H), 3.69 (s, 2H), 2.33 (s, 3H). MS (ESI⁺) : [M+H]⁺ 211.1.

### Example 2.19: Synthesis of 2-(tetrahydropyran-4-ylmethylamino)-1,4-dihydroimidazol-5-one (2.19)

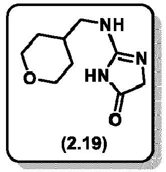

Compound (2.19) was synthesized according to **GP1-A** : reaction carried out in THF, with 2-methylsulfanyl-1,4-dihydroimidazol-5-one (1.1) (3.073 mmol) and 3 eq of tetrahydropyran-4-ylmethanamine, at 110 °C (sealed tube, heating block), for 16h. The product directly precipitated in the reaction medium: it was isolated after filtration, washing with cold THF, then pentane. Beige solid, 70% (424 mg). ¹H NMR (400 MHz, DMSO-*d*₆, 343K) δ_{H} 7.47 (br s, 1H, NH, D₂O exchanged), 7.22 (br s, 1H, NH, D₂O exchanged), 3.90 - 3.80 (m, 2H), 3.61 (s, 2H), 3.28 (td, *J* = 11.6, 2.0 Hz, 2H), 3.11 - 3.05 (m, 2H), 1.80 - 1.73 (m, 1H), 1.64 - 1.52 (m, 2H), 1.19 (qd, *J* = 11.8, 4.4 Hz, 2H). MS (ESI⁺) : [M+H]⁺ 198.1.

### Example 2.20: Synthesis of 2-[4-(4-methylpiperazin-1-yl)anilino]-1,4-dihydroimidazol-5-one (2.20)

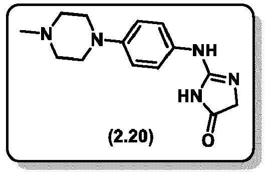

Compound (2.20) was synthesized according to **GP1-C** : reaction carried out in dioxane, with 2-methylsulfanyl-1,4-dihydroimidazol-5-one (1.1) (4.61 mmol), 2 eq of 4-(4-methylpiperazin-1-yl)aniline and 3 eq of AcOH, at 130 °C (heating block), for 3h. Purification by FC (elution: DCM/MeOH (7N NH₃): 99/1 to 85/15). The final product required two successive triturations in EtOH at 0 °C. Beige solid, 59% (749 mg). ¹H NMR (400 MHz, DMSO-*d*₆, 300K) δ_{H} 9.66 (br s, 1H, NH, D₂O exchanged), 7.43 (br s, 1H, NH, D₂O exchanged), 7.33 - 7.19 (m, 2H), 6.91 (d, *J* = 8.9 Hz, 2H), 3.67 (s, 2H), 3.13 - 3.03 (m, 4H), 2.47 - 2.41 (m, 4H), 2.21 (s, 3H). MS (ESI⁺) : [M+H]⁺ 274.2.

### Example 2.21 : Synthesis of 2-(2-pyridylamino)-1,4-dihydroimidazol-5-one (2.21)

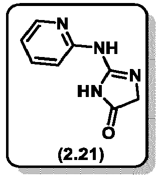

Compound (2.21) was synthesized according to **GP1-C** : reaction carried out in dioxane, with 2-methylsulfanyl-1,4-dihydroimidazol-5-one (1.1) (6.15 mmol), 2.5 eq of 2-aminopyridine and 3 eq of AcOH, at 130 °C (heating block), for 12h. Purification by FC (elution: DCM/MeOH (7N NH₃): 99/1 to 85/15). The final product required a trituration in DCM at room temperature. Light beige solid, 35% (384 mg). ¹H NMR (400 MHz, DMSO-*d*₆, 300K) δ_{H} 10.99 (bs, 1H, NH, D₂O exchanged), 9.20 (bs, 1H, NH, D₂O exchanged), 8.30 - 8.22 (m, 1H), 7.79 - 7.73 (m, 1H), 7.15 (d, *J* = 8.3 Hz, 1H), 7.05 (dd, *J* = 7.3, 5.0 Hz, 1H), 3.91 (s, 2H). MS (ESI⁺) : [M+H]⁺ 177.2.

### Example 2.22 : Synthesis of (±)-2-(oxepan-3-ylamino)-1,4-dihydroimidazol-5-one (2.22)

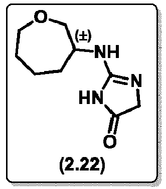

Compound 2.22 was synthesized according to **GP1-A**: reaction carried out in THF, with 2-methylsulfanyl-1,4-dihydroimidazol-5-one (1.1) (851 µmol), 2.5 eq of oxepan-3-amine, at 110 °C (heating block), for 12h The product directly precipitated in the reaction medium: it was isolated after filtration, washing with cold THF, then pentane. Light beige solid, 73% (122 mg). ¹H NMR (400 MHz, DMSO-*d*₆, 343K) δ_{H} 7.33 (br s, 1H, NH, D₂O exchanged), 7.05 (br s, 1H, NH, D₂O exchanged), 3.96 - 3.79 (m, 1H), 3.76 - 3.62 (m, 3H), 3.61 (s, 2H), 3.56 - 3.46 (m, 1H), 1.91 - 1.77 (m, 1H), 1.77 - 1.60 (m, 4H), 1.59 - 1.46 (m, 1H). MS (ESI⁺) : [M+H]⁺ 198.1.

### Example 3: General protocol 2 - Palladium-catalyzed vinylation of heteroarylbromides

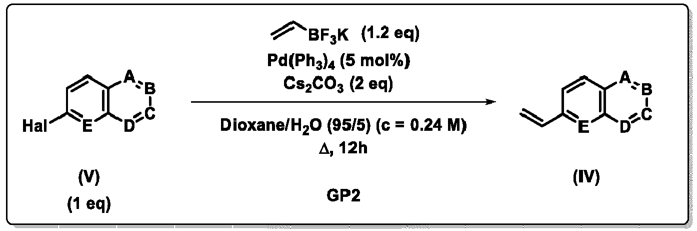

In the above scheme, A, B, C, D and E are as defined above and Hal represents a halogen atom, in particular seleted from an iodine and a bromine atom.

**GP2:** a mixture of the appropriate heteroarylhalide (1 eq), potassium vinyltrifluoroborate (1.2 eq), Cs₂CO₃ (2 eq), and Pd(PPh3)4 (5 mol%) in dioxane/H₂O (95/5) (C = 0.4 M) was thoroughly purged with vacuum/argon cycles. The mixture was brought to reflux and heated for 12h. Upon completion (¹H NMR), the mixture was partitioned between H₂O and AcOEt. The aqueous layer was extracted three times with AcOEt. The combined organic layers were dried over MgSO₄, filtered, concentrated *in vacuo,* adsorbed on silica, and purified by FC on silica gel using the appropriate gradient of solvents (see details below) to yield the desired vinylheteroaryl in analytically pure form.

### Example 3.1: Synthesis of 6-vinylquinazoline (3.1)

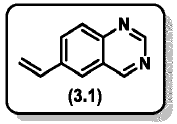

Compound (3.1) was synthesized according to **GP5** : reaction carried out with 6-bromoquinazoline (8.61 mmol). Purification by FC : elution : cyclohexane/AcOEt : 99/1 to 0/1. Orange oil, 95% (1.28 g). ¹H NMR (400 MHz, CDCl₃, 300K) δ_{H} 9.36 (s, 1H), 9.28 (s, 1H), 8.09 - 7.95 (m, 2H), 7.85 - 7.75 (m, 1H), 6.89 (dd, *J* = 17.7, 11.0 Hz, 1H), 5.94 (d, *J* = 17.6 Hz, 1H), 5.47 (d, *J* = 10.9 Hz, 1H). ¹³C NMR (101 MHz, CDCl₃, 300K) δ_{C} 122.6, 117.7, 112.4, 99.7, 97.9, 94.2, 91.1, 87.8, 87.1, 79.3. MS (ESI⁺) : [M+H]⁺ 157.1.

### Example 3.2: Synthesis of 6-vinylphthalazine (3.2)

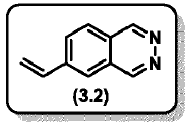

Compound (3.2) was synthesized according to **GP5:** reaction carried out with 6-bromophthalazine (16.74 mmol). Purification by FC : elution : cyclohexane/AcOEt: 9/1 to 0/1. Orange oil, 93% (2.42 g). ¹H NMR (400 MHz, CDCl₃, 300K) δ_{H} 9.50 (s, 1H), 9.48 (s, 1H), 8.02 (dd, *J* = 8.4, 1.6 Hz, 1H), 7.91 (d, *J* = 8.5 Hz, 1H), 7.85 (s, 1H), 6.92 (dd, *J* = 17.6, 10.9 Hz, 1H), 6.02 (d, *J* = 17.5 Hz, 1H), 5.55 (d, *J* = 10.9 Hz, 1H). ¹³C NMR (101 MHz, CDCl₃, 300K) δ_{C} 151.3, 150.8, 142.0, 135.6, 130.3, 127.1, 126.6, 126.1, 123.8, 118.4. MS (ESI⁺) : [M+H]⁺ 157.2.

### Example 4: General protocol 3 - Synthesis of heteroarylcarbaldehydes from vinylheteroaryls - Osmium-catalyzed Lemieux-Johnson oxidation

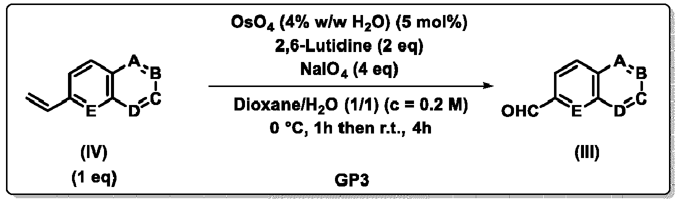

In the above scheme, A, B, C, D and E are as defined above.

**GP3:** NaIO₄ (4 eq) was added portionwise to a stirred solution of the appropriate vinylheteroaryl (1 eq), 2,6-lutidine (2 eq), OsO₄ (4% w/w H₂O sol., 5 mol%), in dioxane/H₂O (1/1) (C = 0.2 M) maintained at 0 °C. The reaction mixture was vigorously stirred at 0 °C for 1h, brought back to room temperature and stirred another 4h. Upon completion (TLC), the mixture was partitioned between AcOEt and H₂O. The aqueous layer was extracted with AcOEt (x3). The combined organic layers were washed with sat. NH₄Cl_{(aq)}, sat. NaHCO_{3(aq)}, dried over MgSO₄, filtered, and concentrated *in vacuo* (trace amounts of 2,6-lutidine were removed by co-evaporation with toluene). The solid residue was adsorbed on silica and purified by FC on silica gel using the appropriate gradient of solvents (see details below for each compound).

### Example 4.1: Synthesis of quinazoline-6-carbaldehyde (4.1)

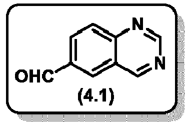

Compound (4.1) was synthesized according to **GP3** : reaction carried out with 6-vinylquinazoline (3.1) (8.13 mmol). Purification by FC : elution : cyclohexane/AcOEt : 9/1 to 0/1. Beige solid, 24% (314 mg). ¹H NMR (400 MHz, CDCl₃, 300K) δ_{H} 10.23 (s, 1H), 9.58 (s, 1H), 9.46 (s, 1H), 8.47 (d, *J* = 1.8 Hz, 1H), 8.42 (dd, *J* = 8.8, 1.8 Hz, 1H), 8.18 (d, *J* = 8.7 Hz, 1H). ¹³C NMR (101 MHz, CDCl₃, 300K) δ_{C} 190.5, 161.7, 157.7, 153.0, 135.5, 132.5, 131.5, 130.1, 124.8. MS (ESI⁺) : [M+H]⁺ 177.1.

### Example 4.2: Synthesis of phthalazine-6-carbaldehyde (4.2)

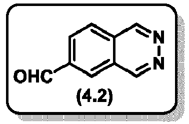

Compound (4.2) was synthesized according to **GP3** : reaction carried out with 6-vinylphthalazine (3.2) (15.43 mmol). Purification by FC : elution : cyclohexane/AcOEt : 9/1 to 0/1. Beige solid, 18% (448 mg). ¹H NMR (400 MHz, DMSO-*d*₆, 300K) δ_{H} 10.27 (s, 1H), 9.88 (s, 1H), 9.83 (s, 1H), 8.82 - 8.74 (m, 1H), 8.41 (dd, *J* = 8.3, 1.6 Hz, 1H), 8.33 (d, *J* = 8.3 Hz, 1H). ¹³C NMR (101 MHz, DMSO-*d*₆, 300K) 192.5, 151.6, 151.3, 138.7, 130.8, 130.2, 128.0, 127.6, 125.6. MS (ESI⁺) : [M+H]⁺ 177.2.

### Example 5: General protocol 4 - Knoevenagel condensation between thiohydantoin and heteroarylcarbalhydes (ROUTE 2)

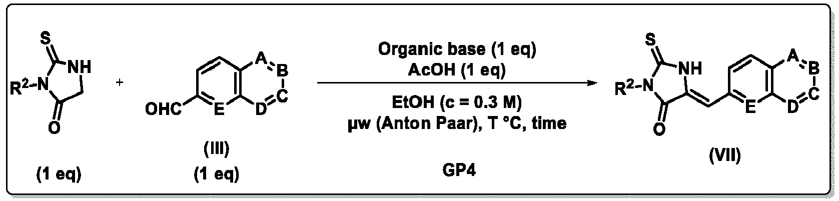

In the above scheme, A, B, C, D and E are as defined above and R² represents a hydrogen atom, a (C₁-C₄)alkyl or a (C₃-C₆)cycloalkyl group, in particular R² represents a hydrogen atom or a methyl group.

**GP4:** a stirred solution of 2-thiohydantoin (1 eq), the appropriate heteroarylcarbaldehyde (1 eq), organic base (1 eq) and AcOH (1 eq) in EtOH (C = 0.3 M) was heated in a sealed tube in a microwave oven (Anton Paar) for the indicated time at the adequate temperature (see details below for each compound). Upon completion (followed by consumption of the heteroarylcarbaldehyde on TLC), the reaction media was cooled down and added onto stirred water. The precipitated solid was stirred for 30 min and filtered off on a fritted glass funnel, thoroughly dried, and could be used in the next step without further purification. Higher purity may be achieved by trituration in EtOH.

### Example 5.1: Synthesis of (5Z)-5-(quinoxalin-6-ylmethylene)-2-thioxo-imidazolidin-4-one (5.1)

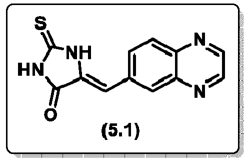

Compound 5.1 was synthesized according to **GP4** : reaction carried out with 2-thiohydantoin (1.90 mmol), quinoxaline-6-carbaldehyde, AcOH, and piperidine as the organic base. Reaction temperature: 110 °C, time: 3h. The final product required a trituration in EtOH. Yellow solid, 89% (438 mg). ¹H NMR (400 MHz, DMSO-*d*₆, 300K) δ_{H} 12.53 (br s, 1H, D₂O exchanged), 12.51 (br s, 1H, D₂O exchanged), 8.99 (d, *J* = 1.9 Hz, 1H), 8.95 (d, *J* = 1.8 Hz, 1H), 8.49 (d, *J* = 1.9 Hz, 1H), 8.17 (dd, *J* = 8.8, 2.0 Hz, 1H), 8.09 (d, *J* = 8.7 Hz, 1H), 6.73 (s, 1H). ¹³C NMR (101 MHz, DMSO-*d*₆, 300K) δ_{C} 179.8, 165.7, 146.4, 146.1, 142.3, 142.1, 134.3, 131.5, 130.3, 129.6, 129.3, 109.6. MS (ESI⁺) : [M+H]⁺ 257.1.

### Example 5.2: Synthesis of (5Z)-5-(6-quinolylmethylene)-2-thioxo-imidazolidin-4-one (5.2)

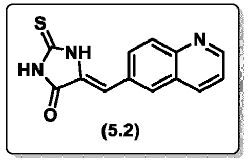

Compound 5.2 was synthesized according to **GP4** : reaction carried out with 2-thiohydantoin (6.62 mmol), quinoline-6-carbaldehyde, AcOH, and piperidine as the organic base. Reaction temperature: 110 °C, time: 3h. The final product required a trituration in EtOH. Yellow solid, 86% (1.45 g). ¹H NMR (400 MHz, DMSO-*d*₆, 300K) δ_{H} 12.41 (br s, 2H, D₂O exchanged), 8.96 - 8.89 (m, 1H), 8.42 - 8.40 (m, 1H), 8.37 (d, *J* = 8.0 Hz, 1H), 8.06 - 7.97 (m, 2H), 7.57 (dd, *J* = 8.3, 4.2 Hz, 1H), 6.65 (s, 1H). ¹³C NMR (101 MHz, DMSO-*d*₆, 300K) δ_{C} 179.5, 165.7, 151.4, 147.4, 136.4, 131.2, 130.5, 129.5, 129.2, 128.6, 127.9, 122.1, 110.5. MS (ESI⁺) : [M+H]⁺ 256.1.

### Example 5.3: Synthesis of (5Z)-5-(6-isoquinolylmethylene)-2-thioxo-imidazolidin-4-one (5.3)

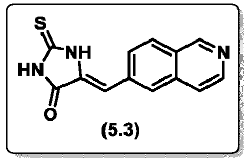

Compound 5.3 was synthesized according to **GP4** : reaction carried out with 2-thiohydantoin (31.81 mmol), isoquinoline-6-carbaldehyde, AcOH, and piperidine as the organic base. Reaction temperature: 110 °C, time: 3h. The final product required a trituration in EtOH. Yellow solid, 85% (6.902 g). ¹H NMR (400 MHz, DMSO-*d*₆, 300K) δ_{H} 12.45 (br s, 2H, D₂O exchanged), 9.31 (s, 1H), 8.54 (d, *J* = 5.7 Hz, 1H), 8.37 (d, *J* = 1.7 Hz, 1H), 8.12 (d, *J* = 8.6 Hz, 1H), 7.94 (dd, *J* = 8.6, 1.7 Hz, 1H), 7.83 (d, *J* = 5.8 Hz, 1H), 6.63 (s, 1H). ¹³C NMR (101 MHz, DMSO-*d*₆, 300K) δ_{C} 179.8, 165.8, 152.0, 143.5, 135.2, 134.3, 129.7, 128.9, 127.8, 127.6, 127.4, 120.5, 109.9. MS (ESI⁺) : [M+H]⁺ 256.0.

### Example 5.4: Synthesis of (5Z)-5-(quinazolin-6-ylmethylene)-2-thioxo-imidazolidin-4-one (5.4)

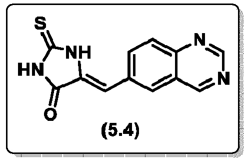

Compound 5.4 was synthesized according to **GP4** : reaction carried out with 2-thiohydantoin (1.94 mmol), quinazoline-6-carbaldehyde (4.1), AcOH, and piperidine as the organic base. Reaction temperature: 110 °C, time: 3h. The final product required a trituration in EtOH. Brown solid, 95% (472 mg). ¹H NMR (400 MHz, DMSO-*d*₆, 300K) δ_{H} 12.43 (s, 2H), 9.58 (s, 1H), 9.31 (s, 1H), 8.54 (d, *J* = 2.0 Hz, 1H), 8.26 (dd, *J* = 8.8, 2.1 Hz, 1H), 8.00 (d, *J* = 8.8 Hz, 1H), 6.66 (s, 1H). ¹³C NMR (101 MHz, DMSO-*d*₆, 300K) δ_{C} 179.7, 165.7, 160.9, 155.5, 148.9, 136.1, 132.1, 129.6, 128.7, 127.9, 124.7, 109.3. MS (ESI⁺) : [M+H]⁺ 257.0.

### Example 5.5: Synthesis of (5Z)-5-(1,5-naphthyridin-2-ylmethylene)-2-thioxo-imidazolidin-4-one (5.5)

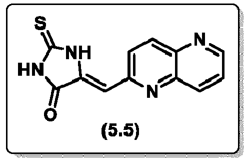

Compound 5.5 was synthesized according to **GP4** : reaction carried out with 2-thiohydantoin (6.37 mmol), 1,5-naphthyridine-2-carbaldehyde, AcOH, and piperidine as the organic base. Reaction temperature: 110 °C, time: 3h. The final product required a trituration in EtOH. Yellow solid, 97% (1.58 g). ¹H NMR (400 MHz, CF3COOD, 300K) δ_{H} 9.72 (d, *J* = 8.5 Hz, 1H), 9.17 (dd, *J* = 5.5, 1.4 Hz, 1H), 8.71 (dd, *J* = 9.0, 0.9 Hz, 1H), 8.28 (dd, *J* = 8.8, 5.5 Hz, 1H), 8.16 (d, *J* = 9.0 Hz, 1H), 6.98 (s, 1H). ¹³C NMR (101 MHz, CF₃COOD, 300K) δ_{C} 181.1, 168.7, 160.7, 151.4, 146.7, 145.8, 136.4, 136.3, 134.4, 132.0, 127.8, 109.9. MS (ESI⁺) : [M+H]⁺ 257.1.

### Example 5.6: Synthesis of (5Z)-5-(cinnolin-6-ylmethylene)-2-thioxo-imidazolidin-4-one (5.6)

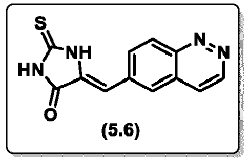

Compound 5.6 was synthesized according to **GP4** : reaction carried out with 2-thiohydantoin (3.79 mmol), cinnoline-6-carbaldehyde, AcOH, and piperidine as the organic base. Reaction temperature: 110 °C, time: 3h. The final product required a trituration in EtOH. Yellow solid, 84% (813 mg). ¹H NMR (400 MHz, DMSO-*d*₆, 300K) δ_{H} 12.47 (br s, 2H, D₂O exchanged), 9.39 (d, *J* = 5.8 Hz, 1H), 8.46 - 8.38 (m, 2H), 8.21 - 8.11 (m, 2H), 6.65 (s, 1H). ¹³C NMR (101 MHz, DMSO-*d*₆, 300K) δ_{C} 180.0, 165.7, 149.2, 145.8, 135.1, 132.6, 130.6, 129.0, 128.0, 125.7, 122.8, 108.7. MS (ESI⁺) : [M+H]⁺ 257.0.

### Example 5.7: Synthesis of (5Z)-5-(phthalazin-6-ylmethylene)-2-thioxo-imidazolidin-4-one (5.7)

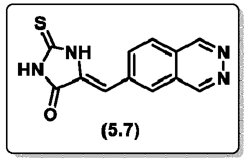

Compound 5.6 was synthesized according to **GP4** : reaction carried out with 2-thiohydantoin (2.76 mmol), phthalazine-6-carbaldehyde (4.2), AcOH, and piperidine as the organic base. Reaction temperature: 110 °C, time: 3h. The final product required a trituration in EtOH. Brown solid, 97% (320 mg). ¹H NMR (400 MHz, DMSO-*d*₆, 300K) δ_{H} ¹H NMR (400 MHz, DMSO-*d*₆) δ 12.49 (s, 1H), 9.69 - 9.64 (m, 1H), 9.64 - 9.60 (m, 1H), 8.52 - 8.47 (m, 1H), 8.23 (dd, *J* = 8.5, 1.7 Hz, 1H), 8.15 (d, *J* = 8.4 Hz, 1H), 6.66 (s, 1H). ¹³C NMR (101 MHz, DMSO-*d*₆, 300K) δ_{C} 180.0, 165.7, 151.0, 150.7, 136.7, 134.2, 130.3, 127.0, 126.6, 126.0, 125.1, 108.8. MS (ESI⁺) : [M+H]⁺ 257.1.

### Example 6: General protocol 5 - S-Alkylation of (5Z)-5-heteroarylmethylene-2-thioxo-imidazolidin-4-ones (ROUTE 2)

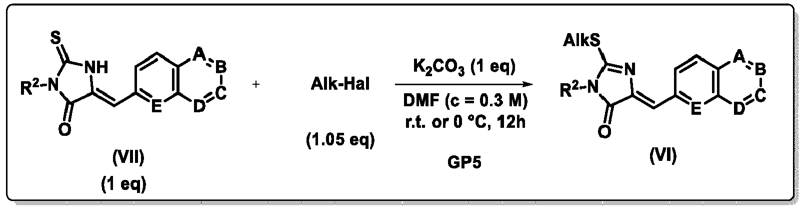

In the above scheme, A, B, C, D and E are as defined above, R² represents a hydrogen atom, a (C₁-C₄)alkyl or a (C₃-C₆)cycloalkyl group, in particular R² represents a hydrogen atom or a methyl group, Alk is a (C₁-C₅)alkyl, in particular selected from a methyl and an ethyl group and Hal is an halide, in particular a iodine or a bromine.

**GP5** : The appropriate alkyliodide (1.05 eq) was added dropwise to a stirred solution of 5-heteroarylmethylene-2-thioxo-imidazolidin-4-one (1 eq) and K₂CO₃ (1 eq) in DMF (C = 0.3 M) at the appropriate temperature (see details below). The resulting mixture was stirred at the appropriate temperature, for the indicated time. Upon completion (TLC), the mixture was poured into water. The precipitated solid was stirred for 30 min and filtered off on a fritted glass funnel, thoroughly dried, and could be used in the next step without further purification. Trace impurities resulting from *N*3-alkylation may be removed by trituration or purification on FC (see details below).

### Example 6.1: Synthesis of (4Z)-2-ethylsulfanyl-4-(quinoxalin-6-ylmethylene)-1H-imidazol-5-one (6.1)

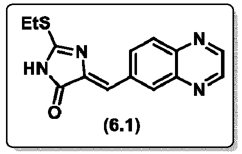

Compound (6.1) was synthesized according to GP5 : reaction carried out with (5Z)-5-(quinoxalin-6-ylmethylene)-2-thioxo-imidazolidin-4-one (5.1) (24.97 mmol) and EtI, at room temperature for 12 h. Yellow solid, 77% (5.467 g). ¹H NMR (400 MHz, DMSO-*d*₆) δ_{H} ¹H NMR (400 MHz, DMSO-*d*₆, 300K) δ 11.93 (br s, 1H, NH, D₂O exchanged), 8.93 (d, *J* = 1.8 Hz, 1H), 8.90 (d, *J* = 1.8 Hz, 1H), 8.84 - 8.78 (m, 1H), 8.65 (dd, *J* = 8.9, 1.9 Hz, 1H), 8.05 (d, *J* = 8.8 Hz, 1H), 6.94 (s, 1H), 3.33 (q, *J* = 7.3 Hz, 2H), 1.45 (t, *J* = 7.4 Hz, 3H). ¹³C NMR (101 MHz, DMSO-*d*₆, 300K) δ_{C} 170.4, 166.4, 146.3, 146.0, 142.4, 142.2, 141.0, 136.2, 132.2, 131.9, 129.1, 118.6, 24.4, 14.5. MS (ESI⁺) : [M+H]⁺ 285.1.

### Example 6.2: Synthesis of (4Z)-2-methylsulfanyl-4-(quinoxalin-6-ylmethylene)-1H-imidazol-5-one (6.2)

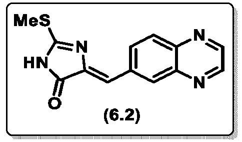

Compound (6.2) was synthesized according to GP5 : reaction carried out with (5Z)-5-(quinoxalin-6-ylmethylene)-2-thioxo-imidazolidin-4-one (5.1) (15.83 mmol) and MeI, from 0 °C to room temperature, for 12h. The final product required a trituration in DCM. Yellow solid, 81% (4.06 g). ¹H NMR (400 MHz, DMSO-*d*₆, 300K) δ_{H} 11.99 (br s, 1H, NH, D₂O exchanged), 9.01 - 8.92 (m, 2H), 8.86 (s, 1H), 8.74 (dd, *J* = 8.8, 1.9 Hz, 1H), 8.11 (d, *J* = 8.8 Hz, 1H), 7.00 (s, 1H), 2.74 (s, 3H). ¹³C NMR (101 MHz, DMSO-*d*₆, 300K) δ_{C} 170.6, 167.1, 146.3, 146.1, 142.4, 142.2, 141.0, 136.3, 132.3, 132.0, 129.2, 118.7, 12.4. MS (ESI⁺) : [M+H]⁺ 271.1.

### Example 6.3: Synthesis of (4Z)-2-ethylsulfanyl-4-(6-quinolylmethylene)-1H-imidazol-5-one (6.3)

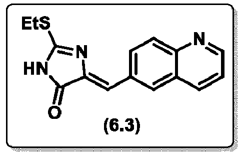

Compound (6.3) was synthesized according to GP5 : reaction carried out with (5Z)-5-(6-quinolylmethylene)-2-thioxo-imidazolidin-4-one (5.2) (45.83 mmol) and EtI, at room temperature for 12 h. The final product required a trituration in acetone. Yellow solid, 77% (9.99 g). ¹H NMR (400 MHz, DMSO-*d*₆, 300K) δ_{H} 11.86 (br s, 1H, NH, D₂O exchanged), 8.90 (dd, *J* = 4.2, 1.7 Hz, 1H), 8.67 (dd, *J* = 8.9, 1.9 Hz, 1H), 8.64 - 8.57 (m, 1H), 8.33 (d, *J* = 8.1 Hz, 1H), 8.02 (d, *J* = 8.8 Hz, 1H), 7.53 (dd, *J* = 8.3, 4.2 Hz, 1H), 6.90 (s, 1H), 3.34 (q, *J* = 7.3 Hz, 2H), 1.46 (t, *J* = 7.3 Hz, 3H). ¹³C NMR (101 MHz, DMSO-*d*₆, 300K) δ_{C} 170.5, 165.3, 151.3, 147.7, 140.1, 136.5, 132.7, 131.7, 131.3, 129.1, 127.8, 122.0, 119.5, 24.4, 14.5. MS (ESI⁺) : [M+H]⁺ 284.1.

### Example 6.4: Synthesis of (4Z)-2-methylsulfanyl-4-(6-quinolylmethylene)-1H-imidazol-5-one (6.4)

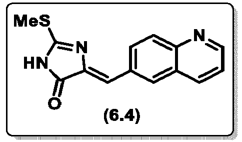

Compound (6.4) was synthesized according to GP5 : reaction carried out with (5Z)-5-(6-quinolylmethylene)-2-thioxo-imidazolidin-4-one (5.2) (3.92 mmol) and MeI, from 0 °C to room temperature, for 12h. Yellow solid, 82% (871 mg). ¹H NMR (400 MHz, DMSO-*d*₆, 300K) δ_{H} 11.92 (br s, 1H, NH, D₂O exchanged), 8.95 - 8.86 (m, 1H), 8.72 (d, *J* = 8.9 Hz, 1H), 8.64 (s, 1H), 8.37 (d, *J* = 8.1 Hz, 1H), 8.03 (d, *J* = 8.9 Hz, 1H), 7.55 (dd, *J* = 8.3, 4.2 Hz, 1H), 6.91 (s, 1H), 2.74 (s, 3H). ¹³C NMR (101 MHz, DMSO-*d*₆, 300K) δ_{C} 170.7, 166.0, 151.3, 147.7, 140.1, 136.5, 132.7, 131.8, 131.4, 129.1, 127.8, 122.0, 119.6, 12.4. MS (ESI⁺) : [M+H]⁺ 270.1.

### Example 6.5: Synthesis of (4Z)-2-methylsulfanyl-4-(6-isoquinolylmethylene)-1H-imidazol-5-one (6.5)

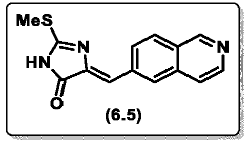

Compound (6.5) was synthesized according to GP5 : reaction carried out with (5Z)-5-(6-isoquinolylmethylene)-2-thioxo-imidazolidin-4-one (5.3) (785 µmol) and MeI, at room temperature, from 0 °C to room temperature, for 12h. Yellow solid, 84% (178 mg). ¹H NMR (400 MHz, DMSO-*d*₆) δ_{H} 11.98 (br s, 1H, NH, D₂O exchanged), 9.30 (s, 1H), 8.69 - 8.57 (m, 2H), 8.52 (d, *J* = 5.7 Hz, 1H), 8.13 (d, *J* = 8.6 Hz, 1H), 7.82 (d, *J* = 5.7 Hz, 1H), 6.90 (s, 1H), 2.74 (s, 3H). ¹³C NMR (101 MHz, DMSO-*d*₆, 300K) δ_{C} 170.5, 166.9, 151.9, 143.3, 140.8, 136.3, 135.0, 129.6, 129.2, 127.5, 127.5, 120.5, 118.9, 12.2. MS (ESI⁺): [M+H]⁺ 270.1.

### Example 6.6: Synthesis of (4Z)-2-ethylsulfanyl-4-(quinazolin-6-ylmethylene)-1H-imidazol-5-one (6.6)

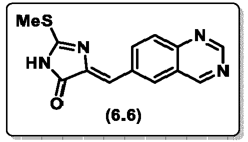

Compound (6.6) was synthesized according to GP5 : reaction carried out with (5Z)-5-(quinazolin-6-ylmethylene)-2-thioxo-imidazolidin-4-one (5.4) (1.74 mmol) and MeI, from 0 °C to room temperature, for 12h. Purification by FC : elution : DCM/MeOH : 99/1 to 9/1. Yellow solid, 59% (277 mg). ¹H NMR (400 MHz, DMSO-*d*₆, 300K) δ_{H} 11.97 (br s, 1H, NH, D₂O exchanged), 9.61 (s, 1H), 9.30 (s, 1H), 8.91 (dd, *J* = 8.9, 1.9 Hz, 1H), 8.85 - 8.80 (m, 1H), 8.03 (d, *J* = 8.9 Hz, 1H), 6.93 (s, 1H), 2.75 (s, 3H). ¹³C NMR (101 MHz, DMSO-*d*₆, 300K) δ_{C} 170.5, 167.0, 161.1, 155.6, 149.2, 140.8, 136.4, 134.2, 130.8, 127.9, 124.7, 118.4, 12.4. MS (ESI⁺) : [M+H]⁺ 271.1.

### Example 6.7: Synthesis of (4Z)-2-methylsulfanyl-4-(1,5-naphthyridin-2-ylmethylene)-1H-imidazol-5-one (6.7)

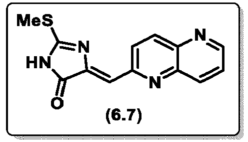

Compound (6.7) was synthesized according to GP5 : reaction carried out with (5Z)-5-(1,5-naphthyridin-2-ylmethylene)-2-thioxo-imidazolidin-4-one (5.5) (6.01 mmol) and MeI, from 0 °C to room temperature, for 12h. The final product required a purification by FC (elution : DCM/MeOH : 99/1 to 9/1), followed by a trituration in refluxing DCM. Yellow solid, 26% (416 mg). ¹H NMR (400 MHz, CF3COOD, 300K) δ_{H} 9.40 (d, *J* = 8.7 Hz, 1H), 9.26 (dd, *J =* 5.6, 1.5 Hz, 1H), 8.85 (d, *J* = 9.0 Hz, 1H), 8.36 (dd, *J* = 8.8, 5.5 Hz, 1H), 8.30 (d, *J* = 9.0 Hz, 1H), 7.54 (s, 1H), 3.03 (s, 3H). ¹³C NMR (101 MHz, CF₃COOD, 300K) δ_{C} 178.5, 164.0, 158.7, 151.2, 148.7, 145.2, 136.7, 134.9, 133.6, 132.6, 128.8, 120.3, 15.7. MS (ESI⁺): [M+H]⁺ 271.1.

### Example 6.8: Synthesis of (4Z)-4-(cinnolin-6-ylmethylene)-2-methylsulfanyl-1H-imidazol-5-one (6.8)

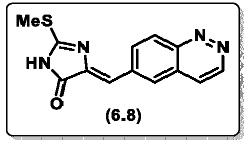

Compound (6.8) was synthesized according to GP5 : reaction carried out with (5Z)-5-(cinnolin-6-ylmethylene)-2-thioxo-imidazolidin-4-one (5.6) (2.99 mmol) and MeI, from 0 °C to room temperature, for 12h. Yellow solid, 65% (626 mg). ¹H NMR (400 MHz, DMSO-*d*₆) δ_{H} ¹H NMR (400 MHz, DMSO-*d*₆, 300K) δ 12.04 (s, 1H), 9.38 (d, *J* = 5.9 Hz, 1H), 8.93 - 8.83 (m, 1H), 8.70 (s, 1H), 8.46 (d, *J* = 9.0 Hz, 1H), 8.21 (d, *J* = 5.8 Hz, 1H), 6.93 (s, 1H), 2.76 (s, 3H). MS (ESI⁺) : [M+H]⁺ 271.1.

### Example 6.9: Synthesis of (4Z)-2-methylsulfanyl-4-(phthalazin-6-ylmethylene)-1H-imidazol-5-one (6.9)

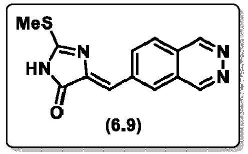

Compound (6.9) was synthesized according to GP5 : reaction carried out with (5Z)-5-(phthalazin-6-ylmethylene)-2-thioxo-imidazolidin-4-one (5.7) (2.56 mmol) and MeI, from 0 °C to room temperature, for 12h. The final product required a trituration in refluxing THF. Brown solid, 77% (532 mg). ¹H NMR (400 MHz, DMSO-*d*₆, 300K) δ_{H} 12.04 (br s, 1H, NH, D₂O exchanged), 9.74 - 9.60 (m, 2H), 8.95 - 8.74 (m, 2H), 8.17 (d, *J* = 8.6 Hz, 1H), 6.93 (s, 1H), 2.76 (s, 3H). ¹³C NMR (101 MHz, DMSO-*d*₆, 300K) δ_{C} 171.2, 151.6, 151.2, 142.1, 139.2, 135.2, 129.4, 127.1, 127.0, 126.5, 125.7, 118.3, 13.0. MS (ESI⁺): [M+H]⁺ 271.2.

### Example 7: General protocols 6 (GP6) and 7 (GP7) for the synthesis of compounds of formula (I)

### General protocol 6 - Knoevenagel condensation between N2-functionalized 2-amino-1,4-dihydroimidazol-5-ones and heteroarylcarbalhydes (ROUTE 1)

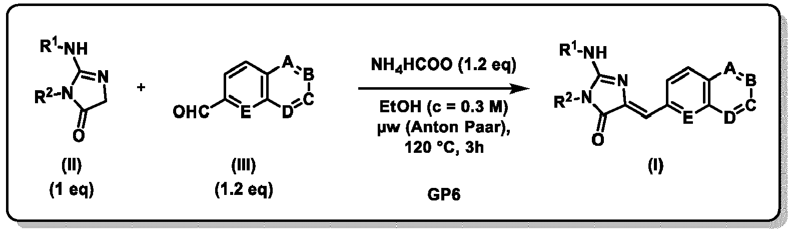

In the above scheme, A, B, C, D, E and R¹ are as defined above and R² represents a hydrogen atom, a (C₁-C₄)alkyl or a (C₃-C₆)cycloalkyl group, in particular R² represents a hydrogen atom or a methyl group.

**GP6:** a stirred solution of the appropriate *N*2-functionalized 2-amino-1,4-dihydroimidazol-5-one (1 eq), heteroarylcarboxaldehyde (1.2 eq) and NH4HCOO (1.2 eq) in EtOH (C = 0.3 M) was heated in a sealed tube in a microwave oven (Anton Paar) at 120 °C for 3h. Upon completion (followed by consumption of the heteroarylcarboxaldehyde on TLC), the mixture was brought back to room temperature, adsorbed on silica and purified by FC (see details below). After FC, higher purity may be achieved by reprecipitation, trituration, or recrystallization (see details below).

### General protocol 7 - Addition of amines on (4Z)-4-heteroarylmethylene-2-alkylsulfanyl-1H-imidazol-5-ones (ROUTE 2)

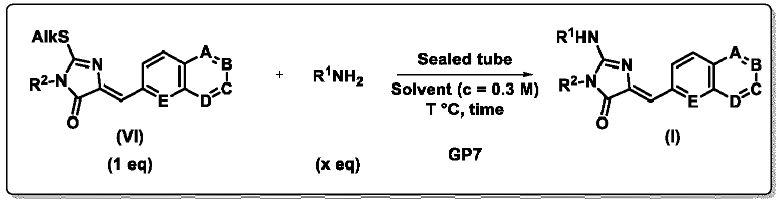

In the above scheme, A, B, C, D, E and R¹ are as defined above, R² represents a hydrogen atom, a (C₁-C₄)alkyl or a (C₃-C₆)cycloalkyl group, in particular R² represents a hydrogen atom or a methyl group and Alk is a (C₁-C₅)alkyl, in particular selected from a methyl and an ethyl group.

**GP7:** A stirred solution of the appropriate amine (x eq), (4Z)-4-heteroarylmethylene-2-alkylsulfanyl-1*H*-imidazol-5-one^{(a)} (1 eq) in the appropriate solvent (C = 0.3 M) was heated in a sealed tube (heating block). Upon completion (followed by consumption of the isothiourea on TLC), the mixture was brought back to room temperature.
- **GP7-A** : **direct precipitation of the desired product:** The reaction medium was stirred 1h at 0 °C. The precipitated solid was filtered off on a fritted-glass funnel. High purity may be achieved after filtration by washing, reprecipitation, trituration, or recrystallization (see Table 3 for details).
- **GP7-B : the product failed to precipitate** : the reaction mixture was concentrated *in vacuo,* adsorbed on silica, and purified by FC. High purity may be achieved after filtration by reprecipitation, trituration, or recrystallization.
- **GP7-C** : **the product failed to precipitate** : the reaction mixture was concentrated *in vacuo.* The resulting crude was triturated in EtOH (at r.t. or reflux), filtered off on a fritted-glass funnel.
- (a) May require activation with AcOH, depending on the amine (see details below).

### Selected examples from quinoxaline sub-series:

### Example 7.1: Synthesis of (4Z)-2-(cycloheptylamino)-4-(quinoxalin-6-ylmethylene)-1H-imidazol-5-one (1)

Reaction was carried out according to GP7-B, in THF (0.3M), on a 277 µmol scale of (6.2), with 3 eq of cycloheptylamine, at 120 °C (sealed tube, heating block), for 4h. Purification by FC (elution: DCM/MeOH: 99/1 to 88/10). Isolated yield: 47%.

### Example 7.2: Synthesis of (4Z)-2-(cyclooctylamino)-4-(quinoxalin-6-ylmethylene)-1H-imidazol-5-one (2)

Reaction was carried out according to GP7-B, in THF (0.3M), on a 277 µmol scale of (6.2), with 3 eq of cyclooctylamine, at 120 °C (sealed tube, heating block), for 4h. Purification by FC (elution: DCM/MeOH: 99/1 to 90/10). Isolated yield: 52%.

### Example 7.3: Synthesis of (4Z)-2-(cyclohexylmethylamino)-4-(quinoxalin-6-ylmethylene)-1H-imidazol-5-one (3)

Reaction was carried out according to GP7-B, in THF (0.3M), on a 740 µmol scale of (6.2), with 4 eq of cyclohexylmethanamine, at 120 °C (sealed tube, heating block), for 4h. Purification by FC (elution: DCM/MeOH: 99/1 to 90/10). Isolated yield: 45%.

### Example 7.4: Synthesis of (4Z)-2-[[(1R,2R)-2-methoxycyclopentyl]amino]-4-(quinoxalin-6-ylmethylene)-1H-imidazol-5-one (4)

Reaction was carried out according to GP7-B, in dioxane (0.3M), on a 277 µmol scale of (6.2), with 3 eq of (1*R*,2*R*)-2-methoxycyclopentanamine, at 120 °C (sealed tube, heating block), for 7h. Purification by FC (elution: DCM/MeOH: 99/1 to 90/10). Isolated yield: 54%.

### Example 7.5: Synthesis of (±)-(4Z)-2-[[trans-4-hydroxycycloheptyl]aminol]-4-(quinoxalin-6-ylmethylene)-1H-imidazol-5-one (6)

Reaction was carried out according to GP7-B, in dioxane (0.3M), on a 277 µmol scale of (6.2), with 3 eq of (**±**)-*trans*-4-hydroxycycloheptanamine, at 120 °C (sealed tube, heating block), for 7h. Purification by FC (elution: DCM/MeOH: 99/1 to 90/10). Isolated yield: 57%.

### Example 7.6: Synthesis of (±)-(4Z)-2-[[trans-4-methoxycycloheptyl]amino]-4-(quinoxalin-6-ylmethylene)-1H-imidazol-5-one (7)

Reaction was carried out according to GP7-B, in dioxane (0.3M), on a 277 µmol scale of (6.2), with 3 eq of **(±)**-*trans*-4-methoxycycloheptanamine, at 120 °C (sealed tube, heating block), for 7h. Purification by FC (elution: DCM/MeOH: 99/1 to 90/10). Isolated yield: 55%.

### Example 7.7: Synthesis of (±)-(4Z)-2-[[cis-3-methoxycycloheptyl]amino]-4-(quinoxalin-6-ylmethylene)-1H-imidazol-5-one (8)

Reaction was carried out according to GP7-B, in dioxane (0.3M), on a 277 µmol scale of (6.2), with 3 eq of **(±)**-*cis*-3-methoxycycloheptanamine, at 120 °C (sealed tube, heating block), for 7h. Purification by FC (elution: DCM/MeOH: 99/1 to 90/10). Isolated yield: 63%.

### Example 7.8: Synthesis of (4Z)-2-[[(1R)-1-(hydroxymethyl)-3-methylbutyl]amino]-4-(quinoxalin-6-ylmethylene)-1H-imidazol-5-one (9)

Reaction was carried out according to GP7-B, in dioxane (0.3M), on a 277 µmol scale of (6.2), with 3 eq of (*R*)-leucinol, at 120 °C (sealed tube, heating block), for 6h. Purification by FC (elution: DCM/MeOH: 99/1 to 90/10). Isolated yield: 50%.

### Example 7.9: Synthesis of (4Z)-2-[[(1R)-1-(methoxymethyl)-3-methyl-butyl]amino]-4-(quinoxalin-6-ylmethylene)-1H-imidazol-5-one (10)

Reaction was carried out according to GP7-B, in dioxane (0.3M), on a 277 µmol scale of (6.2), with 3 eq of (2*R*)-1-methoxy-4-methyl-pentan-2-amine, at 120 °C (sealed tube, heating block), for 26h. Purification by FC (elution: DCM/MeOH: 99/1 to 90/10). Isolated yield: 67%.

### Example 7.10: Synthesis of (4Z)-2-[[(1R)-1-(ethoxymethyl)-3-methyl-butyl]amino]-4-(quinoxalin-6-ylmethylene)-1H-imidazol-5-one (11)

Reaction was carried out according to GP7-B, in dioxane (0.3M), on a 430 µmol scale of (6.2), with 3 eq of (2*R*)-1-ethoxy-4-methyl-pentan-2-amine, at 130 °C (sealed tube, heating block), for 24h. Purification by FC (elution: DCM/MeOH: 99/1 to 90/10). The final product required a reprecipitation from DCM/pentane at 0 °C. Isolated yield: 51%.

### Example 7.11: Synthesis of (4Z)-2-[[(1R)-1-(benzyloxymethyl)-3-methyl-butyl]amino]-4-(quinoxalin-6-ylmethylene)-1H-imidazol-5-one (12)

Reaction was carried out according to GP7-B, in dioxane (0.3M), on a 276 µmol scale of (6.2), with 3 eq of (2*R*)-1-benzyloxy-4-methyl-pentan-2-amine, at 130 °C (sealed tube, heating block), for 24h. Purification by FC (elution: DCM/MeOH: 99/1 to 90/10). The final product required a reprecipitation from DCM/pentane at 0 °C. Isolated yield: 50%.

### Example 7.12: Synthesis of (4Z)-2-[[(1R)-1-[(4-fluorophenyl)methoxymethyl]-3-methyl-butyl]amino]-4-(quinoxalin-6-ylmethylene)-1H-imidazol-5-one (13)

Reaction was carried out according to GP7-B, in dioxane (0.3M), on a 363 µmol scale of (6.2), with 3 eq of (2*R*)-1-[(4-fluorophenyl)methoxy]-4-methyl-pentan-2-amine, at 130 °C (sealed tube, heating block), for 24h. Purification by FC (elution: DCM/MeOH: 99/1 to 90/10). The final product required a reprecipitation from DCM/pentane at 0 °C. Isolated yield: 46%.

### Example 7.13: Synthesis of (4Z)-2-[[(1R)-1-(fluoromethyl)-3-methyl-butyl]amino]-4-(quinoxalin-6-ylmethylene)-1H-imidazol-5-one (14)

Reaction was carried out according to GP7-B, in dioxane (0.3M), on a 185 µmol scale of (6.2), with 3 eq of (2*R*)-1-fluoro-4-methyl-pentan-2-amine, at 120 °C (sealed tube, heating block), for 72h. Purification by PTLC (elution: DCM/MeOH: 98/2). Isolated yield: 44%.

### Example 7.14: Synthesis of (4Z)-2-(3-noradamantylamino)-4-(quinoxalin-6-ylmethylene)-1H-imidazol-5-one (16)

Reaction was carried out according to GP7-B, in dioxane (0.3M), on a 587 µmol scale of (6.2), with 4 eq of 3-noradamantanamine and 6 eq of AcOH, at 160 °C (sealed tube, heating block), for 24h. Purification by FC (elution: DCM/MeOH: 99/1 to 90/10). The final product required a trituration in refluxing EtOH. Isolated yield: 47%.

### Example 7.15: Synthesis of (4Z)-2-(1-adamantylamino)-4-(quinoxalin-6-ylmethylene)-1H-imidazol-5-one (17)

Reaction was carried out according to GP7-A, in dioxane (0.3M), on a 277 µmol scale of (6.2), with 3 eq of adamantan-1-amine and 9 eq of AcOH, at 150 °C (sealed tube, heating block), for 30h. The product directly precipitated in the reaction medium: it was isolated after filtration, washing with cold THF, then pentane. The final product required a trituration in EtOH. Isolated yield: 43%.

### Example 7.16: Synthesis of (4Z)-2-[(3-hydroxy-1-adamantyl)amino]-4-(quinoxalin-6-ylmethylene)-1H-imidazol-5-one (18)

Reaction was carried out according to GP7-B, in dioxane (0.3M), on a 277 µmol scale of (6.2), with 3 eq of 3-hydroxyadamantan-1-amine and 9 eq of AcOH, at 150 °C (sealed tube, heating block), for 30h. Purification by FC (elution: DCM/MeOH: 99/1 to 90/10). The final product required a trituration in EtOH. Isolated yield: 42%.

### Example 7.17: Synthesis of (4Z)-2-[(3-methoxy-1-adamantyl)amino]-4-(quinoxalin-6-ylmethylene)-1H-imidazol-5-one (19)

Reaction was carried out according to GP7-B, in dioxane (0.3M), on a 277 µmol scale of (6.2), with 3 eq of 3-methoxyadamantan-1-amine and 9 eq of AcOH, at 150 °C (sealed tube, heating block), for 30h. Purification by FC (elution: DCM/MeOH: 99/1 to 90/10). The final product required a trituration in EtOH. Isolated yield: 72%.

### Example 7.18: Synthesis of (4Z)-2-[(3-fluoro-1-adamantyl)amino]-4-(quinoxalin-6-ylmethylene)-1H-imidazol-5-one (20)

Reaction was carried out according to GP7-B, in dioxane (0.3M), on a 481 µmol scale of (6.2), with 3 eq of 3-fluoroadamantan-1-amine and 6 eq of AcOH, at 150 °C (sealed tube, heating block), for 48h. Purification by FC (elution: DCM/MeOH: 99/1 to 90/10). The final product required a trituration in refluxing EtOH. Isolated yield: 28%.

### Example 7.19: Synthesis of (4Z)-4-(quinoxalin-6-ylmethylene)-2-[[2-(trifluoromethyl)phenyl]methylamino]-1H-imidazol-5-one (22)

Reaction was carried out according to GP7-A, in THF (0.3M), on a 277 µmol scale of (6.2), with 3 eq of (2-(trifluoromethyl)phenyl)methanamine, at 120 °C (sealed tube, heating block), for 24h. The product directly precipitated in the reaction medium: it was isolated after filtration, washing with cold THF, then pentane. The final product required a trituration in EtOH. Isolated yield: 70%.

### Example 7.20: Synthesis of (4Z)-2-[[(1S,2S)-2-hydroxyindan-1-yl]amino]-4-(quinoxalin-6-ylmethylene)-1H-imidazol-5-one (23)

Reaction was carried out according to GP7-A, in THF (0.3M), on a 277 µmol scale of (6.2), with 3 eq of (1*S*,2*S*)-1-aminoindan-2-ol, at 120 °C (sealed tube, heating block), for 7h. The product directly precipitated in the reaction medium: it was isolated after filtration, washing with cold THF, then pentane. The final product required a trituration in EtOH. Isolated yield: 43%.

### Example 7.21: Synthesis of (4Z)-2-[[(1R)-2-hydroxy-1-phenyl-ethyl]amino]-4-(quinoxalin-6-ylmethylene)-1H-imidazol-5-one (24)

Reaction was carried out according to GP7-B, in THF (0.3M), on a 277 µmol scale of (6.2), with 3 eq of (2*R*)-2-amino-2-phenyl-ethanol, at 120 °C (sealed tube, heating block), for 24h. Purification by FC (elution: DCM/MeOH: 99/1 to 90/10). Isolated yield: 33%.

### Example 7.22: Synthesis of (4Z)-2-[[(1R)-2-methoxy-1-phenyl-ethyl]amino]-4-(quinoxalin-6-ylmethylene)-1H-imidazol-5-one (26)

Reaction was carried out according to GP7-B, in THF (0.3M), on a 277 µmol scale of (6.2), with 3 eq of (1*R*)-2-methoxy-1-phenyl-ethanamine, at 120 °C (sealed tube, heating block), for 44h. Purification by FC (elution: DCM/MeOH: 99/1 to 90/10). Isolated yield: 39%.

### Example 7.23: Synthesis of (4Z)-2-[[(2R)-2-hydroxy-2-phenyl-ethyl]amino]-4-(quinoxalin-6-ylmethylene)-1H-imidazol-5-one (27)

Reaction was carried out according to GP7-A, in dioxane (0.3M), on a 277 µmol scale of (6.2), with 3 eq of (1*R*)-2-amino-1-phenyl-ethanol, at 120 °C (sealed tube, heating block), for 2h. The product directly precipitated in the reaction medium: it was isolated after filtration, washing with cold THF, then pentane. The final product required a trituration in EtOH. Isolated yield: 62%.

### Example 7.24: Synthesis of (4Z)-2-[[(1R)-2-amino-1-phenyl-ethyl]amino]-4-(quinoxalin-6-ylmethylene)-1H-imidazol-5-one dihydrochloride (28)

Reaction was carried out according to GP7-B, in THF (0.3M), on a 277 µmol scale of (6.2), with 3 eq of *tert*-butyl *N*-[(2*R*)-2-amino-2-phenyl-ethyl]carbamate, at 120 °C (sealed tube, heating block), for 45h. Purification by FC (elution: DCM/MeOH: 99/1 to 90/10). The isolated carbamate was engaged in a final deprotection step with HCl in dioxane (4M) at 40 °C. Isolated yield: 42% (2 steps).

### Example 7.25: Synthesis of (4Z)-2-[(5-methylpyrazin-2-yl)methylamino]-4-(quinoxalin-6-ylmethylene)-1H-imidazol-5-one (30)

Reaction was carried out according to GP7-B, in dioxane (0.3M), on a 740 µmol scale of (6.2), with 4 eq of (5-methylpyrazin-2-yl)methanamine, at 130 °C (sealed tube, heating block), for 24h. Purification by FC (elution: DCM/MeOH: 99/1 to 90/10). The final product required two successive triturations in refluxing THF. Isolated yield: 31%.

### Example 7.26: Synthesis of (4Z)-2-[(4-methylthiazol-2-yl)methylamino]-4-(quinoxalin-6-ylmethylene)-1H-imidazol-5-one (31)

Reaction was carried out according to GP7-B, in THF (0.3M), on a 277 µmol scale of (6.2), with 3 eq of (4-methylthiazol-2-yl)methanamine, at 120 °C (sealed tube, heating block), for 7h. Purification by FC (elution: DCM/MeOH: 99/1 to 90/10). The final product required a trituration in EtOH. Isolated yield: 50%.

### Example 7.27: Synthesis of (4Z)-4-(quinoxalin-6-ylmethylene)-2-(tetrahydropyran-4-ylmethylamino)-1H-imidazol-5-one (32)

Reaction was carried out according to GP7-B, in THF (0.3M), on a 277 µmol scale of (6.2), with 3 eq of tetrahydropyran-4-ylmethanamine, at 120 °C (sealed tube, heating block), for 2h. Purification by FC (elution: DCM/MeOH: 99/1 to 90/10). The final product required a trituration in EtOH. Isolated yield: 47%.

### Example 7.28: Synthesis of (4Z)-2-[4-(4-Methylpiperazin-1-yl)anilino]-4-(quinoxalin-6-ylmethylene)-1H-imidazol-5-one (33)

Reaction was carried out according to GP7-A, in dioxane (0.3M), on a 277 µmol scale of (6.2), with 5 eq of 4-(4-methylpiperazin-1-yl)aniline, at 150 °C (sealed tube, heating block), for 6h. The product directly precipitated in the reaction medium: it was isolated after filtration, washing with cold THF, then pentane. The final product required a trituration in EtOH. Isolated yield: 63%.

### Example 7.29: Synthesis of (4Z)-2-[(1-methylpyrazol-3-yl)amino]-4-(quinoxalin-6-ylmethylene)-1H-imidazol-5-one (34)

Reaction was carried out according to GP7-B, in dioxane (0.3M), on a 740 µmol scale of (6.2), with 3 eq of 1-methylpyrazol-3-amine and 6 eq of AcOH, at 130 °C (sealed tube, heating block), for 24h. Purification by FC (elution: DCM/MeOH: 99/1 to 90/10). The final product required a trituration in EtOH. Isolated yield: 36%.

### Example 7.30: Synthesis of (4Z)-2-(2-pyridylamino)-4-(quinoxalin-6-ylmethylene)-1H-imidazol-5-one (35)

Reaction was carried out according to GP7-A, in dioxane (0.3M), on a 277 µmol scale of (6.2), with 5 eq of 2-aminopyridine and 15 eq of AcOH, at 150 °C (sealed tube, heating block), for 52h. The product directly precipitated in the reaction medium: it was isolated after filtration, washing with cold THF, then pentane. The final product required a trituration in EtOH. Isolated yield: 80%.

### Example 7.31: Synthesis of (±)-(4Z)-2-[(6,6-dimethyltetrahydropyran-3-yl)amino]-4-(quinoxalin-6-ylmethylene)-1H-imidazol-5-one (36)

Reaction was carried out according to GP7-B, in THF (0.3M), on a 277 µmol scale of (6.2), with 3 eq of (±)-6,6-dimethyltetrahydropyran-3-amine, at 120 °C (sealed tube, heating block), for 2h. Purification by FC (elution: DCM/MeOH: 99/1 to 90/10). Isolated yield: 44%.

### Example 7.32: Synthesis of (4Z)-4-(quinoxalin-6-ylmethylene)-2-[[(3R)-tetrahydrofuran-3-yl]amino]-1H-imidazol-5-one (37)

Reaction was carried out according to GP7-B, in THF (0.3M), on a 277 µmol scale of (6.2), with 4 eq of (3*R*)-tetrahydrofuran-3-amine, at 120 °C (sealed tube, heating block), for 24h. Purification by FC (elution: DCM/MeOH: 99/1 to 90/10). Isolated yield: 37%.

### Example 7.33: Synthesis of (4Z)-4-(quinoxalin-6-ylmethylene)-2-[[(3S)-tetrahydropyran-3-yl]amino]-1H-imidazol-5-one (38)

Reaction was carried out according to GP7-B, in THF (0.3M), on a 740 µmol scale of (6.2), with 3 eq of (3S)-tetrahydropyran-3-amine, at 120 °C (sealed tube, heating block), for 24h. Purification by FC (elution: DCM/MeOH: 99/1 to 90/10). Isolated yield: 50%.

### Example 7.34: Synthesis of (4Z)-4-(quinoxalin-6-ylmethylene)-2-[[(3R)-tetrahydropyran-3-yl]amino]-1H-imidazol-5-one (39)

Reaction was carried out according to GP7-B, in THF (0.3M), on a 740 µmol scale of (6.2), with 3 eq of (3*R*)-tetrahydropyran-3-amine hydrochloride and 6 eq of DIPEA, at 120 °C (sealed tube, heating block), for 24h. Purification by FC (elution: DCM/MeOH: 99/1 to 90/10). Isolated yield: 36%.

### Example 7.35: Synthesis of (4Z)-2-[[(3R,4R)-4-Hydroxytetrahydropyran-3-yl]amino]-4-(quinoxalin-6-ylmethylene)-1H-imidazol-5-one (40)

Reaction was carried out according to GP7-B, in THF (0.3M), on a 277 µmol scale of (6.2), with 3 eq of (3*R*,4*R*)-3-aminotetrahydropyran-4-ol, at 120 °C (sealed tube, heating block), for 24h. Purification by FC (elution: DCM/MeOH: 99/1 to 90/10). The final product required a trituration in EtOH. Isolated yield: 45%.

### Example 7.36: Synthesis of (4Z)-2-(1,4-dioxepan-6-ylamino)-4-(quinoxalin-6-ylmethylene)-1H-imidazol-5-one (42)

Reaction was carried out according to GP7-A, in THF (0.3M), on a 521 µmol scale of (6.2), with 2.5 eq of 1,4-dioxepan-6-amine hydrochloride and 6 eq of DIPEA, at 135 °C (sealed tube, heating block), for 12h. The product directly precipitated in the reaction medium: it was isolated after filtration, washing with cold THF, then pentane. The final product required a trituration in MeOH. Isolated yield: 42%.

### Selected examples from quinoline sub-series:

### Example 7.37: Synthesis of (4Z)-2-(cycloheptylamino)-4-(6-quinolylmethylene)-1H-imidazol-5-one (44)

Reaction was carried out according to GP7-B, in THF (0.3M), on a 743 µmol scale of (6.4), with 4 eq of cycloheptylamine, at 120 °C (sealed tube, heating block), for 24h. Purification by FC (elution: DCM/MeOH: 99/1 to 90/10). Isolated yield: 51%.

### Example 7.38: Synthesis of (4Z)-2-[[(1R)-1-(hydroxymethyl)-3-methyl-butyl]amino]-4-(6-quinolylmethylene)-1H-imidazol-5-one (46)

Reaction was carried out according to GP7-B, in THF (0.3M), on a 743 µmol scale of (6.4), with 4 eq of (*R*)-leucinol, at 120 °C (sealed tube, heating block), for 24h. Purification by FC (elution: DCM/MeOH: 99/1 to 90/10). Isolated yield: 29%.

### Example 7.39: Synthesis of (4Z)-2-[[(1R)-1-(methoxymethyl)-3-methyl-butyl]amino]-4-(6-quinolylmethylene)-1H-imidazol-5-one (47)

Reaction was carried out according to GP7-B, in THF (0.3M), on a 557 µmol scale of (6.4), with 4 eq of (2*R*)-1-methoxy-4-methyl-pentan-2-amine, at 130 °C (sealed tube, heating block), for 24h. Purification by FC (elution: DCM/MeOH: 99/1 to 90/10). The final product required a trituration in ACN at 0 °C. Isolated yield: 43%.

### Example 7.40: Synthesis of (4Z)-2-(3-noradamantylamino)-4-(6-quinolylmethylene)-1H-imidazol-5-one (48)

Reaction was carried out according to GP7-B, in dioxane (0.3M), on a 587 µmol scale of (6.4), with 4 eq of 3-noradamantanamine and 6 eq of AcOH, at 160 °C (sealed tube, heating block), for 24h. Purification by FC (elution: DCM/MeOH: 99/1 to 90/10). The final product required a trituration in EtOH. Isolated yield: 50%.

### Example 7.41: Synthesis of (4Z)-2-(1-adamantylamino)-4-(6-quinolylmethylene)-1H-imidazol-5-one (49)

Reaction was carried out according to GP7-A, in dioxane (0.3M), on a 557 µmol scale of (6.4), with 4 eq of adamantan-1-amine and 6 eq of AcOH, at 150 °C (sealed tube, heating block), for 24h. The product directly precipitated in the reaction medium: it was isolated after filtration, washing with cold THF, then pentane. The final product required two successive triturations in refluxing EtOH. Isolated yield: 47%.

### Example 7.42: Synthesis of (4Z)-2-[(3-hydroxy-1-adamantyl)amino]-4-(6-quinolylmethylene)-1H-imidazol-5-one (50)

Reaction was carried out according to GP7-B, in dioxane (0.3M), on a 557 µmol scale of (6.4), with 4 eq of 3-hydroxydamantan-1-amine and 6 eq of AcOH, at 155 °C (sealed tube, heating block), for 24h. Purification by FC (elution: DCM/MeOH: 99/1 to 90/10). The final product required a trituration in ACN at 0 °C. Isolated yield: 40%.

### Example 7.43: Synthesis of (4Z)-2-[[(1R)-2-methoxy-1-phenyl-ethyl]amino]-4-(6-quinolylmethylene)-1H-imidazol-5-one (52)

Reaction was carried out according to GP7-B, in THF (0.3M), on a 557 µmol scale of (6.4), with 4 eq of (1*R*)-2-methoxy-1-phenyl-ethanamine and 6 eq AcOH, at 130 °C (sealed tube, heating block), for 24h. Purification by FC (elution: DCM/MeOH: 99/1 to 90/10). The final product required a trituration in ACN at 0 °C. Isolated yield: 41%.

### Example 7.44: Synthesis of (4Z)-2-[(1-methylpyrazol-3-yl)amino]-4-(6-quinolylmethylene)-1H-imidazol-5-one (54)

Reaction was carried out according to GP7-B, in dioxane (0.3M), on a 743 µmol scale of (6.4), with 3 eq of 1-methylpyrazol-3-amine and 6 eq of AcOH, at 130 °C (sealed tube, heating block), for 24h. Purification by FC (elution: DCM/MeOH: 99/1 to 90/10). The final product required a trituration in EtOH. Isolated yield: 22%.

### Example 7.45: Synthesis of (4Z)-4-(6-quinolylmethylene)-2-[[(3R)-tetrahydropyran-3-yl]amino]-1H-imidazol-5-one (56)

Reaction was carried out according to GP7-B, in THF (0.3M), on a 743 µmol scale of (6.4), with 3 eq of (3*R*)-tetrahydropyran-3-amine hydrochloride and 6 eq of DIPEA, at 120 °C (sealed tube, heating block), for 24h. Purification by FC (elution: DCM/MeOH: 99/1 to 90/10). Isolated yield: 62%.

### Selected examples from isoquinoline sub-series :

### Example 7.46: Synthesis of (4Z)-2-(cycloheptylamino)-4-(6-isoquinolylmethylene)-1H-imidazol-5-one (57)

Reaction was carried out according to GP7-B, in THF (0.3M), on a 743 µmol scale of (6.5), with 4 eq of cycloheptylamine, at 120 °C (sealed tube, heating block), for 36h. Purification by FC (elution: DCM/MeOH: 99/1 to 90/10). The final product required a trituration in ACN at 0 °C. Isolated yield: 25%.

### Example 7.47: Synthesis of (4Z)-2-[[(1R)-1-(hydroxymethyl)-3-methylbutyl]amino]-4-(6-isoquinolylmethylene)-1H-imidazol-5-one (59)

Reaction was carried out according to GP7-B, in THF (0.3M), on a 743 µmol scale of (6.5), with 4 eq of (*R*)-leucinol, at 120 °C (sealed tube, heating block), for 24h. Purification by FC (elution: DCM/MeOH: 99/1 to 90/10). The final product required a trituration in DCM at 0 °C. Isolated yield: 25%.

### Example 7.48: Synthesis of (4Z)-4-(6-isoquinolylmethylene)-2-[[(1R)-1-(methoxymethyl)-3-methyl-butyl]amino]-1H-imidazol-5-one (60)

Reaction carried out according to GP6, on a 352 µmol scale of (2.5), with 1.2 eq of isoquinoline-6-carbaldehyde. Purification by FC (elution: DCM/MeOH: 99/1 to 90/10). The final product required a trituration in ACN at 0 °C. Isolated yield: 27%.

### Example 7.49: Synthesis of (4Z)-2-(1-adamantylamino)-4-(6-isoquinolylmethylene)-1H-imidazol-5-one (61)

Reaction carried out according to GP6, on a 352 µmol scale of (2.11), with 1.2 eq of isoquinoline-6-carbaldehyde. Purification by FC (elution: DCM/MeOH: 99/1 to 90/10). The final product required a reprecipitation from DCM/pentane at 0 °C. Isolated yield: 50%.

### Example 7.50: Synthesis of (4Z)-2-(benzylamino)-4-(6-isoquinolylmethylene)-1H-imidazol-5-one (62)

Reaction was carried out according to GP7-B, in THF (0.3M), on a 743 µmol scale of (6.5), with 4 eq of benzylamine, at 120 °C (sealed tube, heating block), for 24h. Purification by FC (elution: DCM/MeOH: 99/1 to 90/10). The final product required a trituration in DCM at 0 °C. Isolated yield: 24%.

### Example 7.51: Synthesis of (4Z)-4-(6-isoquinolylmethylene)-2-[[(1R)-2-methoxy-1-phenyl-ethyl]amino]-1H-imidazol-5-one (63)

Reaction carried out according to GP6, on a 352 µmol scale of (2.15), with 1.2 eq of isoquinoline-6-carbaldehyde. Purification by FC (elution: DCM/MeOH: 99/1 to 90/10). The final product required a reprecipitation from DCM/pentane at 0 °C. Isolated yield: 31%.

### Example 7.52: Synthesis of (4Z)-4-(6-Isoquinolylmethylene)-2-[[(3R)-tetrahydrofuran-3-yl]amino]-1H-imidazol-5-one (66)

Reaction was carried out according to GP7-B, in THF (0.3M), on a 743 µmol scale of (6.5), with 4 eq of (3*R*)-tetrahydrofuran-3-amine, at 120 °C (sealed tube, heating block), for 24h. Purification by FC (elution: DCM/MeOH: 99/1 to 90/10). Isolated yield: 31%.

### Example 7.53: Synthesis of (4Z)-4-(6-isoquinolylmethylene)-2-[[(3R)-tetrahydropyran-3-yl]amino]-1H-imidazol-5-one (67)

Reaction was carried out according to GP7-B, in THF (0.3M), on a 743 µmol scale of (6.5), with 3 eq of (3*R*)-tetrahydropyran-3-amine hydrochloride and 6 eq of DIPEA, at 130 °C (sealed tube, heating block), for 24h. Purification by FC (elution: DCM/MeOH: 99/1 to 90/10). Isolated yield: 37%.

### Selected examples from quinazoline sub-series :

### Example 7.54: Synthesis of (4Z)-2-(1-adamantylamino)-4-(quinazolin-6-ylmethylene)-1H-imidazol-5-one (68)

Reaction was carried out according to GP7-A, in dioxane (0.3M), on a 277 µmol scale of (6.6), with 3 eq of adamantan-1-amine and 9 eq of AcOH, at 150 °C (sealed tube, heating block), for 2h. The product directly precipitated in the reaction medium: it was isolated after filtration, washing with cold THF, then pentane. The final product required a trituration in EtOH. Isolated yield: 49%.

### Example 7.55: Synthesis of (4Z)-2-[[(1R)-2-methoxy-1-phenyl-ethyl]amino]-4-(quinazolin-6-ylmethylene)-1H-imidazol-5-one (69)

Reaction was carried out according to GP7-B, in dioxane (0.3M), on a 277 µmol scale of (6.6), with 3 eq of (1*R*)-2-methoxy-1-phenyl-ethanamine, at 120 °C (sealed tube, heating block), for 30h. Purification by FC (elution: DCM/MeOH: 99/1 to 90/10). The final product required a trituration in EtOH. Isolated yield: 62%.

### Selected examples from naphthyrydine sub-series :

### Example 7.56: Synthesis of (4Z)-2-(1-adamantylamino)-4-(1,5-naphthyridin-2-ylmethylene)-1H-imidazol-5-one (70)

Reaction was carried out according to GP7-B, in dioxane (0.3M), on a 277 µmol scale of (6.7), with 3 eq of adamantan-1-amine and 9 eq of AcOH, at 150 °C (sealed tube, heating block), for 2h. Purification by FC (elution: DCM/MeOH: 99/1 to 90/10). The final product required a trituration in EtOH. Isolated yield: 75%.

### Example 7.57: Synthesis of (4Z)-2-[[(1R)-2-methoxy-1-phenyl-ethyl]amino]-4-(1,5-naphthyridin-2-ylmethylene)-1H-imidazol-5-one (71)

Reaction was carried out according to GP7-B, in dioxane (0.3M), on a 277 µmol scale of (6.7), with 3 eq of (1*R*)-2-methoxy-1-phenyl-ethanamine, at 120 °C (sealed tube, heating block), for 30h. Purification by FC (elution: DCM/MeOH: 99/1 to 90/10). The final product required a trituration in EtOH. Isolated yield: 49%.

### Selected examples from cinnoline sub-series :

### Example 7.58: Synthesis of (4Z)-4-(cinnolin-6-ylmethylene)-2-[[(1R)-2-methoxy-1-phenyl-ethyl]amino]-1H-imidazol-5-one (72)

Reaction was carried out according to GP7-B, in dioxane (0.3M), on a 279 µmol scale of (6.8), with 3 eq of (1*R*)-2-methoxy-1-phenyl-ethanamine, at 120 °C (sealed tube, heating block), for 96h. Purification by FC (elution: DCM/MeOH: 99/1 to 90/10). The final product required a trituration in EtOH. Isolated yield: 60%.

### Selected examples from phthalazine sub-series :

### Example 7.59: Synthesis of (4Z)-2-[[(1R)-2-methoxy-1-phenyl-ethyl]amino]-4-(phthalazin-6-ylmethylene)-1H-imidazol-5-one (73)

Reaction was carried out according to GP7-B, in dioxane (0.3M), on a 279 µmol scale of (6.9), with 3 eq of (1*R*)-2-methoxy-1-phenyl-ethanamine, at 120 °C (sealed tube, heating block), for 96h. Purification by FC (elution: DCM/MeOH: 99/1 to 90/10). The final product required a trituration in EtOH. Isolated yield: 57%.

### Example 8: Biological activity

### MATERIAL AND METHODS

### PROTEIN KINASE ASSAYS

### 1. Overview

Assays were performed by ProQinase GmbH (Engesserstr. 4, D-79108 Freiburg, Germany, www.proqinase.com).The IC₅₀ profile of all compounds was determined using 12 protein kinases (CDK2/cyclin E, CK1ε, CLK1, 2, 3, 4, DYRK1A, 1B, 2, 3, 4, GSK3β). IC₅₀ values were measured by testing 10 concentrations (10 µM to 30 nM) of each compound in singlicate.

### 2. Test Compounds

The compounds were provided as 1µM stock solutions in 100% DMSO. Prior to testing, the 1 µM stock solutions were subjected to a serial, semi-logarithmic dilution using 100 % DMSO as a solvent. This resulted in 10 distinct concentrations, with a dilution endpoint of 3 x 10 nM/100 % DMSO, with 100 % DMSO as controls. In the process, 90 µl H₂O were added to each well of each compound dilution plate. To minimize potential precipitation, the H₂O was added to each plate only a few minutes before the transfer of the compound solutions into the assay plates. The plate was shaken thoroughly, resulting in a compound dilution plate/ 10 % DMSO.

For the assays (see below), 5 µL solution from each well of the compound dilution plates/10 % DMSO were transferred into the assay plates. The final volume of the assay was 50 µL. All compounds were tested at 10 final assay concentrations in the range from 10 µM to 30 nM. The final DMSO concentration in the reaction cocktails was 1 % in all cases.

### 3. Recombinant Protein Kinases

All protein kinases provided by ProQinase were expressed in Sf9 insect cells or in *E. coli* as recombinant GST-fusion proteins or His-tagged proteins, either as full-length or enzymatically active fragments. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy.

### 4. Protein Kinase Assay

A radiometric protein kinase assay (33PanQinase^{®} Activity Assay) was used for measuring the kinase activity of the 12 protein kinases. All kinase assays were performed in 96-well FlashPlatesTM from PerkinElmer (Boston, MA, USA) in a 50 µL reaction volume. The reaction cocktail was pipetted in four steps in the following order:
- 25 µL of assay buffer (standard buffer/[γ-³³P]-ATP),
- 10 µL of ATP solution (in H₂O),
- 5 µL of test compound (in 10 % DMSO),
- 10 µL of enzyme/substrate mixture.

The assay for all protein kinases contained 70 mM HEPES-NaOH pH 7.5, 3 mM MgCl₂, 3 mM MnCl₂, 3 µM Na-orthovanadate, 1.2 mM DTT, 50 µg/ml PEG20000, ATP (variable concentrations, corresponding to the apparent ATP-Km of the respective kinase), [γ-³³P]-ATP (approx. 6.5 x 10-05 cpm per well), protein kinase (variable amounts), and substrate (variable amounts). The reaction cocktails were incubated at 30°C for 60 minutes. The reaction was stopped with 50 µL of 2% (v/v) H₃PO₄, plates were aspirated and washed two times with 200 µL 0.9% (w/v) NaCl. Incorporation of ³³Pi was determined with a microplate scintillation counter (Microbeta, Wallac). The IC₅₀ values for all compounds were calculated from the dose response curves

### 5. Quality controls

As a parameter for assay quality, the Z'-factor (Zhang et al., J. Biomol. Screen. 2: 67-73, 1999) for the low and high controls of each assay plate (n = 8) was used. ProQinase's criterion for repetition of an assay plate is a Z'-factor below 0.4 (Iversen et al., J. Biomol. Screen. 3: 247-252, 2006).

Their activity has been classified according to two criteria, kinase inhibition potency and kinase selectivity.

The **kinase inhibition potency classification** was done according to the following ranges of IC₅₀ values:
Some compounds have an IC₅₀ activity varying from above 0.050 µM. These compounds correspond to the above-reported class **E.** Some compounds have an IC₅₀ activity varying from 0.025 to 0.050 µM. These compounds correspond to the above-reported class **D.** Some compounds of the invention have an IC₅₀ activity varying from 0.010 to 0.025 µM. These compounds correspond to the above-reported class **C.** Further some particular compounds have an IC₅₀ activity varying from 0.005 to 0.010 µM. These compounds correspond to the above-reported class **B.** Even preferred are compounds of the invention that have an IC₅₀ activity of less than 0.005 µM. These compounds correspond to the above-reported class **A.** This classification was applied to CLK1 and DYRK1A. Said letters A to E were used to quote the activity/efficacy of the compounds of the invention in the following Table 4, 4A and 4B.

The **kinase selectivity classification** was based on comparing the IC₅₀ values of DYRK1A with that of CLK1 or with that of DYRK1B. The classification was done according to the following ranges of values: **I**: ratio of IC₅₀ on CLK1 or DYRK1B over IC₅₀ on DYRK1A ≥ 10 fold (most DYRK1A-selective compounds); **II**: ratio between 2 and 10 fold; **III:** ratio between 0.5 and 2 fold; **IV:** ratio between 0.1 and 0.5 fold; V: ratio > 0.1 fold (most CLK1- or DYRK1B-selective compounds). Said numbers I to V were used to quote the relative selectivity of the compounds of the invention in the following Tables.

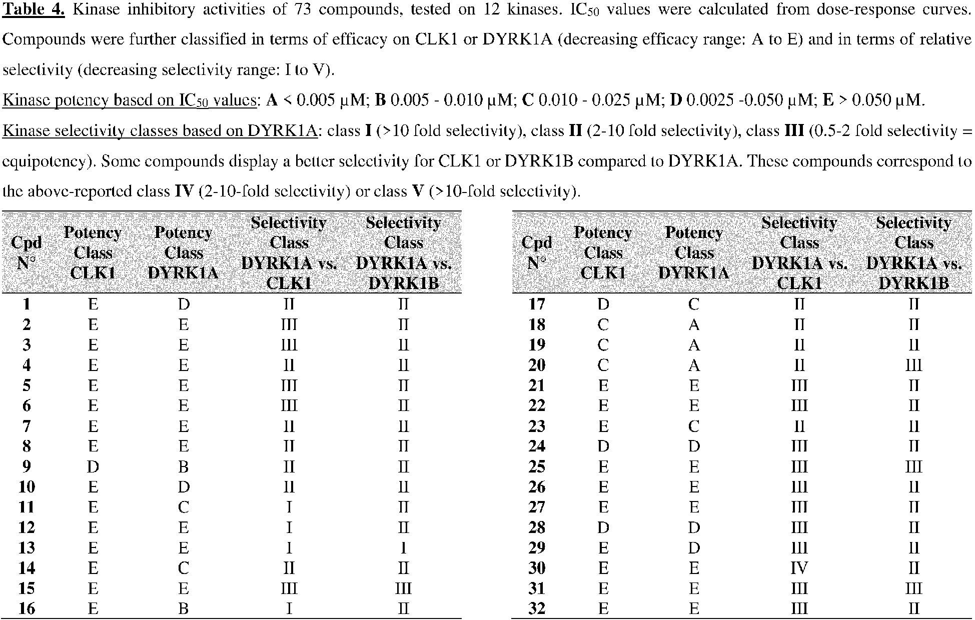

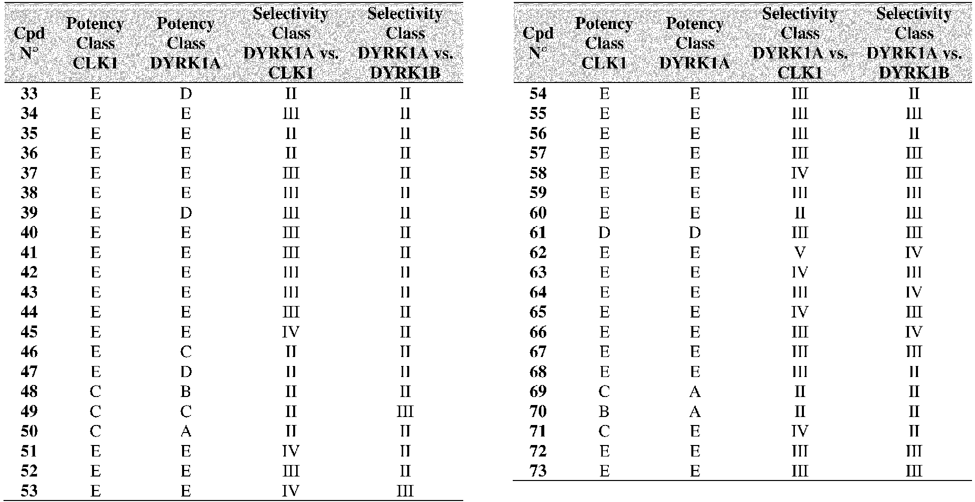

**Table 4A. Most potent CLK1 inhibitors (IC₅₀ ≤ 10 nM). Kinase inhibition potency classes: IC₅₀ values: A ≤ 0.005 µM; B ≤ 0.010 µM; C ≤ 0.025 µM; D ≤ 0.050 µM; E > 0.050 µM. Kinase selectivity classes based on DYRK1A: class I (>10 fold selectivity), class II (2-10 fold selectivity), class III (0.5-2 fold selectivity = equipotency). Some compounds display a better selectivity for CLK1 or DYRK1B compared to DYRK1A. These compounds correspond to the above-reported class IV (2-10-fold selectivity) or class V (>10-fold selectivity).**

| **Cpd N°** | **Potency Class CLK1** | **Potency Class DYRK1A** | **Selectivity Class DYRK1A vs. CLK1** |
|---|---|---|---|
| **70** | B | A | II |
| **20** | C | A | II |
| **50** | C | A | II |
| **69** | C | A | II |
| **18** | C | A | II |
| **48** | C | B | II |
| **19** | C | A | II |
| **49** | C | C | II |
| **71** | C | E | IV |

The most potent CLK1 inhibitors, with an IC₅₀ lower or equal to 10 nM are compounds (20) and (70).

The most potent CLK2 inhibitors, with an IC₅₀ lower or equal to 100 nM are compounds (17), (19), (20), (49), (50), (61), (69) and (70).

The most potent CLK3 inhibitors, with an IC₅₀ lower or equal to 500 nM is compound (70).

The most potent CLK4 inhibitors, with an IC₅₀ lower or equal to 10 nM are compounds (16), (18), (19), (20), (48), (50), (69), (70) and (71).

**Table 4B. Most potent DYRK1A inhibitors (IC₅₀ ≤ 10 nM). Kinase Inhibition Efficacy Classes and Kinase Selectivity Classes as described in Table 4A.**

| **Cpd N°** | **Potency Class CLK1** | **Potency Class DYRK1A** | **Selectivity Class DYRK1A vs. CLK1** | **Selectivity Class DYRK1A vs. DYRK1B** |
|---|---|---|---|---|
| **50** | C | A | II | II |
| **18** | C | A | II | II |
| **20** | C | A | II | III |
| **19** | C | A | II | II |
| **70** | B | A | II | II |
| **69** | C | A | II | II |
| **16** | E | B | I | II |
| **48** | C | B | II | II |
| **9** | D | B | II | II |
| **49** | C | B | II | III |

The most potent DYRK1a inhibitors, with an IC₅₀ lower or equal to 10 nM are compounds (9), (16), (18), (19), (20), (48), (49), (50), (69) and (70).

The most potent DYRK1B inhibitors, with an IC₅₀ lower or equal to 10 nM are compounds (18), (19), (20), (50), (69) and (70).

The most potent DYRK2 inhibitors, with an IC₅₀ lower or equal to 250 nM are compounds (16), (19), (20), (48), (50) and (69).

The most potent DYRK3 inhibitors, with an IC₅₀ lower or equal to 250 nM are compounds (20), (48), (49), (50), (61), (69) and (70).

The most potent DYRK4 inhibitors, with an IC₅₀ lower or equal to 300 nM are compounds (16) and (50).

**Table 4C. Most DYRK1A selective inhibitors compared to CLK1. Kinase Selectivity Classes as described in Table 4A.**

| **Cpd N°** | **Selectivity Class DYRK1A vs. CLK1** |
|---|---|
| **13** | I |
| **11** | I |
| **16** | I |
| **12** | I |
| **33** | II |
| **7** | II |
| **1** | II |
| **4** | II |
| **36** | II |
| **17** | II |
| **70** | II |
| **49** | II |
| **35** | II |
| **48** | II |
| **69** | II |
| **23** | II |
| **20** | II |
| **47** | II |
| **8** | II |
| **14** | II |
| **9** | II |
| **60** | II |
| **46** | II |
| **18** | II |
| **50** | II |
| **19** | II |
| **10** | II |

**Table 4D. Most CLK1 selective inhibitors compared to DYRK1A. Kinase Selectivity Classes as described in Table 4A.**

| **Cpd N°** | **Selectivity Class DYRK1A vs. CLK1** |
|---|---|
| **62** | V |
| **45** | IV |
| **71** | IV |
| **53** | IV |
| **63** | IV |
| **65** | IV |
| **30** | IV |
| **58** | IV |
| **51** | IV |
| **62** | V |

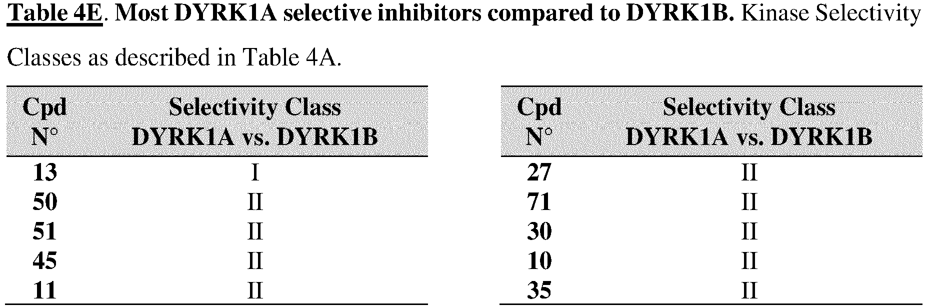

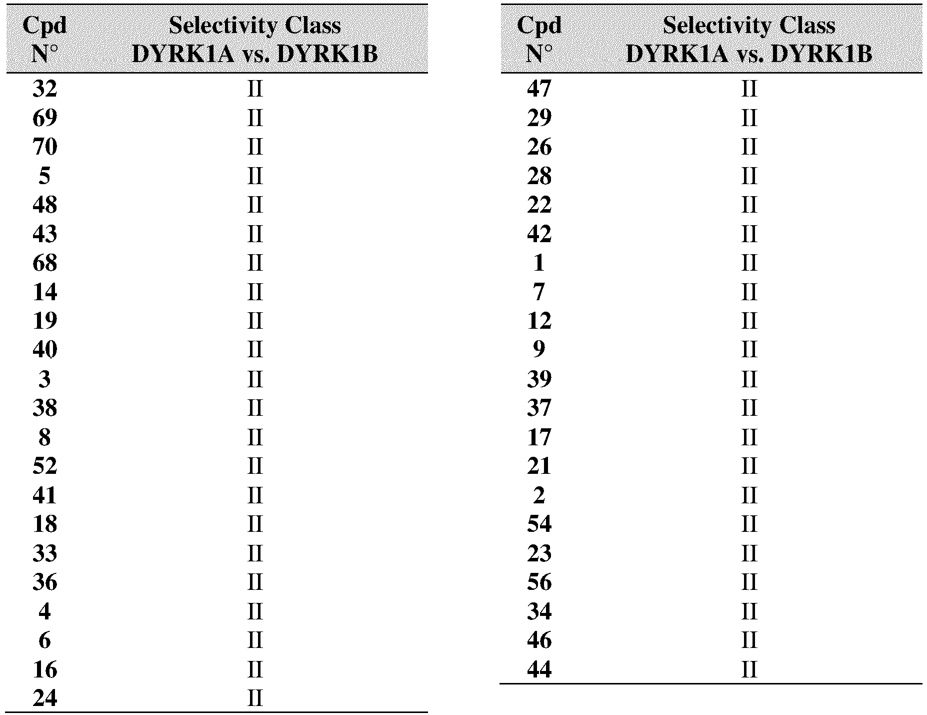

**Table 4F. Most DYRK1B selective inhibitors compared to DYRK1A. Kinase Selectivity Classes as described in Table 4A.**

| **Cpd N°** | **Selectivity Class DYRK1A vs. DYRK1B** |
|---|---|
| **64** | IV |
| **66** | IV |
| **62** | IV |

### RESULTS

Most compounds of the present invention present an IC₅₀ activity of less than 2 µM on CLKs or DYRKs. All the compounds were inhibitors of **DYRK1A** and CLK1 (Table 4).

### Compounds were preferably inhibitory on CLK1 (Table 4A), CLK4, DYRK1A (Table 4B) and DYRK1B.

Some are most selective for **DYRK1A** vs. CLK1 (Table 4C), **DYRK1A** vs. DYRK1B (Table 4E), DYRK1B vs. DYRK1A (Table 4F) or CLK1 vs. DYRK1A (Table 4D). Some compounds display a better selectivity for DYRK1A compared to CLK1 or DYRK1B. These compounds correspond to the above-reported class **I** (> 10-fold selectivity) or class **II** (2 to 10-fold selectivity). Some compounds are equipotent. These compounds correspond to the above-reported class **III** (0.5 to 2-fold selectivity). Some compounds display a better selectivity for CLK1 or DYRK1B compared to DYRK1A. These compounds correspond to the above-reported class **IV** (2 to 10-fold selectivity) or class **V** (>10-fold selectivity).

### CONCLUSION

Based on the previous results, it can be concluded that the compounds of formula (I) are suitable chemical compounds in the prevention and/or treatment of cognitive deficits and neuroinflammation associated with Down syndrome (Trisomy 21), Alzheimer's disease and related diseases, dementia and/or tauopathies; other neurodegenerative diseases (Parkinson's disease; Pick disease, including Niemann-Pick Type C Disease); CDKL5 Deficiency Disorder; type 1 and type 2 diabetes; abnormal folate and methionine metabolism; tendinopathy and osteoarthritis, in particular knee osteoarthritis,; Duchenne muscular dystrophy; several cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), malaria, Leishmaniasis, Chagas and sleeping sickness *(Trypanosoma* sp.), and cattle diseases due to unicellular pathogens, viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and in the regulation of body temperature.

It may further be concluded that some compounds of formula (I) are further suitable for treating and/or preventing Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as malaria, Leishmaniasis, Chagas and sleeping sickness *(Trypanosoma* sp.), and cattle diseases due to unicellular pathogens.

Leucettinibs display much increased efficacy (down to sub-micromolar and single-digit micromolar IC₅₀ values) compared to reference compounds. They also display a large range of selectivity towards DYRK1A or CLK1/CLK4. And some products being equipotent on CLKs and DYRKs may also find applications as dual-specificity inhibitors.

The present invention further relates to a pharmaceutical composition comprising at least one compound of formula (I) as defined above or any of its pharmaceutically acceptable salts or at least any of compounds (1) to (73) as defined above or any of its pharmaceutically acceptable salts.

The present invention further relates to a pharmaceutical composition comprising at least one compound of formula (I) as defined above or any of its pharmaceutically acceptable salts or at least any of compounds (1) to (73) as defined above or any of its pharmaceutically acceptable salts and also at least one pharmaceutically acceptable excipient.

Pharmaceutical compositions of the invention can contain one or more compound(s) of the invention in any form described herein.

Still a further object of the present invention consists of a compound of formula (I) as defined above, and compounds (1) to (73) as defined above, or one of their pharmaceutically acceptable salts for use in the treatment and/or prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such malaria, Leishmaniasis, Chagas and sleeping sickness *(Trypanosoma* sp.), and cattle diseases due to unicellular pathogens, and for regulating body temperature.

Still a further object of the present invention consists of a compound of formula (I) as defined above, and compounds (1) to (73) as defined above, or one of their pharmaceutically acceptable salts for use in the treatment and/or prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease; Pick disease, including Niemann-Pick Type C Disease; CDKL5 Deficiency Disorder; type 1 and type 2 diabetes; abnomral folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness *(Trypanosoma* sp.), and cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of a compound of formula (I), as defined above, and compounds (1) to (73) as defined above, or one of their pharmaceutically acceptable salts for use in the treatment and/or prevention of a disease selected from type 1 and type 2 diabetes; viral infections, in partiuclar as mentioned above; tendinopathy and osteoarthritis; cancers, in particular as mentioned above; infections caused by unicellular parasites, such malaria, Leishmaniasis, Chagas and sleeping sickness *(Trypanosoma* sp.); cattle diseases due to unicellular pathogens,and to regulate body temperature.

Still a further object of the present invention consists of a compound of formula (I), as defined above, and compounds (1) to (73) as defined above, or one of their pharmaceutically acceptable salts for use in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions and type 1 and type 2 diabetes.

Still a further object of the present invention consists of the use of at least one compound of formula (I) as defined above, and compounds (1) to (73) as defined above, or one of their pharmaceutically acceptable salts according to the present invention for preparing a drug for preventing and/or treating a disease selected from cognitive deficits and neuroinflammation associated with Down syndrome (Trisomy 21); Alzheimer's disease and related diseases; dementia; tauopathies; and other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, such as megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as malaria, Leishmaniasis, Chagas and sleeping sickness *(Trypanosoma* sp.), cattle diseases due to unicellular pathogens, and for regulating body temperature.

Still a further object of the present invention consists of the use of at least one compound of formula (I) as defined above, and compounds (1) to (73) as defined above, or one of their pharmaceutically acceptable salts according to the present invention for preparing a drug for preventing and/or treating a disease selected from cognitive deficits and neuroinflammation associated with Down syndrome (Trisomy 21), Alzheimer's disease and related diseases, dementia and/or tauopathies; other neurodegenerative diseases such as Parkinson's disease and Pick disease, in particular Niemann-Pick Type C Disease; CDKL5 Deficiency Disorder; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness *(Trypanosoma* sp.), cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of the use of at least one compound of formula (I), as defined above, and compounds (1) to (73) as defined above, or one of their pharmaceutically acceptable salts according to the present invention for preparing a drug for combatting a disease selected from type 1 and type 2 diabetes, viral infections, in particular as mentioned above, osteoarthritis and tendinopathy, cancers and leukemias, in particular as mentioned above, infections caused by unicellular parasites, such malaria, Leishmaniasis, Chagas and sleeping sickness *(Trypanosoma* sp.), cattle diseases due to unicellular pathogens, and to regulate body temperature.

Another object of the invention relates to a therapeutic method for the treatment and/or for the prevention of a disease selected from cognitive deficits and neuroinflammation associated with Down syndrome (Trisomy 21); Alzheimer's disease and related diseases; dementia; tauopathies; and other neurodegenerative diseases such as Parkinson's disease; Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness *(Trypanosoma* sp.), and cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of the use of at least one compound of formula (I), as defined above, and compounds (1) to (73) as defined above, or one of their pharmaceutically acceptable salts according to the present invention for preparing a drug for combatting a disease selected from type 1 and type 2 diabetes, viral infections, in partiuclar as mentioned above, tendinopathy and osteoarthritis, cancers, in particular as mentioned above, infections caused by unicellular parasites, such malaria, Leishmaniasis, Chagas and sleeping sickness *(Trypanosoma* sp.), cattle diseases due to unicellular pathogens, and to regulate body temperature.

According to a particular embodiment, the treatment is continuous or noncontinuous.

A "continuous treatment" means a long-term treatment which can be implemented with various administration frequencies, such as once or twice every day, every three days, once a week, or once every two weeks or once every month or by transdermal patch delivery.

According to one embodiment, the compound of formula (I), or any of its pharmaceutically acceptable salts, is administered at a dose varying from 0.1 to 1000 mg. in particular varying from 0.5 to 500 mg, or for example varying from 5 to 100 mg.

Another object of the invention relates to a therapeutic method for the treatment and/or for the prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such malaria, Leishmaniasis, Chagas and sleeping sickness *(Trypanosoma* sp.), cattle diseases due to unicellular pathogens, and for the regulation of the body temperature, in a patient in need thereof, comprising at least a step of administering a therapeutically effective amount of a compound of formula (I) or of compounds (1) to (73), as defined above. or one of their acceptable salts.

In a specific embodiment, the invention provides a use of a compound of formula (I) according to the invention or a pharmaceutically acceptable salt thereof or a pharmaceutically active derivative thereof or a method according to the invention wherein the compound of formula (I) is to be administered in combination with a co-agent useful in anyone of the hereabove mentioned diseases.

In a specific embodiment, the invention provides a use of a compound of formula (I) according to the invention or a pharmaceutically acceptable salt thereof or a pharmaceutically active derivative thereof or a method according to the invention wherein the compound of formula (I) is to be administered in combination with a co-agent useful in anyone of the hereabove mentioned diseases.

The compounds can be administered through any mode of administration such as, for example, intramuscular, intravenous, intranasal or oral route, transdermal patch, etc.

The inventive composition can further include one or more additives such as diluents, excipients, stabilizers and preservatives. Such additives are well known to those skilled in the art and are described notably in "Ullmann's Encyclopedia of Industrial Chemistry, 6th Ed." (various editors, 1989-1998, Marcel Dekker) and in "Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.

Oral route of administration is in particular preferred in the prophylaxis or treatment aspect of the invention.

## Claims

1. A compound of formula (I) 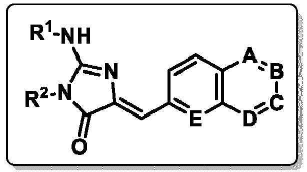 wherein:
A, B, C, D and E are selected from the group consisting of =CH- and -N=,
at least one and not more than two of A, B, C, D and E is -N=,
at least one of A and B is -N=,
R² is selected from a hydrogen atom, a (C₁-C₄)alkyl group and a (C3-C6)cycloalkyl group, and
wherein R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkyl group, a (C₁-C₄)alkoxy group, a halogen atom and a hydroxy group,
(iii). a fused phenyl group, selected from phenyl groups fused with a (C₅-C₆)cycloalkyl, which (C₅-C₆)cycloalkyl is optionally substituted by a hydroxy group,
(iv). a phenyl group, substituted by a (C₄-C₇)heterocycloalkyl group, said (C₄-C₇)heterocycloalkyl group being itself optionally substituted by a (C₁-C₄) alkyl group, or
(v). a R'-L- group, wherein L is either a single bond or a (C₁-C₃)alkanediyl group, optionnally susbstituted by a group selected from a hydroxy group and a (C₁-C₃)alkoxy group, and
R' represents:
(v.1). a (C₃-C₈)cycloalkyl group, optionally substituted by a group selected from a hydroxy group and a (C₁-C₃) alkoxy group,
(v.2). a (C3-C9)heterocycloalkyl group, optionally substituted by one or two groups selected from a hydroxy group and (C₁-C₄)alkyl groups, or
(v.3). a (C₃-C₈)heteroaryl group, optionally substituted by a (C₁-C₄)alkyl group, or
(vi). a R"-L- group wherein L is a (C₁-C₃)alkanediyl group, optionally substituted by a group selected from a -NR^{b}R^{c} group, a (C₁-C₃)alkoxy group and a hydroxy group, and
R" is a phenyl group, optionally substituted by a fluoro(C₁-C₄)alkyl group,
R^{b} and R^{c} independently represent a (C₁-C₆)alkyl group or a hydrogen atom,
with the provision that the compound of the formula below is excluded 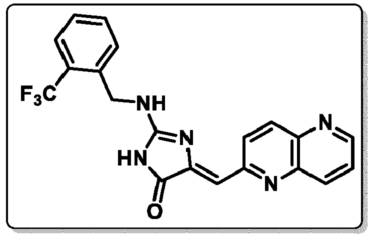
or any of its pharmaceutically acceptable salt.

2. A compound of formula (I) according to claim 1, wherein R¹ represents:
(i). a (C₅-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₉-C₁₀)cycloalkyl group, optionally substituted by a group selected from a (C₁-C₄)alkoxy group, a halogen atom and a hydroxy group,
(iii). a phenyl group fused with a cyclopentyl, which cyclopentyl is substituted by a hydroxy group,
(iv). a phenyl group, substituted by a (C₄-C₇)heterocycloalkyl group, said (C₄-C₇)heterocycloalkyl group being itself optionally substituted by a (C₁-C₄) alkyl group, or
(v). a R'-L- group wherein
• L is either a single bond or a (C₁-C₃)alkanediyl group, optionnally susbstituted by a group selected from a hydroxy group and a (C₁-C₃)alkoxy group, and
• R' represents:
(v.1). a (C₅-C₈)cycloalkyl group, optionally substituted by a group selected from a hydroxy group and a (C₁-C₃) alkoxy group,
(v.2). a (C₅-C₇)heterocycloalkyl group, optionally substituted by one or two groups selected from a hydroxy group and (C₁-C₄)alkyl groups, or
(v.3). a heteroaryl group, optionally substituted by a
(C₁-C₄)alkyl group,
(vi). a R"-L- group wherein
• L is a (C₁-C₃)alkanediyl group, optionally substituted by a group selected from a -NR^{b}R^{c} group, a (C₁-C₃)alkoxy group and a hydroxy group, L being in particular substituted, and
• R" is a phenyl group, optionally substituted by a fluoro(C₁-C4)alkyl group, and
R^{b} and R^{c} independently represent a (C₁-C₆)alkyl group or a hydrogen atom, or any of its pharmaceutically acceptable salt.

3. A compound of formula (I) according to any of claims 1 or 2, wherein R¹ represents:
(i). a (C₅-C₆)alkyl group substituted by a group selected from a hydroxy group, a fluorine atom and a methoxy group, said methoxy group being optionally substituted by a phenyl group, said phenyl group being optionally substituted by a fluorine atom,
(ii). a bridged (C₉-C₁₀)cycloalkyl group in particular an adamantyl or a noradamantyl, optionally substituted by a group selected from a methoxy group, a fluorine atom and a hydroxy group,
(iii). a phenyl group fused with a cyclopentyl, which cyclopentyl is substituted by a hydroxy group,
(iv). a phenyl group, substituted by a *N*-methylpiperazinyl group,
(v). a R'-L- group wherein L is either a single bond or a (C₁-C₃)alkanediyl group, optionnally susbstituted by a group chosen from a hydroxy group and a methoxy group,
and R' is selected from the group consisting of:
(v.1). a (C₅-C₈)cycloalkyl group in particular a cyclopentyl, a cyclohexyl, a cycloheptyl or a cyclooctyl, optionally substituted by a group selected from a hydroxy group and a methoxy group,
(v.2). a (C₆-C₇)heterocycloalkyl group, in particular a tetrahydrofuranyl, a tetrahydropyranyl, an oxepanyl or a dioxepanyl, optionally substituted by one or two groups selected from a hydroxy group and a methyl group,
(v.3). a heteroaryl group, in particular a thiazolyl, a pyrazinyl, a pyrazolyl or a pyridinyl, optionally substituted by a methyl group, or
(vi). a R"-L- group wherein L is a (C₁-C₃)alkanediyl group, optionally substituted by a group selected from the group consisting of a -NH2 group, a methoxy group and a hydroxy group, L being in particular substituted, and R" is a phenyl group, optionally substituted by a trifluoromethyl group,
or any of its pharmaceutically acceptable salts.

4. A compound of formula (I) according to any of the preceding claims, wherein L is selected from a group consisting of a -CH2- group, a -CH(CH₂OH)- group, a -CH(CH₂OCH₃)- group, a -CH(OH)-CH2- group and a -CH(CH2NH2)- group, or any of its pharmaceutically acceptable salts.

5. A compound of formula (I) according to any of the preceding claims, wherein:
(v.1). when R' is a (C₃-C₈)cycloalkyl group, L is a -CH₂- group,
(v.2). when R' is a (C₃-C₉)heterocycloalkyl group, L is a -CH₂- group,
(v.3). when R" is a phenyl, L is selected from the group consisting of a -CH2- group, a -CH(CH₂OH)- group, a -CH(CH₂OCH₃)- group, a -CH(OH)-CH2- group and a -CH(CH2NH2)- group,
(v.4). when R' is a heteroaryl group, L is a -CH₂- group, or any of its pharmaceutically acceptable salts.

6. A compound of formula (I) according to any of the preceding claims, chosen from the following compounds 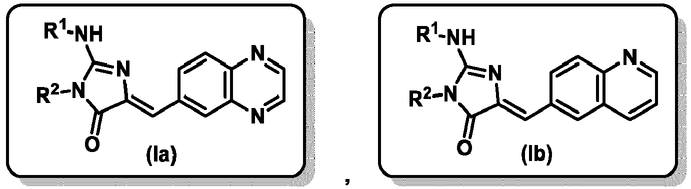 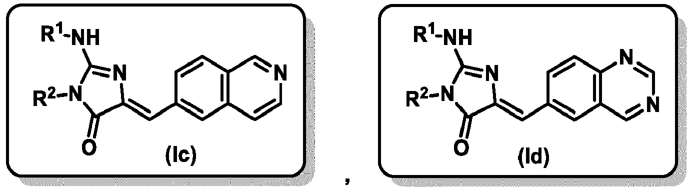 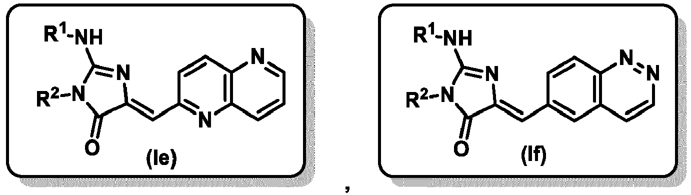 and 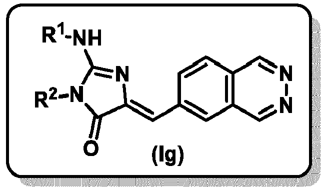
wherein R¹ and R² are as defined in any of the preceding claims,
or any of its pharmaceutically acceptable salts.

7. A compound of formula (I) according to any of the preceding claims, wherein R² is a hydrogen atom or a (C₁-C₄)alkyl group, in particular R² is a hydrogen atom.

8. A compound of formula (I) according to any of the preceding claims, wherein when the compound of formula (I) is chosen from the subformulae (Ia) and (Ib), R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkoxy group, a halogen atom and a hydroxy group, or
(iii). a R'-L- group, wherein L is either a single bond or a (C₁-C₃)alkanediyl group, optionnally susbstituted by a group selected from a hydroxy group and a (C₁-C₃)alkoxy group, and
R' represents:
(iii.1). a (C₃-C₈)cycloalkyl group, optionally substituted by a group selected from a hydroxy group and a (C₁-C₃) alkoxy group,
(iii.2). a (C₃-C₉)heterocycloalkyl group, optionally substituted by one or two groups selected from a hydroxy group and (C₁-C₄)alkyl groups, or
(iii.3). a (C₃-C₈)heteroaryl group, optionally substituted by a (C₁-C₄)alkyl group, or
(iv). a R"-L- group wherein L is a (C₁-C₃)alkanediyl group, optionally substituted by a group selected from a -NR^{b}R^{c} group, a (C₁-C₃)alkoxy group and a hydroxy group, L being in particular substituted, and
R" is a phenyl group, optionally substituted by a fluoro(C₁-C₄)alkyl group,
R^{b} and R^{c} independently represent a (C₁-C₆)alkyl group or a hydrogen atom, or any of its pharmaceutically acceptable salt.

9. A compound of formula (I) according to any of the preceding claims, wherein R¹ represents:
(i). a (C₄-C₆)alkyl group substituted by a group selected from a hydroxy group, a halogen and a (C₁-C₃)alkoxy group, said (C₁-C₃)alkoxy group being optionally substituted by a phenyl group, said phenyl group being optionally substituted by a halogen atom,
(ii). a bridged (C₆-C₁₀)cycloalkyl group, optionally substituted by a group selected from a (C₁-C₄)alkoxy group, a halogen atom and a hydroxy group, or
(iii). a R'-L- group, wherein L is either a single bond or a (C₁-C₃)alkanediyl group, optionnally susbstituted by a group selected from a hydroxy group and a (C₁-C₃)alkoxy group, and
R' represents:
(iii.1). a (C₃-C₈)cycloalkyl group, optionally substituted by a group selected from a hydroxy group and a (C₁-C₃) alkoxy group,
(iii.2). a (C₃-C₉)heterocycloalkyl group, optionally substituted by one or two groups selected from a hydroxy group and (C₁-C₄)alkyl groups, or
(iii.3). a (C₃-C₈)heteroaryl group, optionally substituted by a (C₁-C₄)alkyl group, or
(iv). a R"-L- group wherein L is a (C₁-C₃)alkanediyl group, optionally substituted by a group selected from a -NR^{b}R^{c} group, a (C₁-C₃)alkoxy group and a hydroxy group, L bein in particular substituted, and
R" is a phenyl group, optionally substituted by a fluoro(C₁-C₄)alkyl group,
R^{b} and R^{c} independently represent a (C₁-C₆)alkyl group or a hydrogen atom, or any of its pharmaceutically acceptable salt.

10. A compound of formula (I) according to anyone of claims 1 to 9 selected from:
(1). (4Z)-2-(Cycloheptylamino)-4-(quinoxalin-6-ylmethylene)-1*H*-imidazol-5-one,
(2). (4Z)-2-(Cyclooctylamino)-4-(quinoxalin-6-ylmethylene)-1*H*-imidazol-5-one
(3). (4Z)-2-(Cyclohexylmethylamino)-4-(quinoxalin-6-ylmethylene)-1*H-*imidazol-5-one,
(4). (4*Z*)-2-[[(1*R*,2*R*)-2-Methoxycyclopentyl]amino]-4-(quinoxalin-6-ylmethylene)-1*H*-imidazol-5-one,
(5). (4*Z*)-2-[[(1*S*,2*S*)-2-Methoxycyclopentyl]amino]-4-(quinoxalin-6-ylmethylene)-1*H*-imidazol-5-one,
(6). (±)-(4*Z*)-2- [[*trans*-4-Hydroxycycloheptyl]amino] -4-(quinoxalin-6-ylmethylene)-1*H*-imidazol-5-one,
(7). (±)-(4Z)-2-[[*trans*-4-Methoxycycloheptyl]amino]-4-(quinoxalin-6-ylmethylene)-1*H*-imidazol-5-one,
(8). (±)-(4*Z*)-2-[[*cis*-3-Methoxycycloheptyl]amino]-4-(quinoxalin-6-ylmethylene)-1*H*-imidazol-5-one,
(9). (4*Z*)-2-[[(1*R*)-1-(Hydroxymethyl)-3-methyl-butyl]amino]-4-(quinoxalin-6-ylmethylene)-1*H*-imidazol-5-one,
(10). (4Z)-2-[[(1*R*)-1-(Methoxymethyl)-3-methyl-butyl]amino]-4-(quinoxalin-6-ylmethylene)-1*H*-imidazol-5-one,
(11). (4*Z*)-2-[[(1*R*)-1-(Ethoxymethyl)-3-methyl-butyl]amino]-4-(quinoxalin-6-ylmethylene)-1*H*-imidazol-5-one,
(12). (4*Z*)-2-[[(1*R*)-1-(Benzyloxymethyl)-3-methyl-butyl]amino]-4-(quinoxalin-6-ylmethylene)-1*H*-imidazol-5-one,
(13). (4*Z*)-2- [[(1*R*)-1-[(4-Fluorophenyl)methoxymethyl] -3-methylbutyl]ammo]-4-(quinoxalin-6-ylmethylene)-1*H*-imidazol-5-one,
(14). (4*Z*)-2-[[(1*R*)-1-(Fluoromethyl)-3-methyl-butyl]amino]-4-(quinoxalin-6-ylmethylene)-1*H*-imidazol-5-one,
(15). (4*Z*)-2-[[(1*S*)-1-(Fluoromethyl)-3-methyl-butyl]amino]-4-(quinoxalin-6-ylmethylene)-1*H*-imidazol-5-one,
(16). (4*Z*)-2-(3-Noradamantylamino)-4-(quinoxalin-6-ylmethylene)-1*H-*imidazol-5-one
(17). (4*Z*)-2-(1-Adamantylamino)-4-(quinoxalin-6-ylmethylene)-1*H-*imidazol-5-one,
(18). (4*Z*)-2-[(3-Hydroxy-1-adamantyl)amino]-4-(quinoxalin-6-ylmethylene)-1*H*-imidazol-5-one,
(19). (4*Z*)-2-[(3-Methoxy-1-adamantyl)amino]-4-(quinoxalin-6-ylmethylene)-1*H*-imidazol-5-one,
(20). (4*Z*)-2-[(3-Fluoro-1-adamantyl)amino]-4-(quinoxalin-6-ylmethylene)-1*H*-imidazol-5-one,
(21). (4*Z*)-2-(Benzylamino)-4-(quinoxalin-6-ylmethylene)-1*H*-imidazol-5-one,
(22). (4*Z*)-4-(Quinoxalin-6-ylmethylene)-2-[[2-(trifluoromethyl)phenyl]methylamino]-1*H*-imidazol-5-one,
(23). (4*Z*)-2-[[(1*S*,2*S*)-2-Hydroxyindan-1-yl]amino]-4-(quinoxalin-6-ylmethylene)-1*H*-imidazol-5-one,
(24). (4*Z*)-2-[[(1*R*)-2-Hydroxy-1-phenyl-ethyl]amino4-(quinoxalin-6-ylmethylene)-1*H*-imidazol-5-one,
(25). (4*Z*)-2-[[(1*S*)-2-Hydroxy-1-phenyl-ethyl]amino]-4-(quinoxalin-6-ylmethylene)-1*H*-imidazol-5-one,
(26). (4*Z*)-2-[[(1*R*)-2-Methoxy-1-phenyl-ethyl]amino]-4-(quinoxalin-6-ylmethylene)-1*H*-imidazol-5-one,
(27). (4*Z*)-2-[[(2*R*)-2-Hydroxy-2-phenyl-ethyl]amino]-4-(quinoxalin-6-ylmethylene)-1*H*-imidazol-5-one,
(28). (4*Z*)-2-[[(1*R*)-2-Ammo-1-phenyl-ethyl]amino]-4-(quinoxalin-6-ylmethylene)-1*H*-imidazol-5-one dihydrochloride,
(29). (4*Z*)-2-[[(1*S*)-2-Ammo-1-phenyl-ethyl]amino]-4-(quinoxalin-6-ylmethylene)-1*H*-imidazol-5-one dihydrochloride,
(30). (4Z)-2-[(5-Methylpyrazin-2-yl)methylamino]-4-(quinoxalin-6-ylmethylene)-1*H*-imidazol-5-one,
(31). (4*Z*)-2-[(4-Methylthiazol-2-yl)methylamino]-4-(quinoxalin-6-ylmethylene)-1*H*-imidazol-5-one,
(32). (4*Z*)-4-(Quinoxalin-6-ylmethylene)-2-(tetrahydropyran-4-ylmethylamino)-1*H*-imidazol-5-one,
(33). (4*Z*)-2-[4-(4-Methylpiperazin-1-yl)anilino]-4-(quinoxalin-6-ylmethylene)-1*H*-imidazol-5-one,
(34). (4*Z*)-2-[(1-Methylpyrazol-3-yl)amino]-4-(quinoxalin-6-ylmethylene)-1*H*-imidazol-5-one,
(35). (4*Z*)-2-(2-Pyridylamino)-4-(quinoxalin-6-ylmethylene)-1*H*-imidazol-5-one,
(36). (±)-(4*Z*)-2-[(6,6-Dimethyltetrahydropyran-3-yl)amino]-4-(quinoxalin-6-ylmethylene)-1*H*-imidazol-5-one,
(37). (4*Z*)-4-(Quinoxalin-6-ylmethylene)-2-[[(3*R*)-tetrahydrofuran-3-yl]amino]-1*H*-imidazol-5-one,
(38). (4*Z*)-4-(Quinoxalin-6-ylmethylene)-2-[[(3*S*)-tetrahydropyran-3-yl]amino]-1*H*-imidazol-5-one,
(39). (4*Z*)-4-(Quinoxalin-6-ylmethylene)-2-[[(3*R*)-tetrahydropyran-3-yl]amino]-1*H*-imidazol-5-one,
(40). (4*Z*)-2-[[(3*R*,4*R*)-4-Hydroxytetrahydropyran-3-yl]amino]-4-(quinoxalin-6-ylmethylene)-1*H*-imidazol-5-one,
(41). (4*Z*)-2-(Oxepan-3-ylamino)-4-(quinoxalin-6-ylmethylene)-1*H-*imidazol-5-one,
(42). (4*Z*)-2-(1,4-Dioxepan-6-ylamino)-4-(quinoxalin-6-ylmethylene)-1*H-*imidazol-5-one,
(43). (4Z)-2-(Cyclohexylamino)-4-(6-quinolylmethylene)-1*H*-imidazol-5-one,
(44). (4*Z*)-2-(Cycloheptylamino)-4-(6-quinolylmethylene)-1*H*-imidazol-5-one,
(45). (4*Z*)-2-(Cyclohexylmethylamino)-4-(6-quinolylmethylene)-1*H-*imidazol-5-one,
(46). (4*Z*)-2-[[(1*R*)-1-(Hydroxymethyl)-3-methyl-butyl]amino]-4-(6-quinolylmethylene)-1*H*-imidazol-5-one,
(47). (4*Z*)-2-[[(1*R*)-1-(Methoxymethyl)-3-methyl-butyl]amino]-4-(6-quinolylmethylene)-1*H*-imidazol-5-one,
(48). (4*Z*)-2-(3-Noradamantylamino)-4-(6-quinolylmethylene)-1*H*-imidazol-5-one,
(49). (4*Z*)-2-(1-Adamantylamino)-4-(6-quinolylmethylene)-1*H*-imidazol-5-one,
(50). (4*Z*)-2-[(3-Hydroxy-1-adamantyl)amino]-4-(6-quinolylmethylene)-1*H-*imidazol-5-one,
(51). (4*Z*)-2-(Benzylamino)-4-(6-quinolylmethylene)-1*H*-imidazol-5-one,
(52). (4*Z*)-2-[[(1*R*)-2-Methoxy-1-phenyl-ethyl]amino]-4-(6-quinolylmethylene)-1*H*-imidazol-5-one,
(53). (4*Z*)-2-[(5-Methylpyrazin-2-yl)methylamino]-4-(6-quinolylmethylene)-1*H*-imidazol-5-one,
(54). (4*Z*)-2-[(1-Methylpyrazol-3-yl)amino]-4-(6-quinolylmethylene)-1*H-*imidazol-5-one,
(55). (4*Z*)-4-(6-Quinolylmethylene)-2-[[(3*R*)-tetrahydrofuran-3-yl]amino]-1*H*-imidazol-5-one,
(56). (4*Z*)-4-(6-Quinolylmethylene)-2-[[(3*R*)-tetrahydropyran-3-yl]amino]-1*H*-imidazol-5-one,
(57). (4*Z*)-2-(Cycloheptylamino)-4-(6-isoqumolylmethylene)-1*H*-imidazol-5-one,
(58). (4*Z*)-2-(Cyclohexylmethylamino)-4-(6-isoquinolylmethylene)-1*H-*imidazol-5-one,
(59). (4*Z*)-2-[[(1*R*)-1-(Hydroxymethyl)-3-methyl-butyl]amino]-4-(6-isoquinolylmethylene)-1*H*-imidazol-5-one,
(60). (4*Z*)-4-(6-Isoquinolylmethylene)-2-[[(1*R*)-1-(methoxymethyl)-3-methyl-butyl]amino]-1*H*-imidazol-5-one,
(61). (4*Z*)-2-(1-Adamantylamino)-4-(6-isoquinolylmethylene)-1*H*-imidazol-5-one,
(62). (4*Z*)-2-(Benzylamino)-4-(6-isoquinolylmethylene)-1*H*-imidazol-5-one,
(63). (4*Z*)-4-(6-Isoquinolylmethylene)-2-[[(1*R*)-2-methoxy-1-phenylethyl]amino]-1*H*-imidazol-5-one,
(64). (4*Z*)-4-(6-Isoquinolylmethylene)-2-[(5-methylpyrazin-2-yl)methylamino]-1*H*-imidazol-5-one,
(65). (4*Z*)-4-(6-Isoquinolylmethylene)-2-[(1-methylpyrazol-3-yl)amino]-1*H-*imidazol-5-one,
(66). (4*Z*)-4-(6-Isoquinolylmethylene)-2-[[(3*R*)-tetrahydrofuran-3-yl]amino]-1*H*-imidazol-5-one,
(67). (4*Z*)-4-(6-Isoquinolylmethylene)-2-[[(3*R*)-tetrahydropyran-3-yl]amino]-1*H*-imidazol-5-one,
(68). (4*Z*)-2-(1-Adamantylamino)-4-(quinazolin-6-ylmethylene)-1*H-*imidazol-5-one,
(69). (4*Z*)-2-[[(1*R*)-2-Methoxy-1-phenyl-ethyl]amino]-4-(quinazolin-6-ylmethylene)-1*H*-imidazol-5-one,
(70). (4*Z*)-2-(1-Adamantylamino)-4-(1,5-naphthyridin-2-ylmethylene)-1*H-*imidazol-5-one,
(71). (4*Z*)-2-[[(1*R*)-2-Methoxy-1-phenyl-ethyl]amino]-4-(1,5-naphthyridin-2-ylmethylene)-1*H*-imidazol-5-one,
(72). (4*Z*)-4-(Cinnolin-6-ylmethylene)-2-[[(1*R*)-2-methoxy-1-phenylethyl]amino]-1*H*-imidazol-5-one,
(73). (4*Z*)-2-[[(1*R*)-2-Methoxy-1-phenyl-ethyl]amino]4-(phthalazin-6-ylmethylene)-1*H*-imidazol-5-one,
or anyone of their pharmaceutically acceptable salts.

11. A pharmaceutical composition comprising at least one compound as defined in anyone of claims 1 to 9 or or any of its pharmaceutically acceptable salt or any of the compounds (1) to (73) as defined in claim 10.

12. Synthesis process for manufacturing a compound of formula (I) as defined in anyone of claims 1 to 9 or or any of its pharmaceutically acceptable salt or any of the compounds (1) to (73) as defined in claim 10 or any of its pharmaceutically acceptable salts, comprising at least a step of coupling a compound of formula (VI) below 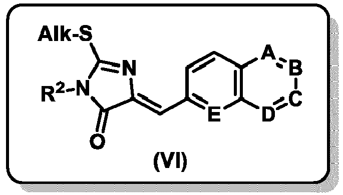 wherein R², A, B, C, D and E are as in any one of claims 1 to 9 and Alk is a (C₁-C₅)alkyl, with an amine of formula R¹NH₂ wherein R¹ is as defined in any one of claims 1 to 9.

13. Synthesis process for manufacturing a compound of formula (I) as defined in anyone of claims 1 to 9 or or any of its pharmaceutically acceptable salt or as any of the compounds (1) to (73) defined in claim 10 or any of its pharmaceutically acceptable salts, comprising at least a step of coupling a compound of formula (II) below 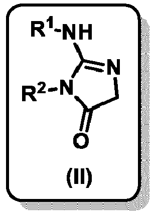
wherein R¹ and R² are as defined in any one of claims 1 to 9
with a compound of formula (III) below 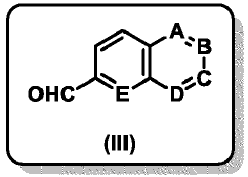
wherein A, B, C, D and E are as defined in any one of claims 1 to 9.

14. A synthetic intermediate selected from the compounds below 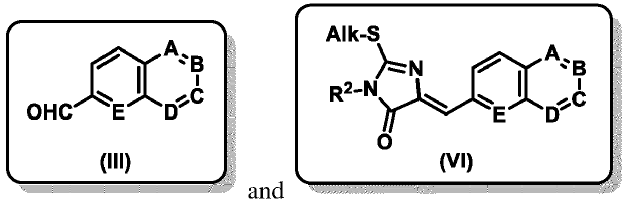 wherein Alk is a (C₁-C₅)alkyl, in particular Alk is selected from the group consisting of an ethyl and a methyl, and wherein R², A, B, C, D and E are as defined in any one of claims 1 to 9.

15. A compound of formula (I) as defined in anyone of claim 1 to 9 or any of its pharmaceutically acceptable salts, or at least any of compounds (1) to (73) as defined in claim 10 or any of its pharmaceutically acceptable salts, for use as a medicament.

16. A compound of formula (I) according to anyone of claims 1 to 9 or any of its pharmaceutically acceptable salts, or at least any of compounds (1) to (73) as defined in claim 10 or any of its pharmaceutically acceptable salts for use in the treatment and/or in the prevention of a disease selected from cognitive deficits and neuroinflammation associated with Down syndrome, Alzheimer's disease and related diseases, dementia and/or tauopathies, as well as other neurodegenerative diseases such as Parkinson's disease, and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointerstinal cancer, breast cancer, such as Triple-negative breast cancer, tissue cancer, including liposarcoma, Hedgehog/GLI-dependent cancer, liver cancer, including Hepatocellular carcinoma and viral infections, such as infections caused by Human immunodeficiency virus type 1 , Human cytomegalovirus, Influenza A, Herpes virus, rhesus macaque cytomegalovirus, varicella-zoster virus, herpes simplex virus, Hepatitis C virus, Chikungunya virus, Dengue virus, Influenza virus and Severe acute respiratory syndrome coronavirus, Cytomegalovirus and Human papillomavirus; neuroinflammation; anemia; infections caused by unicellular parasites, such as malaria, Leishmaniasis, Chagas and sleeping sickness, cattle diseases due to unicellular pathogens,and for regulating body temperature.

17. A compound of formula (I) for use according to anyone of claims 1 to 9 or any of its pharmaceutically acceptable salts, or at least any of compounds (1) to (73) as defined in claim 10 or any of its pharmaceutically acceptable salts for use in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions and type 1 and type 2 diabetes.
